# EUROPÄISCHE PATENTANMELDUNG

(11) **EP 2 060 575 A1**
(43) Veröffentlichungstag der Anmeldung: **20.05.2009**
(21) Anmeldenummer: 09152706.9
(22) Anmeldetag: 19.04.2006
(51) Int. Cl.: C07D 495/04, A61P 11/00, A61K 31/519, C07D 521/00

(54) **Dihydrothienopyrimidine zur Behandlung von entzündlichen Erkrankungen**

(30) Priorität: 21.04.2005 DE 102005019201
(62) Teilanmeldung aus: 06743350.8
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Pouzet, Pascale, 88400, BIBERACH (DE); Hoenke, Christoph, 55218, INGELHEIM (DE); Martyres, Domnic, 88400, BIBERACH (DE); Nickolaus, Peter, 88447, WARTHAUSEN (DE); Jung, Birgit, 88471, LAUPHEIM (DE)
(74) Vertreter: Hammann, Heinz

(57) **Zusammenfassung**

Die Erfindung betrifft neue Dihydrothienopyrimidine der Formel 1, sowie pharmakologisch verträgliche Salze, Diastereomere, Enantiomere, Racemate, Hydrate oder Solvate davon, 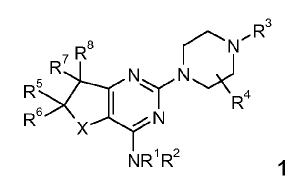 die geeignet sind zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen, sowie pharmazeutische Zusammensetzungen die diese Verbindungen beinhalten.

## Beschreibung

Die Erfindung betrifft neue Dihydrothienopyrimidine der Formel **1,** sowie pharmakologisch verträgliche Salze, Diastereomere, Enantiomere, Racemate, Hydrate oder Solvate davon, 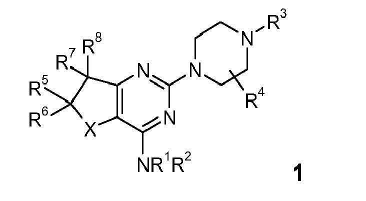 die geeignet sind zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen, sowie pharmazeutische Zusammensetzungen die diese Verbindungen beinhalten.

### STAND DER TECHNIK

US 3,318,881 und BE 663693 offenbaren die Herstellung von Dihydrothieno-[3,2-d]pyrimidine die kardiovaskulären und sedative Eigenschaften besitzen.

### BESCHREIBUNG DER ERFINDUNG

Überraschenderweise konnte nun gefunden werden, dass Dihydrothienopyrimidine der Formel **1** geeignet sind zur Behandlung entzündlicher Erkrankungen. Gegenstand der vorliegenden Erfindung sind deshalb Verbindungen der Formel **1** 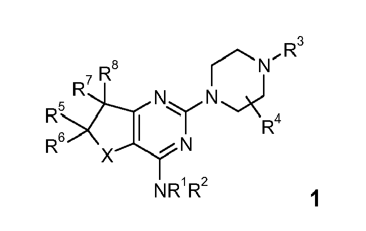 worin
- **X**: O, S, SO oder SO₂;
- **R¹**: H, C₁₋₆-Alkyl, C₂₋₆-Alkenyl, C₂₋₆-Alkinyl, C₆₋₁₀-Aryl oder C₆₋₁₀-Aryl-C₁₋₆-alkylen, C₅₋₁₀-Heteroaryl-C₁₋₆-alkylen;
- **R²**: H oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, ein gegebenenfalls einfach oder mehrfach überbrücktes mono- oder bicyclisches C₃₋₁₀-Cycloalkyl, C₆₋₁₀-Aryl, ein aromatischer oder nicht aromatischer, heterocyclischer C₃₋₁₀-Ring, ein bicyclischer Ring und ein mit einem C₃₋₁₀-Heterocyclus kondensiertes C₆₋₁₀-Aryl;
- oder: **NR¹R²** bedeutet gemeinsam einen heterocyclischen Ring, der gegebenenfalls mit einem oder mehreren Resten ausgewählt aus C₁₋₄-Alkyl, C₂₋₆-Alkenyl, C₂₋₆-Alkinyl, linearem oder verzweigtem C₁₋₆-Alkanol und Oxo substituiert ist;
- **R³**: ein einfach oder mehrfach substituierter Rest ausgewählt aus der Gruppe bestehend aus einem heterocyclischen C₆₋₁₀-Ring, C₃₋₇-Cycloalkyl, C₆₋₁₀-Aryl-C₁-₆-alkylen, C₅₋₁₀-Heteroaryl-C₁₋₆-alkylen, einem kondensierten, bicyclischen Ring, der gegebenenfalls 1-4 Heteroatome unabhängig voneinander ausgewählt aus N, O, S enthalten kann,
- oder **R³**: bedeutet gegebenenfalls substituiertes Phenyl;
- oder **R³**: bedeutet eine Gruppe COR^{3.7}, COCH₂R^{3.8}, CONHR^{3.8} oder SO₂R^{3.8};
wobei
R^{3.7} H, C₁₋₆-Alkyl, C₂₋₆-Alkenyl, C₂₋₆-Alkinyl oder C₆₋₁₀-Aryl;
R^{3.8} C₁₋₆-Alkyl, C₂₋₆-Alkenyl, C₂₋₆-Alkinyl, C₃₋₇-Cycloalkyl oder ein Rest ausgewählt aus der Gruppe bestehend aus C₆₋₁₀-Aryl, einem heterocyclischen C₃₋₁₀-Ring und einem bicyclischen Ring, der gegebenenfalls mit einem oder mehreren Resten ausgewählt aus der Gruppe bestehend aus C₁₋₆-Alkyl, C₂₋₆-Alkenyl, C₂₋₆-Alkinyl, Halogen, NR^{3.8.1}R^{3.8.2}, C₆₋₁₀-Aryl und einem heterocyclischen C₃₋₁₀-Ring substituiert ist; wobei
R^{3.8.1} H, C₁₋₆-Alkyl, C₂₋₆-Alkenyl oder C₂₋₆-Alkinyl;
R^{3.8.2} H, C₁₋₆-Alkyl, C₂₋₆-Alkenyl oder C₂₋₆-Alkinyl;
- **R⁴**: H, C₁₋₄-Alkyl, C₂₋₄-Alkenyl, C₂₋₄-Alkinyl, oder Oxo;
- **R⁵**: H, C₁₋₄-Alkyl, C₂₋₄-Alkenyl oder C₂₋₄-Alkinyl;
- **R⁶**: H, C₁₋₄-Alkyl, C₂₋₄-Alkenyl oder C₂₋₄-Alkinyl;
- **R⁷**: H, C₁₋₄-Alkyl, C₂₋₄-Alkenyl, C₂₋₄-Alkinyl, C₆₋₁₀-Aryl, oder OH;
- **R⁸**: H, C₁₋₄-Alkyl, C₂₋₄-Alkenyl, C₂₋₄-Alkinyl, C₆₋₁₀-Aryl oder OH;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

Bevorzugt sind die obigen Verbindungen der Formel 1, worin
- **X**: O, S, SO oder SO₂;
- **R¹**: H, C₁₋₆-Alkyl, C₆₋₁₀-Aryl, C₆₋₁₀-Aryl-C₁₋₆-alkylen, C₅₋₁₀-Heteroaryl-C₁₋₆-alkylen,
- **R²**: H oder C₁₋₆-Alkyl, das gegebenenfalls mit einem oder mehreren Resten ausgewählt aus der Gruppe bestehend aus C₁₋₆-Haloalkyl, CN, OR^{2.1}, NR^{2.1}R^{2.2}, COOR^{2.1}, CONR^{2.1}R^{2.2}, gegebenenfalls mit C₁₋₄-Alkyl oder mit Oxo substituiertes C₃₋₇-Cycloalkyl, gegebenenfalls mit C₁₋₄-Alkyl, mit Oxo, mit OH oder mit Halogen substituierter aromatischer oder nicht aromatischer Heterocyclus, gegebenenfalls mit C₁₋₄-Alkyl oder mit Oxo substituiertes C₆₋₁₀-Aryl und mit einem C₅₋₆-Heterocyclus kondensiertes C₆₋₁₀-Aryl, wobei dieses kondensierte Ringsystem gegebenenfalls mit C₁₋₄-Alkyl oder mit Oxo substituiert sein kann, substituiert sein kann
wobei
R^{2.1} H oder C₁₋₆-Alkyl;das gegebenenfalls substituiert ist mit einem jeweils gegebenenfalls substituierten C₃₋₇-Cycloalkyl, C₃₋₁₀-Heterocyclus oder C₆₋₁₀-Aryl
R^{2.2} H oder C₁₋₆-Alkyl;
- oder **R²**: ein Rest ausgewählt aus gegebenenfalls einfach oder mehrfach überbrücktem C₃₋₁₀-Cycloalkyl oder ein C₃₋₁₀-Cycloalkyl, das gegebenenfalls kondensiert sein kann mit einem C₆₋₁₀-Arylring, der gegebenenfalls mit einem oder mehreren Resten ausgewählt aus der Gruppe bestehend aus C₁₋₆-Alkyl, OH, CH₂OR^{2.3}, COOR^{2.3}, COR^{2.3}, CONR^{2.3}R^{2.4}, O-C₁₋₆-Alkyl, O-C₇₋₁₁-Aralkyl, NR^{2.3}R^{2.4} und NHCOR^{2.5}substituiert sein kann ,
wobei
R^{2.3} H oder ein Heterocyclus ist oder ein C₁₋₆-Alkyl ist, welches gegebenenfalls mit einem Rest ausgewählt aus C₃₋₇-Cycloalkyl, C₃₋₁₀-Heterocyclus und C₆₋₁₀-Aryl substituiert sein kann, wobei dieser Rest jeweils gegebenenfalls mit einem oder mehreren Resten aus der Gruppe C₁₋₆-Alkyl, Halogen, OH und O-C₁₋₆-Alkyl substituiert sein kann ;
wobei
R^{2.4} H oder C₁₋₆-Alkyl;
R^{2.5} ein Rest ausgewählt aus der Gruppe bestehend aus C₃₋₇-Cycloalkyl, ein heterocyclischer C₃₋₁₀-Ring und C₁₋₆-Alkyl, welcher gegebenenfalls mit OH substituiert sein kann;
- oder R²: bedeutet eine Gruppe der Formel **1a**, 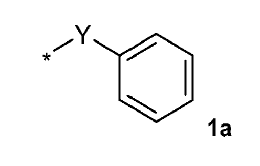 Y C₁₋₆-Alkylen, gegebenenfalls substituiert mit einem oder zwei R^{2.7}
wobei
R^{2.7} jeweils unabhängig voneinander ausgewählt sind aus C₁₋₆-Alkyl, COOH, CONH₂, OR^{2.1}, und COOR^{2.1}; oder R^{2.7} bildet gemeinsam mit einem oder zwei Kohlenstoffatomen von Y einen carbocyclischen Ring mit 3 Kohlenstoffatomen
- oder **R²**: C₆₋₁₀-Aryl, das gegebenenfalls mit einem oder mehreren Resten unabhängig voneinander ausgewählt aus der Gruppe bestehend aus C₁₋₆-Alkyl, C₁₋₆-Haloalkyl, C₂₋₆-Alkenyl, C₂₋₆-Alkinyl, CN, Halogen, OR^{2.8}, COOR^{2.8}, COR^{2.10}, NHCOMe, CONR^{2.3}R^{2.4},einem mit NR^{2.1}R^{2.2} substituierten C₁₋₄Alkylenrest, NR^{2.1}R^{2.2} substituiert sein kann
- oder **R²**: C₆₋₁₀-Aryl, das gegebenenfalls mit einem oder mehreren Resten unabhängig voneinander ausgewählt aus der Gruppe bestehend aus C₆₋₁₀-Aryl-C₁₋₆-alkylen, C₅₋₁₀-Heteroaryl-C₁₋₆-alkylen, C₃₋₇-Cycloalkyl, ein C₃₋₇-Cycloalkyl-C₁₋₄-alkylen, C₆₋₁₀-Aryl, und einem heterocyclischen C₃₋₁₀-Ring substituiert sein kann, wobei diese Reste jeweils gegebenenfalls mit einem oder mehreren Resten ausgewählt aus C₁₋₆-Alkyl, C₁₋₆-Haloalkyl, COOR^{2.8}, CN, Halogen, OR^{2.8}, NHCOR^{2.8}, Oxo, einem C₃₋₁₀-Heterocyclus, einem C₃₋₇-Cycloalkyl-C₁₋₄alkylen, ein C₅₋₁₀-Heterocyclus -C₁₋₄-alkylen und einem NR^{2.1}R^{2.2} -C₁₋₄ alkylen substituiert sein können,
wobei
R^{2.8} H, C₁₋₆-Alkyl, C₆₋₁₀-Aryl, ein NR^{2.1}R^{2.2}-C₁₋₄ alkylenrest, ;
R^{2.10} NHR^{2.10.1} oder ein heterocyclischer C₃₋₁₀-Ring, der gegebenenfalls mit C₁₋₄-Alkyl substituiert sein kann;
R^{2.10.1} H, C₃₋₇-Cycloalkyl, C₂₋₆-Alkenyl, C₂₋₆-Alkinyl,C₁₋₆-Alkyl oder C₁₋₆-Alkyl-O-C₁₋₄-Alkyl
- oder **R²**: bedeutet C₆₋₁₀-Aryl, an das ein aromatischer oder nicht aromatischer C₃₋₁₀-Heterocyclus ankondensiert ist;
- oder **R²**: aromatischer oder nicht aromatischer heterocyclischer C₃₋₁₀-Ring, der gegebenenfalls mit einem oder mehreren Resten ausgewählt aus der Gruppe bestehend aus Halogen, OH, Oxo-, CN, C₁₋₆-Alkyl, C₁₋₆-Alkanol, C₆₋₁₀-Aryl-C₁₋₆-alkylen, C₅₋₁₀-Heteroaryl-C₁₋₆-alkylen, COR^{2.11}, C₃₋₇-Cycloalkyl-C₁₋₄-alkylen und C₃₋₁₀-Heterocyclus-C₁₋₄-alkylen substituiert sein kann;
wobei
R^{2.11} ein Rest ausgewählt aus der Gruppe bestehend aus C₃₋₁₀-Heterocyclus-C₁₋₄-alkylen, C₃₋₇-Cycloalkyl und einem heterocyclischen aromatischen oder nichtaromatischen C₃₋₁₀-Ring,
welcher gegebenenfalls mit C₁₋₆-Alkyl substituiert sein kann, das wiederum gegebenenfalls mit OH, CH₂OH, OMe, NH₂, einem C₃₋₁₀-Heterocyclus oder NHCOO-^{t}Bu substituiert sein kann;
- oder **R²** ein: Rest ausgewählt aus der Gruppe bestehend aus C₂₋₆-Alkeyl oder einem bicyclischen Ring, welcher gegebenenfalls mit Methyl substituiert sein kann;
- oder **NR¹R²**: ein heterocyclischer Ring, der gegebenenfalls mit einem oder mehreren Resten ausgewählt aus der Gruppe C₁₋₄-Alkyl, OH und C₁₋₄-Alkanol substituiert sein kann;
- **R³**: ein Rest ausgewählt aus der Gruppe bestehend aus einem heterocyclischen C₃₋₁₀-Ring, einem C₃₋₇-Cycloalkyl, einem bicyclischen, kondensierten aromatischen oder nicht aromatischem Ringsystem, das gegebenenfalls 1 bis 4 Heteroatome ausgewählt aus S, N, O enthält, C₆₋₁₀-Aryl-C₁₋₆-alkylen, C₅₋₁₀-Heteroaryl-C₁₋₆-alkylen und CH₂-Benzo[1, 3]dioxolyl, der gegebenenfalls mit einem oder mehreren Resten ausgewählt aus OH, Halogen, C₁₋₆-Alkyl, O-C₁₋₆-Alkyl, C₁₋₆-Haloalkyl und CO-R^{3.1} substituiert sein kann,
- oder **R³**: Phenyl, welches gegebenenfalls mit einem oder mehreren Resten ausgewählt aus der Gruppe bestehend aus C₁₋₆-Alkyl, C₂₋₆-Alkenyl, C₂₋₆-Alkinyl, C₁₋₄-Haloalkyl, C₁₋₆-Alkylen-NR^{3.1}R^{3.2}, CN, COOR^{3.1}, CONR^{3.1}R^{3.2}, NR^{3.1}R^{3.2}, NHCOR^{3.1}, CF₃, OR^{3.1}, Halogen, NHCOR^{3.1}, NO₂, SO₂NR^{3.1}R^{3.2} und C₁₋₆-Alkylen-NHCOR^{3.1}substituiert sein kann;
wobei
R^{3.1} H ,C₁₋₆-Alkyl; C₂₋₆-Alkenyl oder C₂₋₆-Alkinyl, gegebenenfalls überbrückter, mono- oder bicyclischer C₃₋₁₀-Heterocyclus oder C₃₋₁₀-Heterocyclus-C₁₋₄-Alkylenrest;
R^{3.2} H , C₁₋₆-Alkyl, , C₂₋₆-Alkenyl oder C₂₋₆-Alkinyl;
- oder **R³**: eine Gruppe der Formel 1b 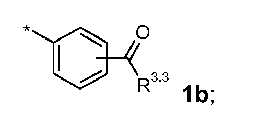 worin
R^{3.3} ein Rest ausgewählt aus der Gruppe bestehend aus einem heterocyclischen C₃₋₁₀-Ring, der gegebenenfalls mit einem oder mehreren Resten ausgewählt aus der Gruppe bestehend aus C₁₋₆-Alkyl, Oxo, COR^{3.3.1}, COR^{3.3.2} C₁₋₆-Alkylen-R^{3.3.2}, CH₂CO-Pyrrolidin und einem heterocyclischen C₃₋₁₀-Ring, worin ein gegebenenfalls im heterocyclischen Ring enthaltenes Schwefelatom, gegebenenfalls als Oxid oder Dioxid vorliegen kann, substituiert sein kann;
wobei
R^{3.3.1} C₁₋₆-Alkyl;
R^{3.3.2} NH₂, NH(C₁₋₆-Alkyl), N(C₁₋₆-Alkyl)₂;
oder R^{3.3} bicyclischer Ring oder heterocyclischer Spiroring;
- oder **R³**: eine Gruppe der Formel **1c;** 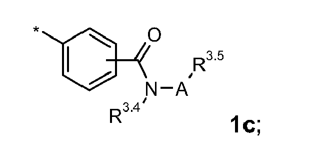 worin
A Bindung oder C₁₋₆-Alkyl, das gegebenenfalls mit Oxo oder NMe₂ substituiert sein kann;
R^{3.4} H oder C₁₋₆-Alkyl;
R^{3.5} ein Rest ausgewählt aus der Gruppe bestehend aus
C₁₋₆-Alkyl, das gegebenenfalls mit einem oder mehreren Resten ausgewählt aus C₃₋₇-Cycloalkyl, C₆₋₁₀-Aryl und einem C₃₋₁₀-Heterocyclus substituiert sein kann, wobei dieser Rest jeweils auch gegebenenfalls mit einem Rest ausgewählt aus der Gruppe OH, Oxo, C₁₋₆-Alkyl, O-C₁₋₆-Alkyl, C₁₋₆-Haloalkyl substituiert sein kann,
oder ein Rest ausgewählt aus einem heterocyclischer C₃₋₁₀-Ring und einem bicyclischen Ring, der gegebenenfalls mit einem oder mehreren Resten unabhängig voneinander ausgewählt aus der Gruppe bestehend aus Oxo, C₁₋₆-Alkyl, OH, C₆₋₁₀-Aryl, einem heterocyclischen C₃₋₁₀-Ring, C₁₋₆-Alkylen-R^{3.5.1}, O-C₁₋₆-Alkylen-R^{3.5.1}und NH-C₁₋₆-Alkylen-R^{3.5.1}substituiert sein kann,
R^{3.5.1} ein Rest ausgewählt aus der Gruppe bestehend aus C₆₋₁₀-Aryl, heterocyclischer C₃₋₁₀-Ring, der gegebenenfalls mit C₁₋₆-Alkyl substituiert sein kann;
oder **R³** bedeutet eine Gruppe der Formel **1d;** 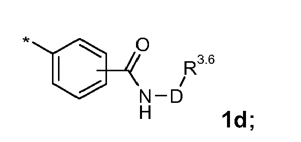 worin
D C₂₋₄-Alkinyl; ein gegebenenfalls überbrückter, bicyclischer C₃₋₁₀-Cycloalkylrest, der gegebenenfalls einem oder mehreren Resten ausgewählt aus mit C₁₋₆-Alkyl, Halogen, OH, C₁₋₆-Haloalkyl und O-C₁₋₆-Alkyl substituiert sein kann,
R^{3.6} Pyridinyl;
- oder **R³**: bedeutet eine einen Rest ausgewählt aus der Gruppe aus COR^{3.7}, COCH₂R^{3.8}, CONHR^{3.8}, SO₂R^{3.8} und einem mit einem C₆₋₁₀-Arylrest kondensierten Heterocyclus, der gegebenenfalls mit Methyl substituiert sein kann,
- oder **R³**: eine Gruppe der Formel **1e**; 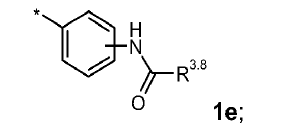 R^{3.7} H, C₁₋₆-Alkyl, C₆₋₁₀-Aryl;
R^{3.8} Rest ausgewählt aus der Gruppe aus C₁₋₆-Alkyl, C₃₋₇-Cycloalkyl oder ein Rest ausgewählt aus der Gruppe bestehend aus C₆₋₁₀-Aryl, heterocyclischer C₃₋₁₀-Ring und ein bicyclischer Ring, der gegebenenfalls mit einem oder mehreren Resten ausgewählt aus der Gruppe bestehend aus C₁₋₆-Alkyl, Halogen, NR^{3.8.1}R^{3.8.2}, C₆₋₁₀-Aryl und einem heterocyclischen C₃₋₁₀-Ring substituiert sein kann;
R^{3.8.1} H oder C₁₋₆-Alkyl;
R^{3.8.2} H oder C₁₋₆-Alkyl;
- **R⁴**: H, C₁₋₄-Alkyl oder Oxo;
- **R⁵**: H oder C₁₋₄-Alkyl;
- **R⁶**: H oder C₁₋₄-Alkyl;
- **R⁷**: H, C₁₋₄-Alkyl, C₆₋₁₀-Aryl oder OH;
- **R⁸**: H, C₁₋₄-Alkyl, C₆₋₁₀-Aryl oder OH;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.
Bevorzugt sind die obigen Verbindungen der Formel **1,** worin
- **X**: O, S, SO oder SO₂; bevorzugt S, SO oder SO₂;
- **R¹**: H, C₁₋₄-Alkyl;
- **R²**: H oder C₁₋₆-Alkyl, gegebenenfalls substituiert mit einem Rest ausgewählt aus der Gruppe bestehend aus C₁₋₄-Haloalkyl, CN, OR^{2.1}, NR^{2.1}R^{2.2}, COOR^{2.1} und CONR^{2.1}R^{2.2} oder gegebenenfalls substituiert mit ein Rest ausgewählt aus der Gruppe bestehend aus C₃₋₇-Cycloalkyl, C₆₋₁₀-Aryl und ein heterocyclischer, aromatischer C₅₋₁₀-Ring, gegebenenfalls substituiert mit Methyl oder Oxo;
R^{2.1} H oder C₁₋₄-Alkyl;
R^{2.2} H oder C₁₋₄-Alkyl;
- oder **R²**: C₃₋₇-Cycloalkyl, gegebenenfalls substituiert mit einem Rest ausgewählt aus der Gruppe bestehend aus C₁₋₄-Alkyl, CH₂OR^{2.3}, COOR^{2.3}, CONR^{2.3}R^{2.4}, O-Benzyl, NR^{2.3}R^{2.4} oder NHCOR^{2.5;}
R^{2.3} H oder C₁₋₄-Alkyl;
R^{2.4} H oder C₁₋₄-Alkyl;
R^{2.5} C₃₋₇-Cycloalkyl, ein heterocyclischer, aromatischer C₅₋₁₀-Ring oder C₁₋₆-Alkyl, gegebenenfalls substituiert mit OH;
- oder **R²**: eine Gruppe der Formel **1a**, 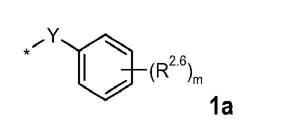 Y C₁₋₄-Alkylen, gegebenenfalls substituiert mit einem oder zwei R^{2.7}
m 0, 1, 2;
R^{2.7} jeweils unabhängig voneinander C₁₋₄-Alkyl, COOH, CONH₂; oder bildet gemeinsam mit einem oder zwei Kohlenstoffatomen von Y einen carbocyclischen Ring mit 3 Kohlenstoffatomen
- oder **R²**: C₆₋₁₀-Aryl, gegebenenfalls substituiert mit einem oder mehreren Resten ausgewählt aus der Gruppe bestehend aus C₁₋₄-Alkyl, CN, Halogen, OR^{2.8}, COOR^{2.8}, COR^{2.10} und NHCOMe
R^{2.8} C₁₋₄-Alkyl oder C₆₋₁₀-Aryl;
R^{2.10} NHR^{2.10.1} oder ein heterocyclischer nichtaromatischer C₃₋₁₀-Ring, gegebenenfalls substituiert mit C₁₋₄-Alkyl; bevorzugt ein heterocyclischer, nichtaromatischer C₃₋₁₀-Ring der ein, zwei oder drei Heteroatome, ausgewählt aus der Gruppe Sauerstoff und Stickstoff enthalten kann;
R^{2.10.1} H, Cyclopropyl oder C₁₋₆-Alkyl, gegebenenfalls substituiert mit O-C₁-₄-Alkyl;
- oder **R²**: C₆₋₁₀-Aryl, gegebenenfalls substituiert mit einem Rest ausgewählt aus der Gruppe bestehend aus Phenyl und einem heterocyclischen C₃₋₁₀-Ring, gegebenenfalls substituiert mit C₁₋₄-Alkyl, COOR^{2.8}, CN, Halogen, OR^{2.8} und NHCOMe, Oxo;
- oder **R²**: ein heterocyclischer, nichtaromatischer C₅₋₁₀-Ring, gegebenenfalls substituiert mit einem Rest ausgewählt aus der Gruppe bestehend aus Benzyl, COR^{2.11};
R^{2.11} ein Rest ausgewählt aus der Gruppe bestehend aus C₃₋₇-Cycloalkyl und einem heterocyclischen aromatischen oder nichtaromatischen C₅₋₁₀-Ring, gegebenenfalls substituiert mit einem oder mehreren C₁₋₄-Alkyl, gegebenenfalls substituiert mit OH, OMe, NH₂ oder NHCOO-^{t}Bu;
- oder **R²**: ein Rest ausgewählt aus der Gruppe bestehend aus C₂₋₆-Alkenyl, Indanyl, 1, 2, 3, 4-Tetrahydro-naphthalyl oder 8-Methyl-8-aza-bicyclo[3.2.1]octan;
- oder **NR¹R²**: bedeutet gemeinsam ein heterocyclischer, nichtaromatischer C₃₋₁₀-Ring der gegebenenfalls mit Methyl substituiert ist;
- **R³**: ein Rest ausgewählt aus der Gruppe bestehend aus ein heterocyclischer, aromatischer C₅₋₁₀-Ring, C₆₋₁₀-Aryl-C₁₋₆-alkylen, C₅₋₁₀-Heteroaryl-C₁₋₆-alkylen und CH₂-Benzo[1, 3]dioxolyl;
- oder **R³**: Phenyl, gegebenenfalls substituiert mit einem oder mehreren Resten ausgewählt aus der Gruppe bestehend aus C₁₋₄-Alkyl, C₁₋₄-Alkylen-NR^{3.1}R^{3.2}, CN, COOR^{3.1}, CONR^{3.1}R^{3.2}, CF₃, OR^{3.1}, Halogen, NHCOR^{3.1}, NO₂, SO₂NR^{3.1}R^{3.2}, C₁₋₄-Alkylen-NHCOR^{3.1};
R^{3.1} H oder C₁₋₄-Alkyl;
R^{3.2} H oder C₁₋₄-Alkyl;
- oder **R³**: eine Gruppe der Formel **1b** 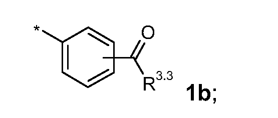 worin
R^{3.3} ein Rest ausgewählt aus der Gruppe bestehend aus einem heterocyclischen, nichtaromatischen C₃₋₁₀-Ring, gegebenenfalls substituiert mit einem oder mehreren Resten ausgewählt aus der Gruppe bestehend aus C₁₋₄-Alkyl, Oxo, COR^{3.3.1}, COR^{3.3.2}, C₁₋₄-Alkylen-R^{3.3.2}, CH₂CO-Pyrrolidin, heterocyclischer C₃₋₁₀-Ring, worin ein gegebenenfalls im heterocyclischen C₃₋₁₀-Ring enthaltenes Schwefelatom, gegebenenfalls als Oxid oder Dioxid vorliegen kann;
R^{3.3.1} C₁₋₄-Alkyl;
R^{3.3.2} NH₂, NH(C₁₋₄-Alkyl) oder N(C₁₋₄-Alkyl)₂;
oder R^{3.3} ein kondensierter, bicyclischer Heteroring oder heterocyclischer Spiroring;
- oder **R³**: bedeutet eine Gruppe der Formel **1c;** 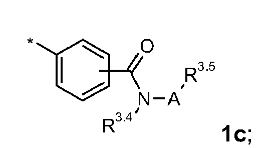 worin
A Bindung oder C₁₋₄-Alkyl, gegebenenfalls substituiert mit Oxo oder NMe₂;
R^{3.4} H oder C₁₋₄-Alkyl;
R^{3.5} ein Rest ausgewählt aus der Gruppe bestehend aus heterocyclischer C₃₋₁₀-Ring oder ein bicyclischer Ring, gegebenenfalls substituiert mit einem oder mehreren Resten unabhängig voneinander ausgewählt aus der Gruppe bestehend aus Oxo, C₁₋₄-Alkyl, OH, C₆₋₁₀-Aryl, heterocyclischer, aromatischer C₅₋₁₀-Ring, C₁₋₄-Alkylen-R^{3.5.1}, O-C₁₋₄-Alkylen-R^{3.5.1}, NH-C₁₋₄-Alkylen-R^{3.5.1};
R^{3.5.1} ein Rest ausgewählt aus der Gruppe bestehend aus C₆₋₁₀-Aryl, heterocyclischer C₃₋₁₀-Ring, gegebenenfalls substituiert mit C₁₋₄-Alkyl;
- oder **R³**: eine Gruppe der Formel **1d**; 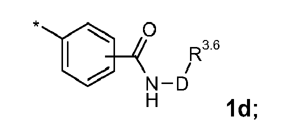 worin
D C₂₋₄-Alkinyl;
R^{3.6} Pyridinyl;
- oder **R³**: eine Gruppe COR^{3.7}, COCH₂R^{3.8}, CONHR^{3.8}, SO₂R^{3.8} oder eine Gruppe der Formel **1e**; 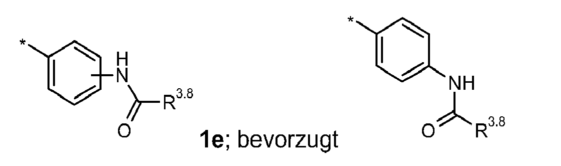 R^{3.7} H, C₁₋₄-Alkyl, C₆₋₁₀-Aryl;
R^{3.8} C₁₋₄-Alkyl, C₃₋₇-Cycloalkyl oder ein Rest ausgewählt aus der Gruppe bestehend aus C₆₋₁₀-Aryl, heterocyclischer C₅₋₁₀-Ring und ein bicyclischer Ring, gegebenenfalls substituiert mit einem oder mehreren Resten ausgewählt aus der Gruppe bestehend aus C₁₋₄-Alkyl, Halogen, NR^{3.8.1}R^{3.8.2}, C₆₋₁₀-Aryl und heterocyclischer, nichtaromatischer C₃₋₁₀-Ring;
R^{3.8.1} H oder C₁₋₄-Alkyl;
R^{3.8.2} H oder C₁₋₄-Alkyl;
- **R⁴**: H, Methyl oder Oxo;
- **R⁵**: H oder Methyl;
- **R⁶**: H oder Methyl;
- **R⁷**: H, Methyl, Phenyl oder OH;
- **R⁸**: H, Methyl, Phenyl oder OH;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

Bevorzugt sind die obigen Verbindungen der Formel **1,** worin
- **X**: O, S, SO oder SO₂; bevorzugt S, SO oder SO₂;
- **R¹**: H, Methyl, Ethyl oder Propyl; bevorzugt H und Propyl
- **R²**: H oder C₁₋₆-Alkyl, das gegebenenfalls mit einem oder mehreren Resten ausgewählt aus der Gruppe bestehend aus CF₃, CN, OH, NMe₂, OMe, COOH und CONMe₂ substituiert sein kann,
- oder **R²**: C₁₋₆-Alkyl, das gegebenenfalls mit einem oder mehreren Resten ausgewählt aus Cyclopropyl, Cyclopentyl, Cyclohexyl, Phenyl, Pyrrolidinyl, Imidazolidinyl, Pyrazolyl, Imidazolyl und Pyridinyl substitutiert sein kann, der gegebenenfalls mit Methyl oder Oxo substituiert sein kann;
- oder **R²**: C₃₋₇-Cycloalkyl, das gegebenenfalls mit einem Rest ausgewählt aus der Gruppe bestehend aus Methyl, OR^{2.3} ,CH₂OR^{2.3}, COOH, CONR^{2.3}R^{2.4}, CONH-^{t}Bu, O-Benzyl, NR^{2.3}R^{2.4} und NHCOR^{2.5} substituiert sein kann, wobei
R^{2.3} H oder Methyl oder 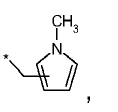 R^{2.4} H oder Methyl;
R^{2.5} CH₂C(CH₃)₃, CH₂C(CH₃)₂(CH₂OH), Cyclopentyl, Tetrahydrofuranyl, Pyrrolyl, Pyrazolyl, Imidazolyl oder Isoxazolyl;
- oder **R²**: eine Gruppe der Formel **1a**, 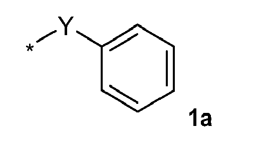 Y C₁₋₄-Alkylen, gegebenenfalls substituiert mit einem oder zwei R^{2.7}
R^{2.7} jeweils unabhängig voneinander C₁₋₄-Alkyl, COOH, CONH₂; oder R^{2.7} bildet gemeinsam mit einem oder zwei Kohlenstoffatomen von Y einen carbocyclischen Ring mit 3 Kohlenstoffatomen
- oder **R²**: C₆₋₁₀-Aryl, das gegebenenfalls mit einem oder mehreren Resten unabhängig voneinander ausgewählt aus der Gruppe bestehend aus Methyl, *tert*-Butyl, CN, F, Cl, Br, OH, OMe, OEt, O-Phenyl, COOH, COOMe, COR^{2.10}, NHCOMe und Morpholinyl-C₁₋₄-alkylen substituiert sein kann,
wobei
R^{2.10} NH₂, NHMe, NH-ⁱPr, NH-Cyclopropyl, NHCH₂CH₂OMe oder ein heterocyclischer, nichtaromatischer C₃₋₁₀-Ring, der ein, zwei oder drei Heteroatome ausgewählt aus der Gruppe Sauerstoff und Stickstoff enthalten kann; bevorzugt NH₂, NHMe, NH-ⁱPr, NH-Cyclopropyl, NHCH₂CH₂OMe, Morpholinyl oder Methyl-Piperazinyl;
- oder **R²**: C₆₋₁₀-Aryl, das gegebenenfalls mit einem Rest ausgewählt aus der Gruppe bestehend aus Phenyl und einem heterocyclischen C₃₋₁₀-Ring substituiert sein kann, welcher gegebenenfalls mit einem oder mehreren Resten aus der Gruppe bestehend aus Methyl, *tert*-Butyl, COOH, COOMe, CN, F, Cl, Br, OH, OMe, OEt und NHCOMe, Oxo substituiert sein kann; bevorzugt Phenyl, Imidazolidinyl, gegebenenfalls substituiert mit Methyl oder Oxo;
- oder **R²**: ein heterocyclischer nichtaromatischer C₃₋₁₀-Ring, der gegebenenfalls mit einem Rest ausgewählt aus der Gruppe bestehend aus Benzyl und COR^{2.11} substituiert sein kann;
wobei
R^{2.11} ein Rest ausgewählt aus der Gruppe bestehend aus Cyclopentyl, Tetrahydrofuranyl, Furan, Pyridyl, Pyrrolyl, Pyrrazolyl, Imidazolyl, der gegebenenfalls mit einer oder zwei Methylgruppen, oder mit einem oder mehreren Resten aus der Gruppe CH₂C(CH₃)₃, C(CH₃)₂(CH₂OH), CH₂OMe, C(CH₃)₂NH₂ und C(CH₃)₂NHCOO-^{t}Bu substituiert sein kann;
- oder **R²**: ein Rest ausgewählt aus der Gruppe bestehend aus C₂₋₆-Alkenyl, Indanyl, 1, 2, 3, 4-Tetrahydro-naphthalyl und 8-Methyl-8-aza-bicyclo[3.2.1]octan;
- oder **NR¹R²**: ein Rest ausgewählt aus der Gruppe bestehend aus Pyrrolidinyl, Piperidinyl, Piperazinyl und Morpholinyl, der gegebenenfalls mit Methyl substituiert sein kann;
- **R³** H: oder ein Rest ausgewählt aus der Gruppe bestehend aus Pyridinyl, Pyrimidinyl, Benzyl, und CH₂-Benzo[1, 3]dioxolyl;
- oder **R³**: Phenyl, das gegebenenfalls mit einem, zwei oder drei Resten ausgewählt aus der Gruppe bestehend aus Methyl, CH₂NH₂, CN, COOH, CONH₂, CF₃, OH, F, Cl, Br, OMe, NHCOMe, NR^{3.1}COR^{3.2}, CONR^{3.1}R^{3.2}, NO₂, SONMe₂ und CH₂NHCOMe substituiert sein kann;
wobei
R^{3.1} H , C₁₋₆-Alkyl oder ein gegebenenfalls überbrückter, mono- oder bicyclischer C₃₋₁₀-Heterocyclus
R^{3.2} H oder C₁₋₆-Alkyl;
- oder **R³**: eine Gruppe der Formel **1b** 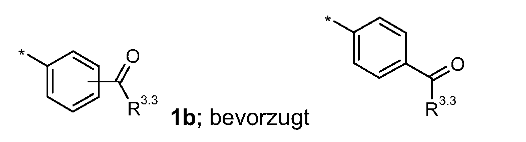 worin
**R^{3.3}** ein Rest ausgewählt aus der Gruppe bestehend aus Piperidinyl, Piperazinyl, Azepanyl, der gegebenenfalls mit einem oder mehreren Resten unabhängig voneinander ausgewählt aus der Gruppe bestehend aus Methyl, Oxo, COCH₃, CONH₂, CH₂NEt₂, CH₂CH₂NMe₂, CH₂CO-Pyrrolidin, Pyridinyl, Isothiazolidinyl-1, 1-dioxid und Thiazolidinyl-1, 1-dioxid substituiert sein kann;
oder R^{3.3} bedeutet einen Rest der Formel 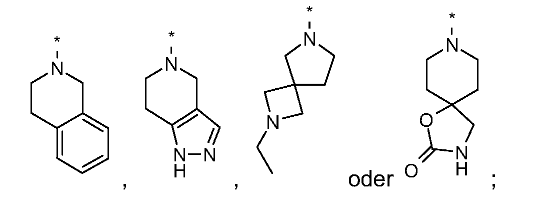
- oder **R³**: bedeutet eine Gruppe der Formel **1c;** 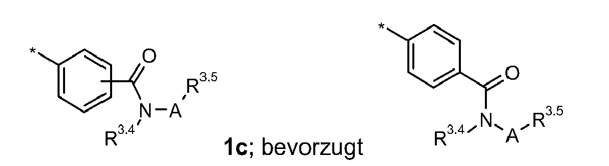 worin
A Bindung oder C₁₋₄-Alkyl, das gegebenenfalls mit Oxo oder mit NMe₂ substituiert sein kann,
R^{3.4} H oder Methyl;
R^{3.5} wobei ein Rest ausgewählt aus der Gruppe bestehend aus Pyrrolidinyl, Piperidinyl, Piperazinyl, Morpholinyl, Cyclohexyl, Imidazolyl, Pyrazolyl, Phenyl, Pyridinyl, Benzimidazolyl, Imidazolidin-2-on, Pyrrolidin-2-on, Pyrrolidin-3-on, Tetrahydro-thiophene 1, 1-dioxide und 1-Aza-bicyclo[2.2.2]octan, der gegebenenfalls mit einem oder mehreren Resten unabhängig voneinander ausgewählt aus der Gruppe bestehend aus Methyl, Ethyl, OH, Phenyl, Pyridinyl, Pyrazolyl, Pyrrolidinyl, (CH₂)ₒ-R^{3.5.1}, O-(CH₂)ₒ-R^{3.5.1} und NH-(CH₂)ₒ-R^{3.5.1}substituiert sein kann;
o 0, 1 oder 2
R^{3.5.1} ein Rest ausgewählt aus der Gruppe bestehend aus Phenyl, Pyrrolidinyl, Piperidinyl und Imidazolidin-2-on, der gegebenenfalls mit Methyl substituiert sein kann;
- oder **R³**: bedeutet eine Gruppe der Formel **1d;** 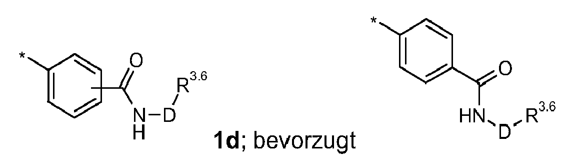 worin
D C₂₋₄-Alkinyl;
R^{3.6} Pyridinyl;
- oder **R³**: bedeutet eine Gruppe COR^{3.7}, COCH₂R^{3.8}, CONHR^{3.8}, SO₂R^{3.8} oder eine Gruppe der Formel **1e**; 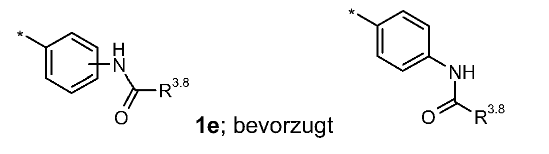 worin
R^{3.7} H, Methyl oder Phenyl;
R^{3.8} ein Rest ausgewählt ist aus der Gruppe bestehend aus *iso*-Propyl, Cyclopropyl, Cyclopentyl, Cyclohexyl, Pyrrolidinyl, Pyrrolidin-2-on, Furanyl und Azabicyclo[2.2.2]octanyl oder ein Rest ausgewählt ist aus der Gruppe bestehend aus Piperidinyl, Pyrazolyl, Imidiazolyl, Isoxazolyl, Pyridinyl, Phenyl, Benzyl, der gegebenenfalls mit einem oder mehreren Resten ausgewählt aus der Gruppe bestehend aus Methyl, Chlor, NH₂, NMe₂, Phenyl und Morpholinyl substituiert sein kann;
- **R⁴**: H, Methyl oder Oxo;
- **R⁵**: H oder Methyl;
- **R⁶**: H oder Methyl;
- **R⁷**: H, Methyl oder OH; bevorzugt H oder Methyl;
- **R⁸**: H, Methyl oder OH; bevorzugt H oder Methyl;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

Bevorzugt sind die obigen Verbindungen der Formel 1, worin
- X: S, SO oder SO₂;
- **R¹**: H, Methyl oder Ethyl;
- **R²**: ein Rest ausgewählt aus der Gruppe bestehend aus H, Ethyl, *n*-Propyl, *iso*-Propyl, *n*-Butyl, *n*-Pentyl, *n*-Hexyl, 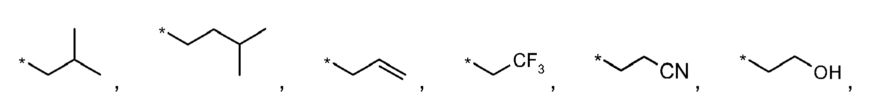 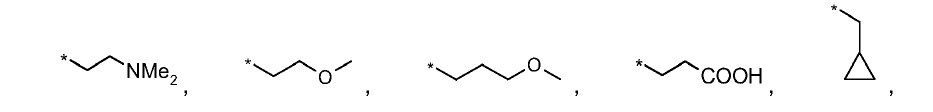 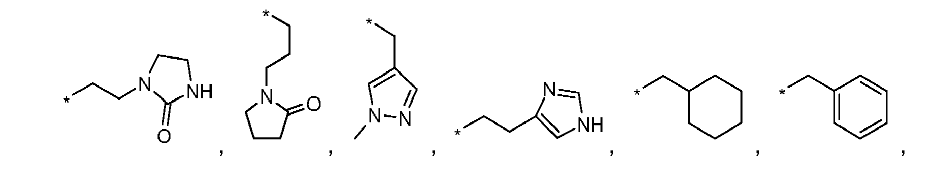 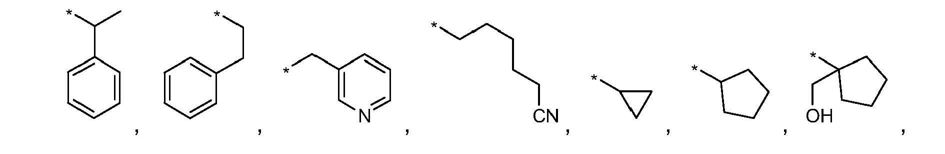 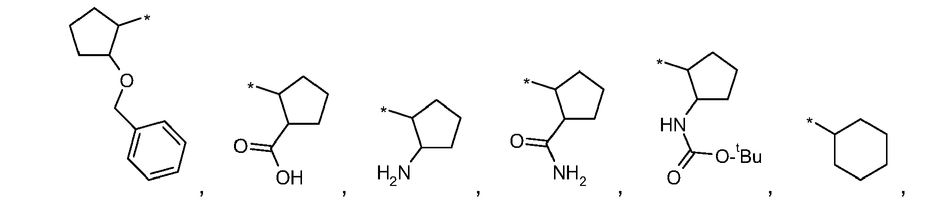 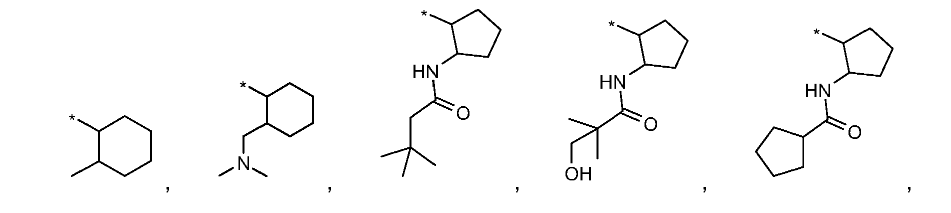 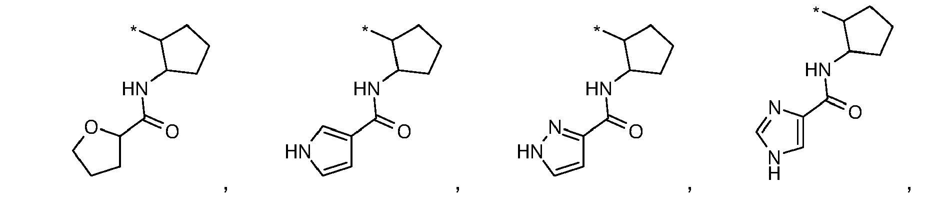 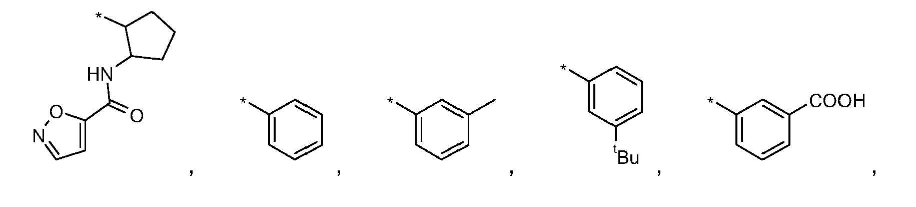 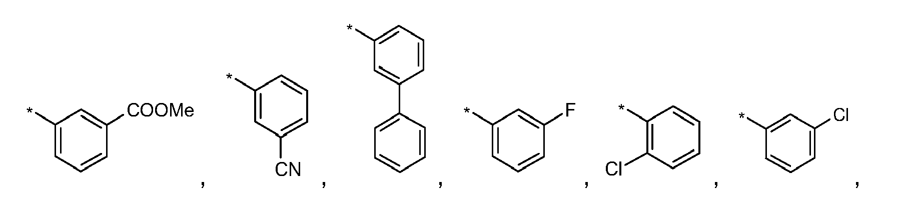 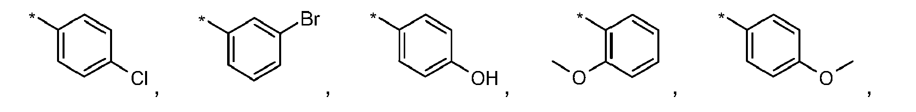 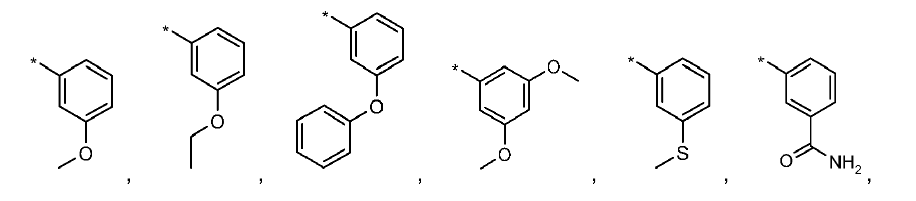 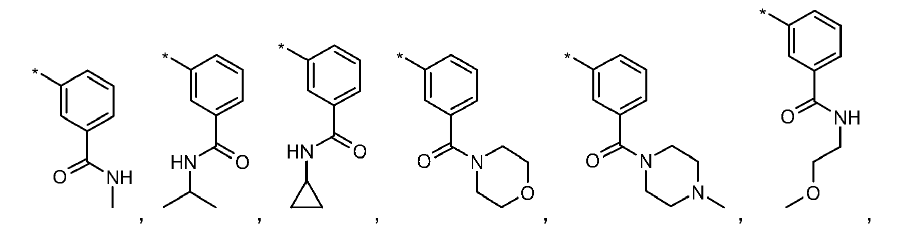 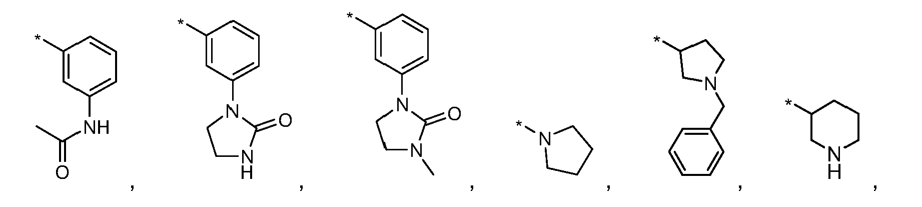 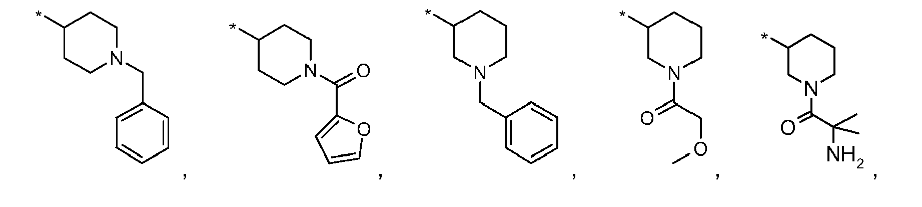 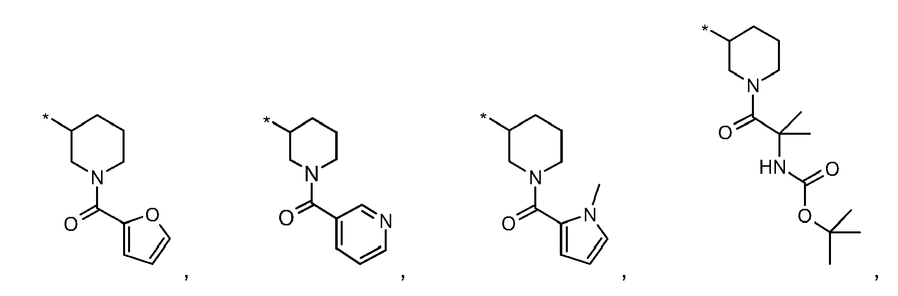 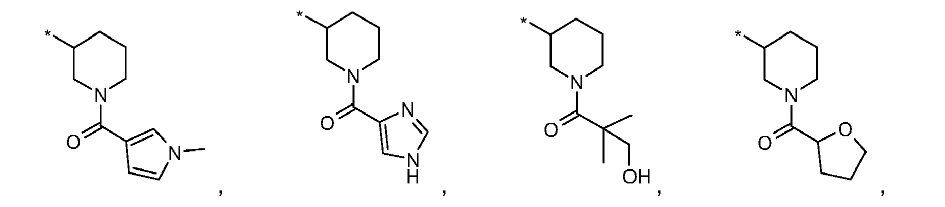 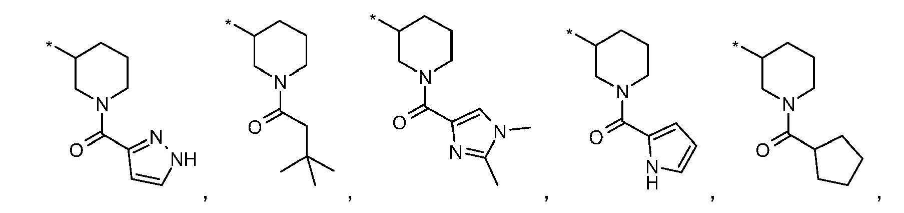 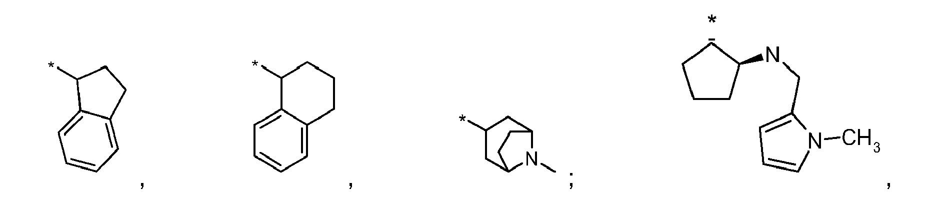 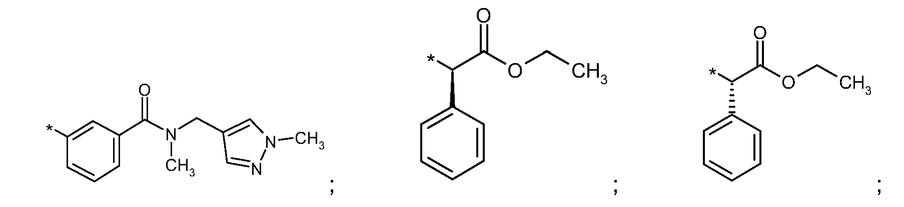 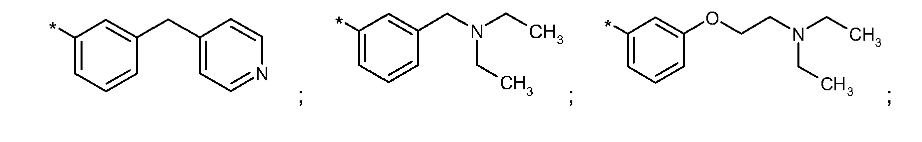 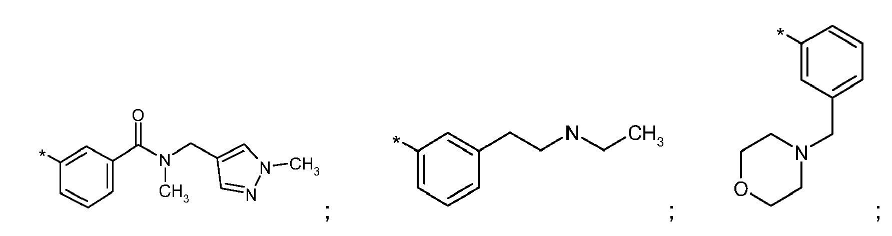 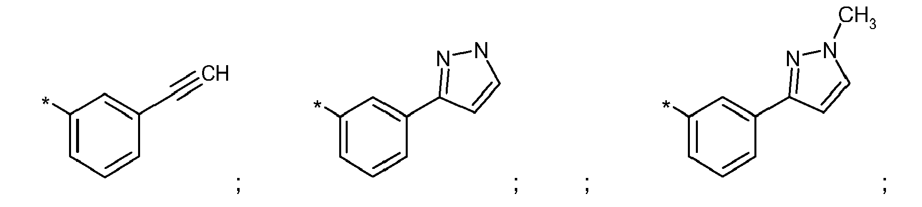 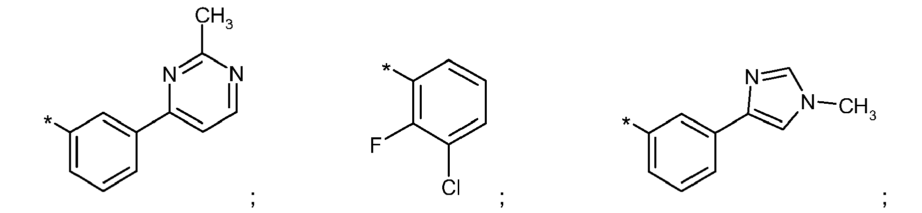 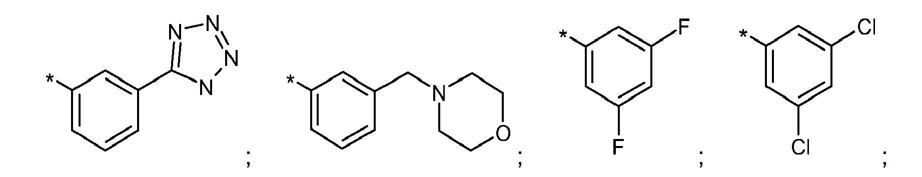 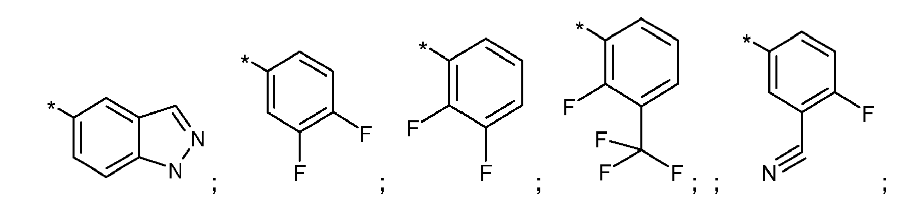 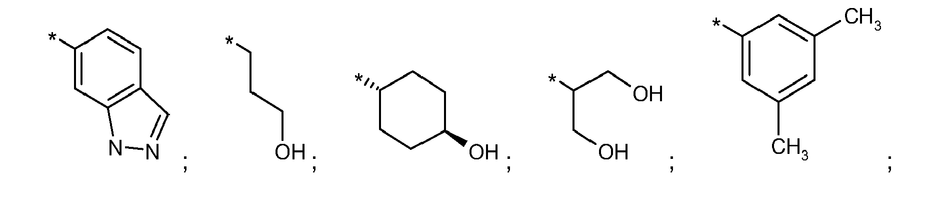 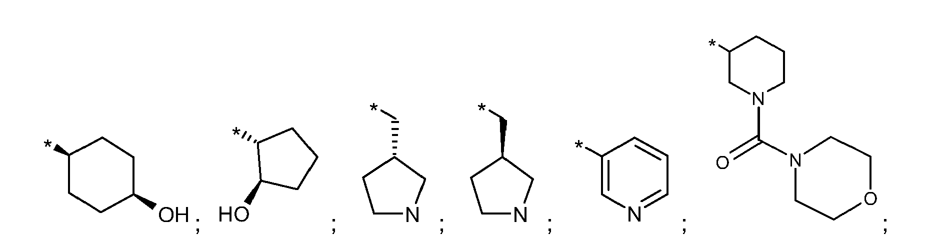 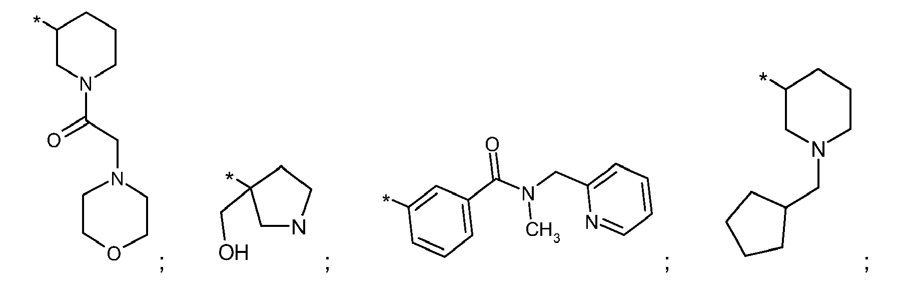 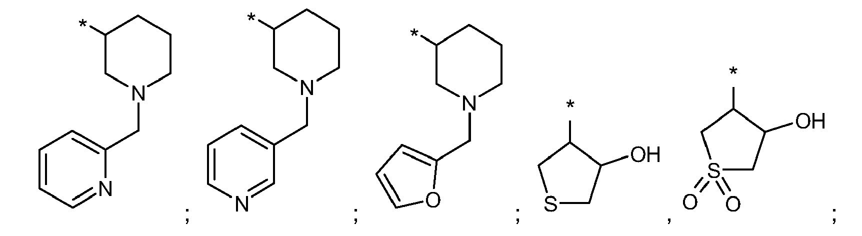 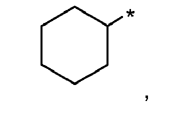
- oder **NR¹R²**: bedeutet ein Rest ausgewählt aus der Gruppe bestehend aus 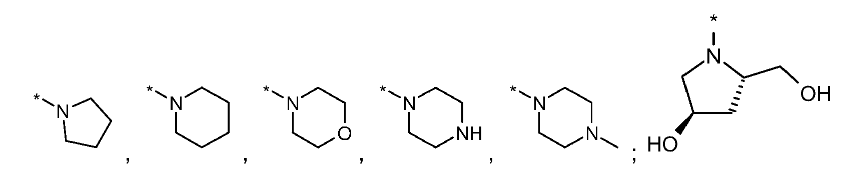
- **R³**: ein Rest ausgewählt aus der Gruppe bestehend aus 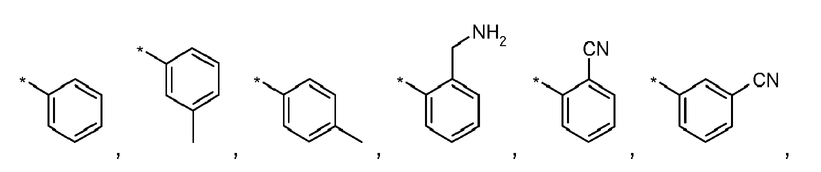 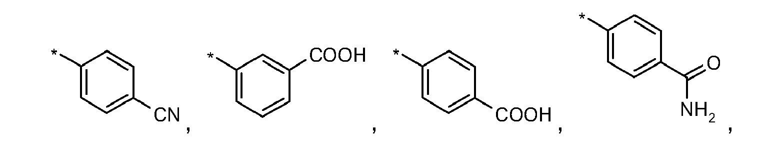 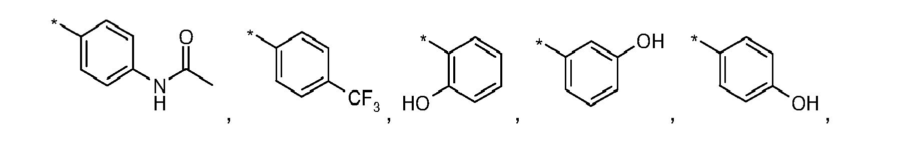 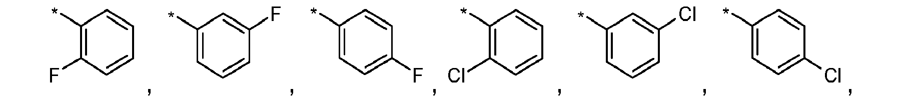 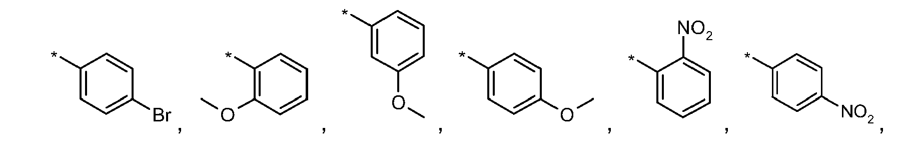 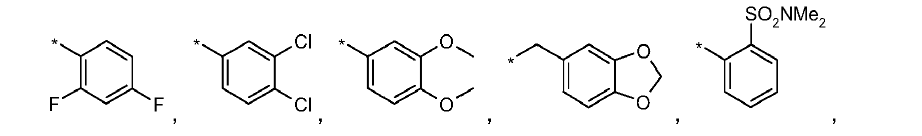 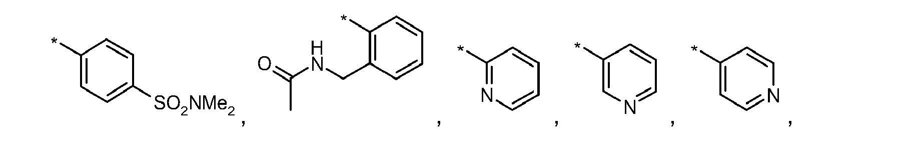 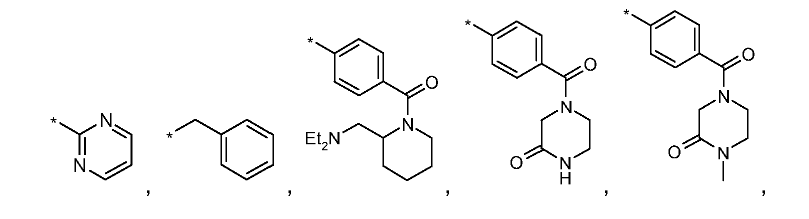 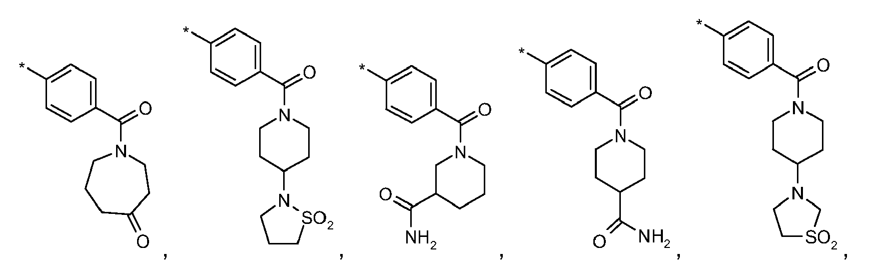 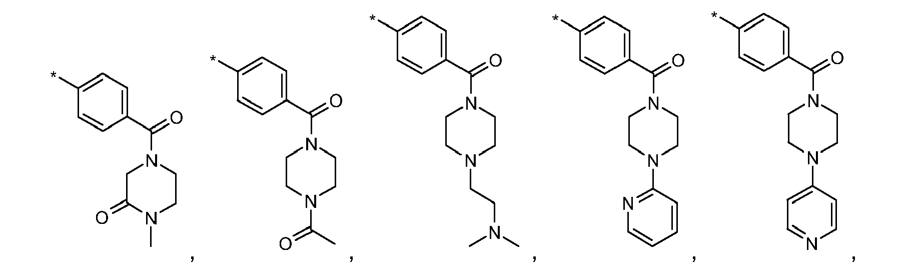 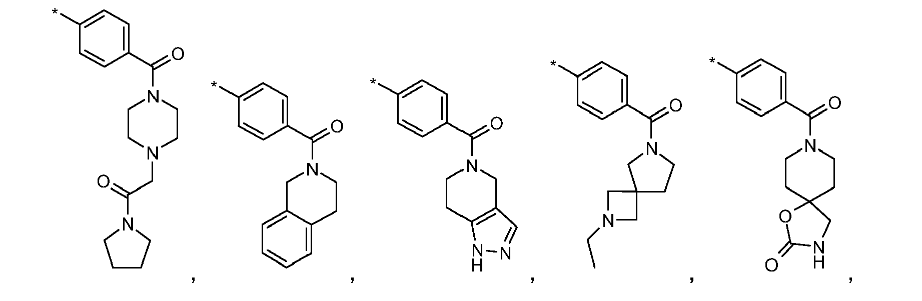 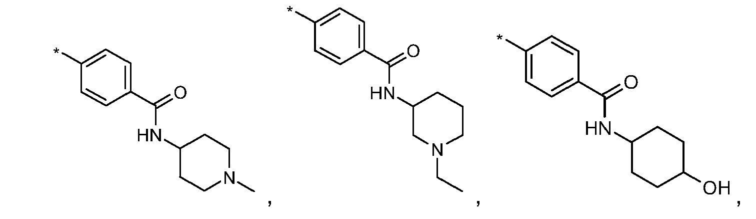 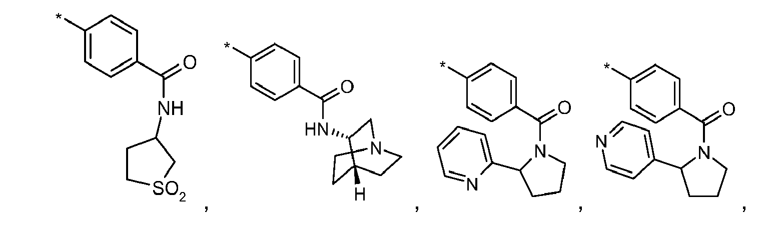 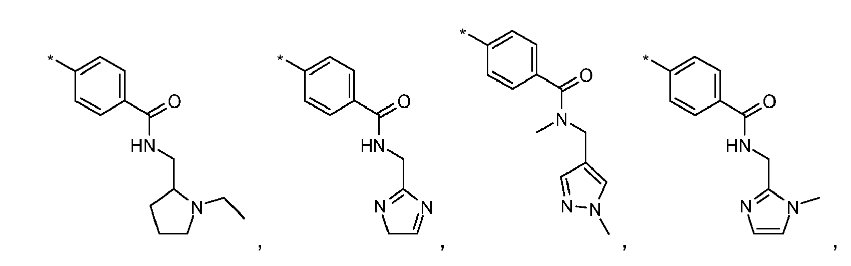 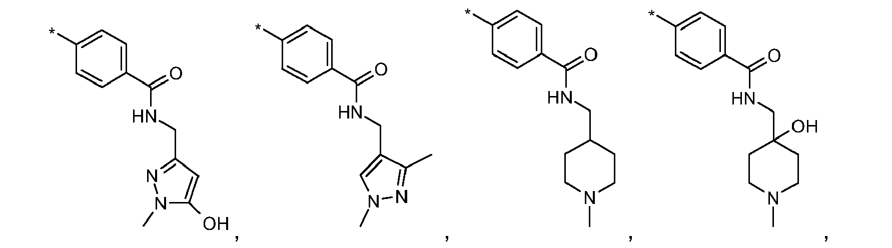 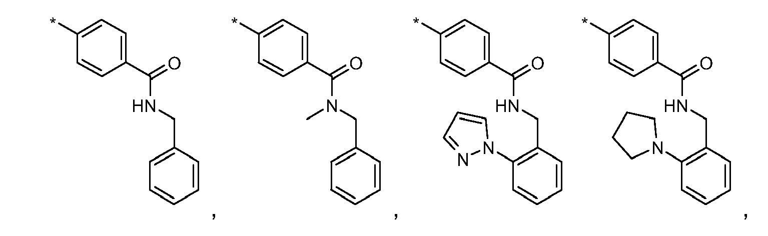 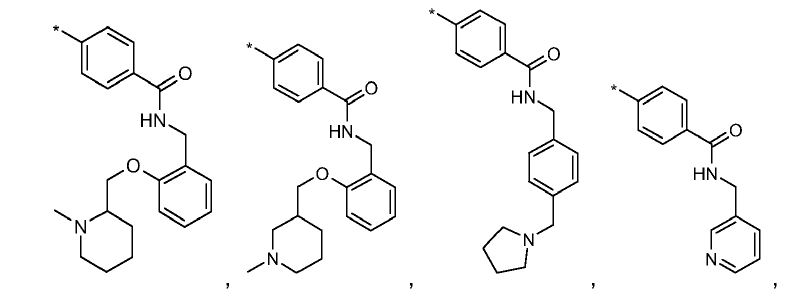 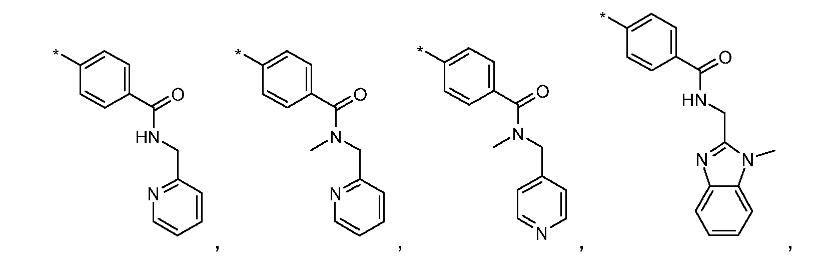 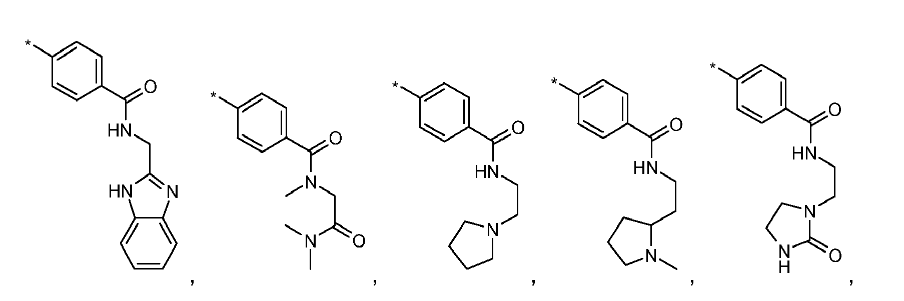 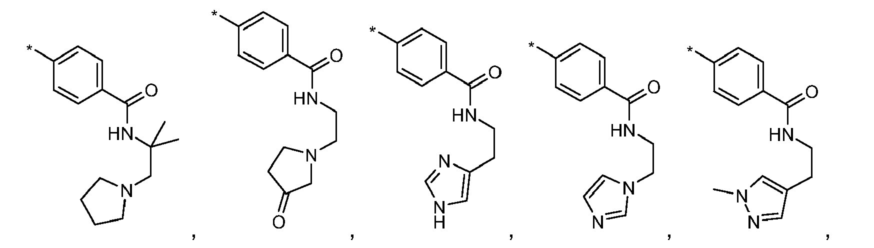 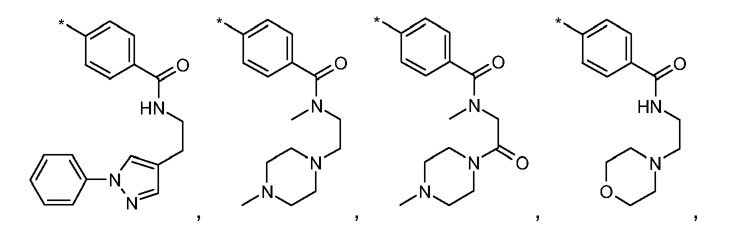 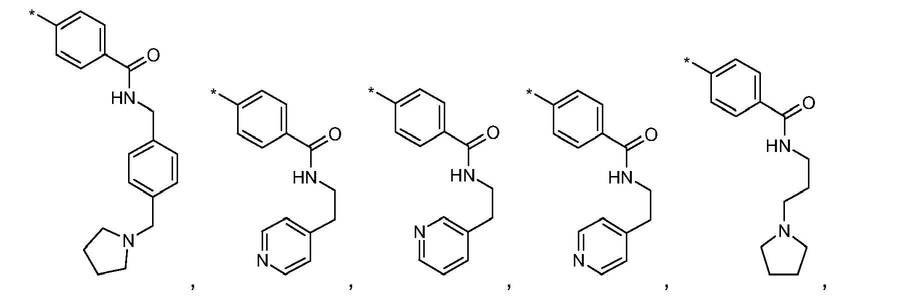 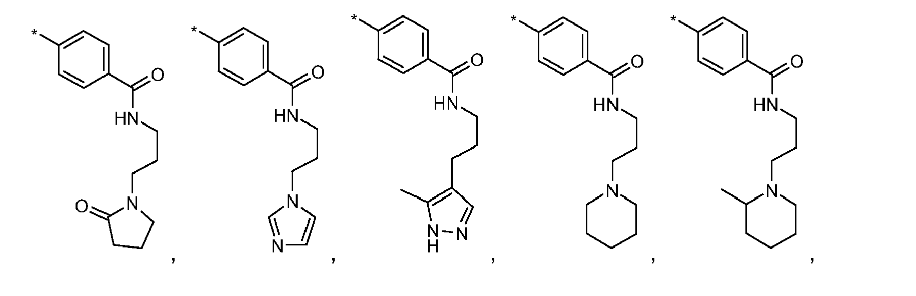 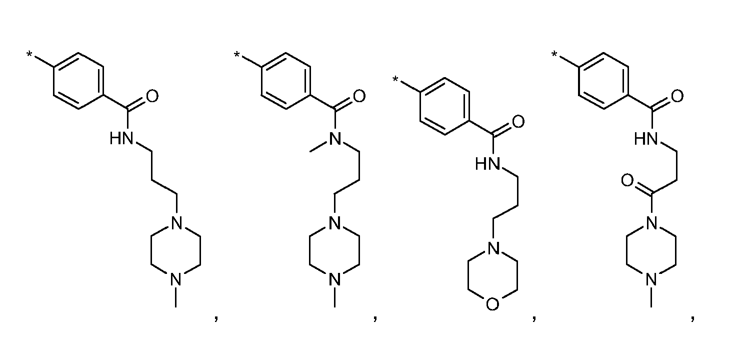 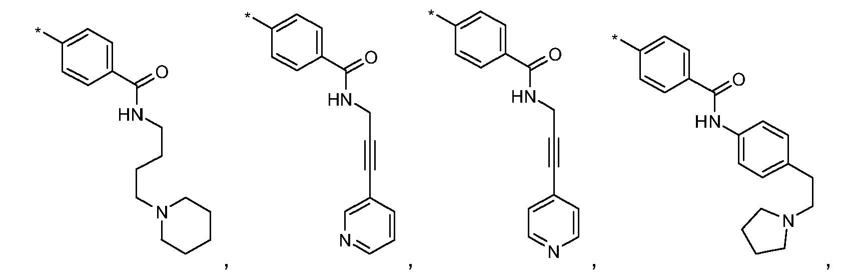 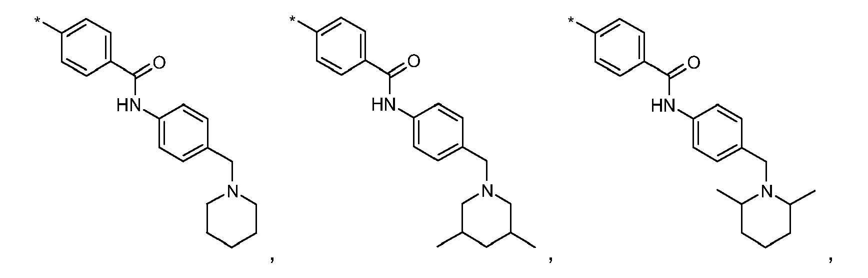 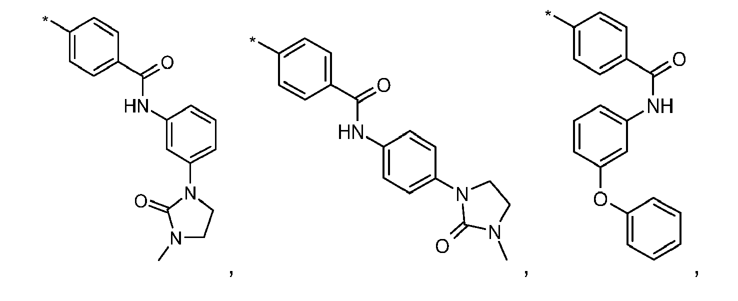 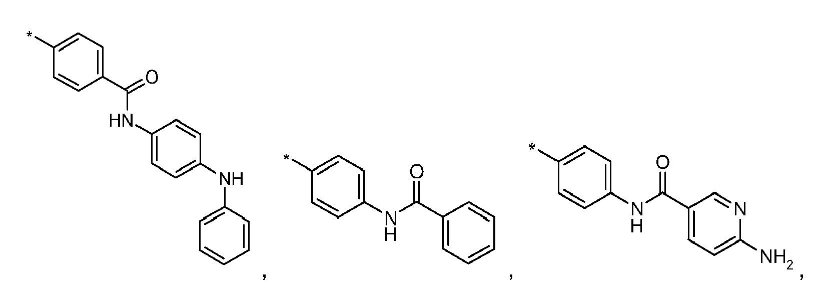 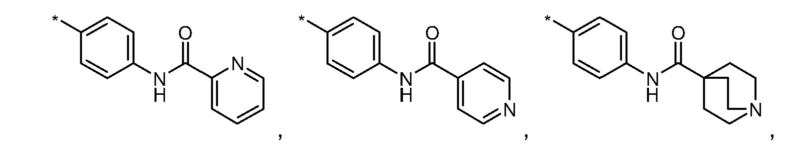 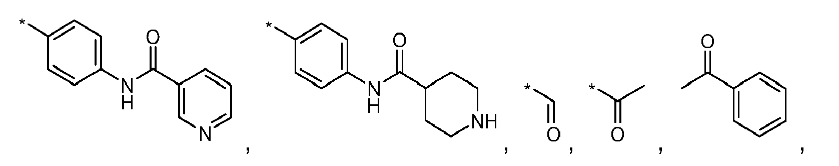 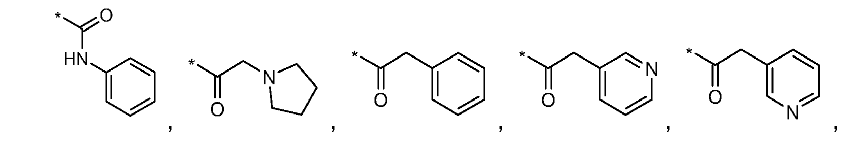 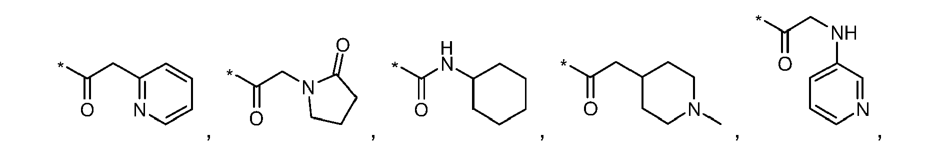 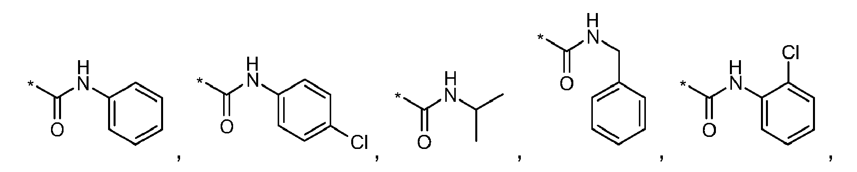 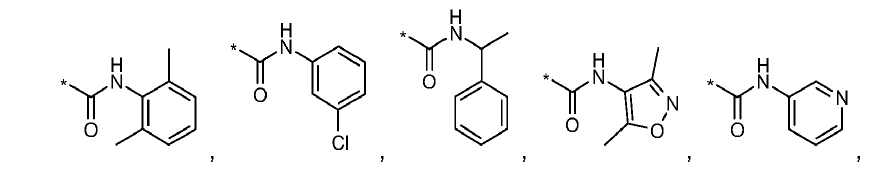 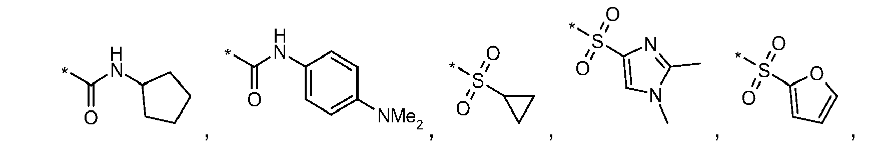 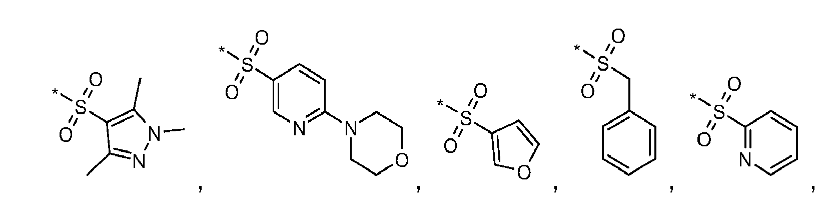 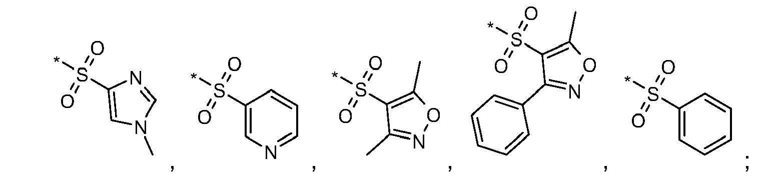 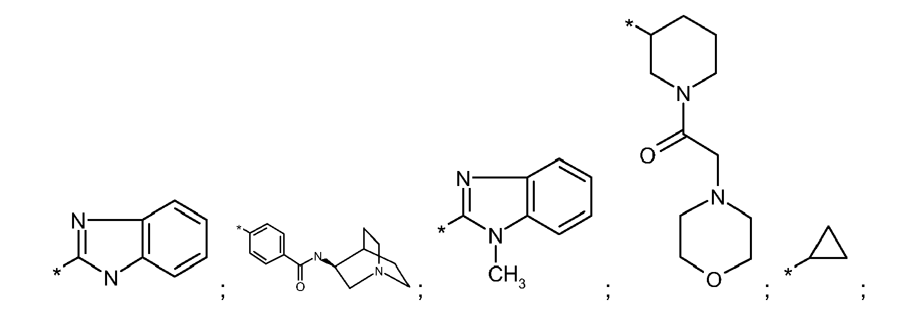
- **R⁴**: H, Methyl oder Oxo;
- **R⁵**: H oder Methyl;
- **R⁶**: H oder Methyl;
- **R⁷**: H oder Methyl;
- **R⁸**: H oder Methyl;
bedeuten, sowie pharmakologisch verträgliche Salze, Diastereomere, Enantiomere, Racemate, Hydrate oder Solvate davon.

Besonders bevorzugt sind die obigen Verbindungen der Formel 1, worin X, R², R³, R⁴, R⁵, R⁶, R⁷ und R⁸ die oben genannte Bedeutung haben und
- **R¹**: H;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin R¹, R², R³, R⁴, R⁵, R⁶, R⁷ und R⁸ die oben genannte Bedeutung haben und
- **X**: SO;
bedeutet, sowie pharmakologisch verträgliche Salze, Diastereomere, Enantiomere, Racemate, Hydrate oder Solvate davon.

Besonders bevorzugt sind die obigen Verbindungen der Formel 1, worin R¹, R², R³, R⁴ und X die oben genannte Bedeutung haben und
- **R⁵, R⁶, R⁷** und **R⁸**: H;
bedeutet, sowie pharmakologisch verträgliche Salze, Diastereomere, Enantiomere, Racemate, Hydrate oder Solvate davon.

Besonders bevorzugt sind die obigen Verbindungen der Formel 1, worin R¹, R², R³, R⁵, R⁶, R⁷ und R⁸ und X die oben genannte Bedeutung haben und
- **R⁴**: H;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin R¹, R³, R⁴, R⁵, R⁶, R⁷, R⁸ und X die oben genannte Bedeutung haben und
worin
- **R²**: C₆₋₁₀-Aryl, das gegebenenfalls mit einem oder mehreren Resten unabhängig voneinander ausgewählt aus der Gruppe bestehend aus C₁₋₆-Alkyl, C₁₋₄-Haloalkyl, C₂₋₆-Alkenyl, C₂₋₆-Alkinyl, CN, Halogen, OR^{2.8} COOR^{2.8}, COR^{2.10}, NR^{2.8}R^{2.9}, NHCOR^{2.8}, SR^{2.8}, SOR^{2.8}, SO₂R^{2.8} und SO₂NR^{2.8}R^{2.9} substituiert sein kann
- oder **R²**: C₆₋₁₀-Aryl, das gegebenenfalls mit einem oder mehreren Resten ausgewählt aus der Gruppe bestehend aus C₆₋₁₀-Aryl-C₁₋₆-alkylen, C₅₋₁₀-Heteroaryl-C₁₋₆-alkylen, C₆₋₁₀-Aryl und einem heterocyclischen C₃₋₁₀-Ring substituiert sein kann, der gegebenenfalls mit einem Rest ausgewählt aus der Gruppe bestehend aus C₁₋₆-Alkyl, C₁₋₆-Haloalkyl, COOR^{2.8}, CN, Halogen, OR^{2.8}, NHCOR^{2.8}, Oxo, ein C₃₋₇-Cycloalkyl-C₁₋₄ alkylen, ein Heterocyclus-C₁₋₄ alkylen und ein NR^{2.1}R^{2.2}-C₁₋₄-alkylen substituiert sein kann,
wobei
R^{2.8} H, C₁₋₆-Alkyl, C₂₋₆-Alkenyl, C₂₋₆-Alkinyl, ein NR^{2.1}R^{2.2}-C₁₋₄-alkylen oder C₆₋₁₀-Aryl;
R^{2.9} H, C₁₋₆-Alkyl, C₂₋₆-Alkenyl oder C₂₋₆-Alkinyl;
R^{2.10} NHR^{2.10.1} ,C₁₋₆-Alkylen-O-C₁₋₄-Alkyl, oder ein heterocyclischer C₃₋₁₀-Ring, der gegebenenfalls mit C₁₋₄-Alkyl substituiert sein kann; R^{2.10.1} H, C₁₋₆-Alkyl, C₂₋₆-Alkenyl, C₂₋₆-Alkinyl oder C₃₋₇-Cycloalkyl;
- oder **R²**: bedeutet C₆₋₁₀-Aryl, an das ein aromatischer oder nicht aromatischer C₃₋₁₀-Heterocyclus ankondensiert ist;
- oder **R²**: C₆₋₁₀-Aryl, das gegebenenfalls mit einem Rest ausgewählt aus der Gruppe bestehend aus C₆₋₁₀-Aryl und einem heterocyclischen C₃₋₁₀-Ring substituiert sein kann, der gegebenenfalls mit einem oder mehreren Resten aus der Gruppe C₁₋₆-Alkyl, C₂₋₆-Alkenyl, C₂₋₆-Alkinyl, C₁₋₄-Haloalkyl, CN, Halogen, OR^{2.8}, COOR^{2.8}, COR^{2.1} NR^{2.8}R^{2.9}, NHCOR^{2.8}, SR^{2.8}, SOR^{2.8}, SO₂R^{2.8}, SO₂NR^{2.8}R^{2.9} und Oxo substituiert sein kann;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und
- **R²**: bedeutet C₆₋₁₀-Aryl, gegebenenfalls substituiert mit einem oder mehreren Resten unabhängig voneinander ausgewählt aus der Gruppe bestehend aus C₁₋₆-Alkyl, C₂₋₆-Alkenyl, C₂₋₆-Alkinyl, C₁₋₄-Haloalkyl, CN, Halogen, OR^{2.8}, COOR^{2.8}, COR^{2.10} NR^{2.8}R^{2.9}, NHCOR^{2.8}, SR^{2.8}, SOR^{2.8}, SO₂R^{2.8} und SO₂NR^{2.8}R^{2.9}
R^{2.8} H, C₁₋₆-Alkyl, C₂₋₆-Alkenyl, C₂₋₆-Alkinyl oder C₆₋₁₀-Aryl;
R^{2.9} H, C₁₋₆-Alkyl, C₂₋₆-Alkenyl oder C₂₋₆-Alkinyl;
R^{2.10} NHR^{2.10.1} C₁₋₆-Alkylen-O-C₁₋₄-Alkyl, oder ein heterocyclischer C₃₋₁₀-Ring, gegebenenfalls substituiert mit C₁₋₄-Alkyl;
R^{2.10.1} H, C₁₋₆-Alkyl, C₂₋₆-Alkenyl, C₂₋₆-Alkinyl oder C₃₋₇-Cycloalkyl;
- oder **R²**: bedeutet C₆₋₁₀-Aryl, gegebenenfalls substituiert mit einem Rest ausgewählt aus der Gruppe bestehend aus C₆₋₁₀-Aryl und einem heterocyclischen C₃₋₁₀-Ring, gegebenenfalls substituiert mit C₁₋₆-Alkyl, C₂₋₆-Alkenyl, C₂₋₆-Alkinyl, C₁₋₄-Haloalkyl, CN, Halogen, OR^{2.8}, COOR^{2.8}, COR^{2.10} NR^{2.8}R^{2.9}, NHCOR^{2.8}, SR^{2.8}, SOR^{2.8}, SO₂R^{2.8}, SO₂NR^{2.8}R^{2.9} und Oxo;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und
- **R²**: C₆₋₁₀-Aryl, das gegebenenfalls mit einem oder mehreren Resten ausgewählt aus der Gruppe bestehend aus C₁₋₄-Alkyl, CN, Halogen, OR^{2.8}, COOR^{2.8}, COR^{2.10} und NHCOMe substituiert sein kann
worin
R^{2.8} C₁₋₄-Alkyl oder C₆₋₁₀-Aryl;
R^{2.10} NHR^{2.10.1}, Morpholinyl, Methyl-Piperazinyl;
R^{2.10.1} H, Cyclopropyl oder C₁₋₄-Alkyl, das gegebenenfalls mit einem oder mehreren Resten ausgewählt aus O-C₁₋₄-Alkyl, OH oder C₆₋₁₀-Aryl substituiert sein kann;
- oder **R²**: C₆₋₁₀-Aryl, das gegebenenfalls mit einem Rest ausgewählt aus der Gruppe bestehend aus Phenyl und einem heterocyclischen C₃₋₁₀-Ring substituiert sein kann, der gegebenenfalls mit C₁₋₄-Alkyl, COOR^{2.8}, CN, Halogen, OR^{2.8}, NHCOMe und Oxo substituiert sein kann;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und worin
- **R²**: C₆₋₁₀-Aryl, gegebenenfalls substituiert mit einem oder mehreren Resten unabhängig voneinander ausgewählt aus der Gruppe bestehend aus Methyl, *tert*-Butyl, CN, F, Cl, Br, OH, OMe, OEt, O-Phenyl, COOH, COOMe, COR^{2.10} und NHCOMe
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und **R²** 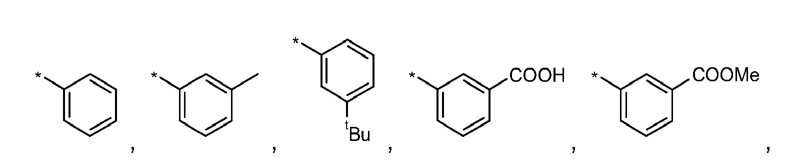 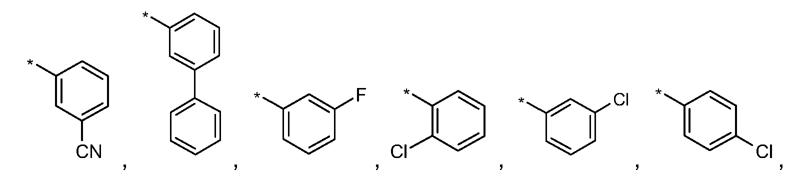 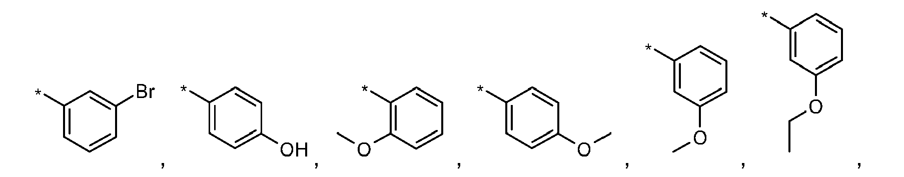 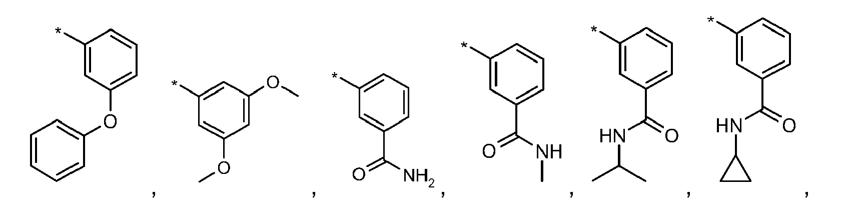 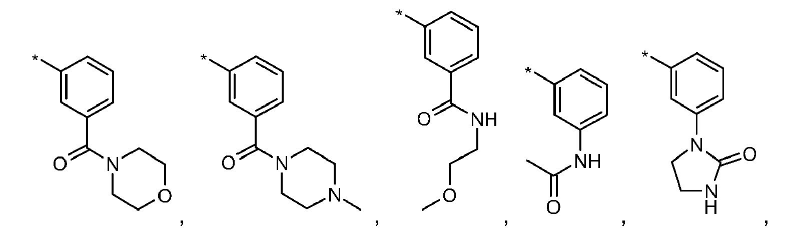 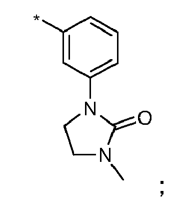 bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und
- **R²**: H;
bedeutet, sowie pharmakologisch verträgliche Salze, Diastereomere, Enantiomere, Racemate, Hydrate oder Solvate davon.

Besonders bevorzugt sind die obigen Verbindungen der Formel 1, worin X, R¹, R³, R⁴, R⁵, R⁶, R⁷ und R⁸ die oben genannte Bedeutung haben und worin
- **R²**: ein Rest ausgewählt aus der Gruppe bestehend aus C₁₋₆-Alkyl, C₂₋₆-Alkenyl und C₂₋₆-Alkinyl; bevorzugt C₁₋₆-Alkyl; besonders bevorzugt Propyl, der gegebenenfalls mit einem Rest ausgewählt aus der Gruppe bestehend aus C₁₋₆-Haloalkyl, CN, OR^{2.1}, NR^{2.1}R^{2.2}, COOR^{2.1}, NHCOR^{2.1}, SR^{2.1}, SOR^{2.1}, SO₂R^{2.1}, und CONR^{2.1}R^{2.2}, vorzugsweise mit einem Rest ausgewählt aus der Gruppe C₁₋₄-Haloalkyl, CN, OR^{2.1}, NR^{2.1}R^{2.2}, COOR^{2.} und CONR^{2.1}R^{2.2} substituiert sein kann oder der gegebenenfalls mit einem Rest ausgewählt aus der Gruppe bestehend aus C₃₋₇-Cycloalkyl, C₆₋₁₀-Aryl und einem heterocyclischen, aromatischen C₃₋₁₀-Ring substituiert sein kann, welcher gegebenenfalls wiederum mit C₁₋₆-Alkyl, C₂₋₆-Alkenyl, C₂₋₆-Alkinyl oder Oxo; bevorzugt mit Methyl oder Oxo substituiert sein kann;
worin
R^{2.1} H oder ausgewählt aus der Gruppe bestehend aus C₁₋₄-Alkyl, C₂₋₆-Alkenyl oder C₂₋₆-Alkinyl, vorzugsweise H oder C₁₋₄-Alkyl;
R^{2.2} H oder ausgewählt aus der Gruppe bestehend aus C₁₋₄-Alkyl, C₂₋₆-Alkenyl oder C₂₋₆-Alkinyl, vorzugsweise H oder C₁₋₄-Alkyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷und R⁸ die oben genannte Bedeutung haben und
- **R²**: ein Rest ausgewählt aus der Gruppe bestehend aus C₁₋₆-Alkyl, C₂₋₆-Alkenyl und C₂₋₆-Alkinyl; bevorzugt C₁₋₆-Alkyl; besonders bevorzugt Propyl, gegebenenfalls substituiert mit einem Rest ausgewählt aus der Gruppe bestehend aus C₁₋₆-Haloalkyl, CN, OR^{2.1}, NR^{2.1}R^{2.2}, NHCOR^{2.1}, SR^{2.1}, SOR^{2.1}, SO₂R^{2.1}, SO₂NR^{2.1}R^{2.2}, COOR^{2.1} und CONR^{2.1}R^{2.2} oder gegebenenfalls substituiert mit ein Rest ausgewählt aus der Gruppe bestehend aus C₃₋₇-Cyclloalkyl, C₆₋₁₀-Aryl und einem heterocyclischen C₃₋₁₀-Ring, gegebenenfalls substituiert mit C₁₋₆-Alkyl, C₂₋₆-Alkenyl, C₂₋₆-Alkinyl oder Oxo;
R^{2.1} H, C₁₋₆-Alkyl, C₂₋₆-Alkenyl oder C₂₋₆-Alkinyl;
R^{2.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und
- **R**²: C₁₋₆-Alkyl, das gegebenenfalls mit einem Rest ausgewählt aus der Gruppe bestehend aus C₁₋₄-Haloalkyl, CN, OR^{2.1}, NR^{2.1}R^{2.2}, COOR^{2.1} und CONR^{2.1}R^{2.2} substituiert sein kann oder das gegebenenfalls mit einem Rest ausgewählt aus der Gruppe bestehend aus C₃₋₇Cycloalkyl, C₆₋₁₀-Aryl und einem heterocyclischen, aromatischen C₃₋₁₀-Ring substituiert sein kann, welcher gegebenenfalls wiederum mit Methyl oder Oxo substituiert sein kann;
worin
R^{2.1} H oder C₁₋₄-Alkyl;
R^{2.2} H oder C₁₋₄-Alkyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und
- **R**²: C₁₋₆-Alkyl; bevorzugt Propyl; besonders bevorzugt *n*-Propyl, gegebenenfalls substituiert mit einem Rest ausgewählt aus der Gruppe bestehend aus CF₃, CN, OH, NMe₂, OMe, COOH und CONMe₂ oder gegebenenfalls substituiert mit einem Rest ausgewählt aus der Gruppe bestehend aus Cyclopropyl, Cyclopentyl, Cyclohexyl, Phenyl, Pyrrolidinyl, Imidazolidinyl, Pyrazolyl, Imidazolyl und Pyridinyl, gegebenenfalls substituiert mit Methyl oder Oxo;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und worin
- **R**²: ein Rest ausgewählt aus der Gruppe bestehend aus Ethyl, *n*-Propyl, *iso*-Propyl, *n*-Butyl, *n*-Pentyl, *n*-Hexyl, 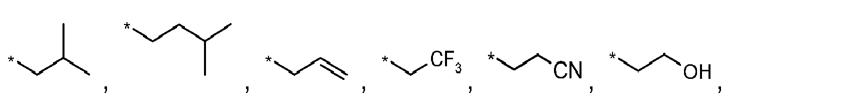 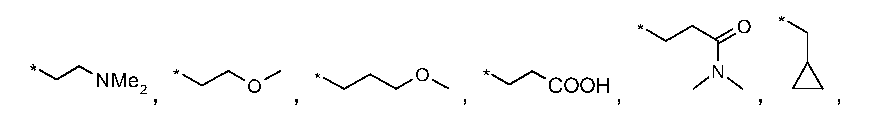 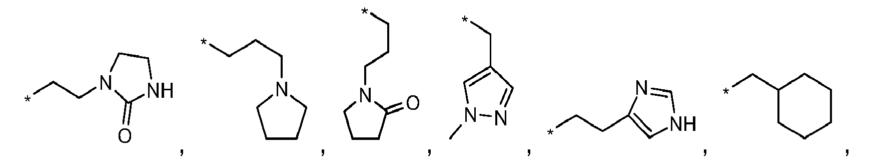 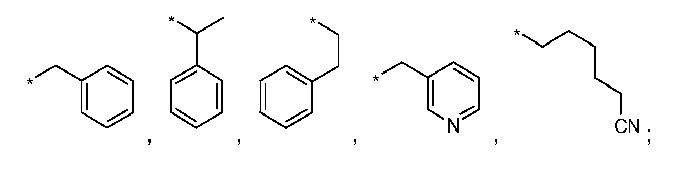
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R³, R⁴, R⁵, R⁶, R⁷ und R⁸ die oben genannte Bedeutung haben und worin
- **R**²: C₆₋₁₀-Aryl, das gegebenenfalls in meta-Position mit einem oder mehreren Resten unabhängig voneinander ausgewählt aus der Gruppe bestehend aus C₁₋₄-Alkyl, CN, Halogen, OR^{2.8}, COOR^{2.8}, COR^{2.10} und NHCOMe substituiert sein kann
wobei
R^{2.8} C₁₋₄-Alkyl oder C₆₋₁₀-Aryl;
R^{2.10} NHR^{2.10.1}, Morpholinyl, Methyl-Piperazinyl;
R^{2.10.1} H, Cyclopropyl oder C₁₋₄-Alkyl, wobei das C₁₋₄-Alkyl gegebenenfalls mit einem oder mehreren Resten ausgewählt aus der Gruppe aus O-C₁₋₄-Alkyl, OH und C₆₋₁₀-Aryl substituiert sein kann;
- oder **R**²: NH(R^{2.10.1}), Cyclohexyl
- oder **NR¹R²**: ein heterocyclischer C₅₋₆-Ring ausgewählt aus der Gruppe bestehend aus Pyrrolidin und Piperazin, der gegebenenfalls mit einem oder mehreren Resten ausgewählt aus der Gruppe bestehend aus C₁₋₄-Alkyl, OH und C₁₋₄-Alkanol substituiert sein kann
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin
- **R**³: Phenyl, das gegebenenfalls mit einem oder mehreren Resten ausgewählt aus der Gruppe bestehend aus C₁₋₆-Alkyl, C₂₋₆-Alkenyl, C₂₋₆-Alkinyl, C₁₋₄-Haloalkyl, C₁₋₆-Alkylen-NR^{3.1}R^{3.2}, CN, Halogen, OR^{3.1}, COOR^{3.1}, CONR^{3.1}R^{3.2}, NR^{3.1}R^{3.2}, NHCOR^{3.1}, NO₂, SR^{3.1}, SOR^{3.1}, SO₂R^{3.1}, SO₂NR^{3.1}R^{3.2} und C₁₋₆-Alkylen-NHCOR^{3.1} substituiert sein kann;
worin
R^{3.1} H, C₁₋₆-Alkyl, C₂₋₆-Alkenyl oder C₂₋₆-Alkinyl;
R^{3.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin
- **R**³: Phenyl, welches gegebenenfalls in para-Position mit einem Rest ausgewählt aus der Gruppe bestehend aus C₁₋₆-Alkyl, C₂₋₆-Alkenyl, C₂₋₆-Alkinyl, C₁₋₄-Haloalkyl, C₁₋₆-alkylen-NR^{3.1}R^{3.2}, CN, Halogen, OR^{3.1}, COOR^{3.1}, CONR^{3.1}R^{3.2}, NR^{3.1}R^{3.2}, NHCOR^{3.1}, NO₂, SR^{3.1}, SOR^{3.1}, SO₂R^{3.1}, SO₂NR^{3.1}R^{3.2} und C₁₋₆-alkylen-NHCOR^{3.1} substituiert sein kann,
worin
R^{3.1} H, C₁₋₆-Alkyl, C₂₋₆-Alkenyl oder C₂₋₆-Alkinyl;
R^{3.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und
- **R**³: bedeutet Phenyl, das gegebenenfalls mit einem oder mehreren Resten ausgewählt aus der Gruppe bestehend aus C₁₋₄-Alkyl, C₁₋₄-Alkylen-NR^{3.1}R^{3.2}, CN, COOR^{3.1}, CONR^{3.1}R^{3.2} CF₃ OR^{3.1}, Halogen, NHCOR^{3.1}, NO₂, SO₂NR^{3.1}R^{3.2} und C₁₋₄-Alkylen-NHCOR^{3.1} substituiert sein kann;
worin
R^{3.1} H oder C₁₋₄-Alkyl;
R^{3.2} H oder C₁₋₄-Alkyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und
- **R**³: Phenyl, das gegebenenfalls mit einem, zwei oder drei Resten ausgewählt aus der Gruppe bestehend aus Methyl, CH₂NH₂, CN, COOH, CONH₂, CF₃, OH, F, Cl, Br, OMe, NHCOMe, NO₂, SONMe₂ und CH₂NHCOMe substituiert sein kann;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin **R**³ eine Gruppe der Formel **1b** 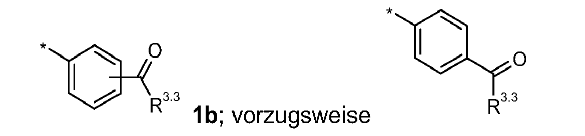 bedeutet, worin
- R^{3.3}: ein heterocyclischer C₃₋₁₀-Ring, der gegebenenfalls mit einem oder mehreren Resten ausgewählt aus der Gruppe bestehend aus C₁₋₆-Alkyl, Oxo, COR^{3.3.1}, COR^{3.3.2}, C₁₋₆-Alkylen-R^{3.3.2}, CH₂CO-Pyrrolidin und einem heterocyclischen C₃₋₁₀-Ring substituiert sein kann, worin ein gegebenenfalls im heterocyclischen C₃₋₁₀-Ring enthaltenes Schwefelatom gegebenenfalls auch als Oxid oder Dioxid vorliegen kann;
wobei
R^{3.3.1} C₁₋₆-Alkyl;
R^{3.3.2} NH₂, NH(C₁₋₆-Alkyl) oder N(C₁₋₆-Alkyl)₂;
- oder R^{3.3}: ein bicyclischer Ring oder ein heterocyclischer Spiroring;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin **R**³ eine Gruppe der Formel **1b** 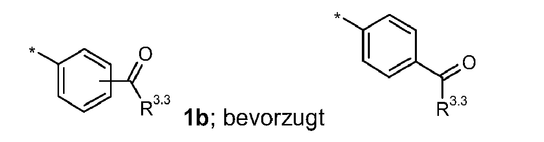 worin
- R^{3.3}: ein Rest ausgewählt aus der Gruppe bestehend aus Piperidinyl, Piperazinyl und Azepanyl, der gegebenenfalls mit einem oder mehreren Resten ausgewählt aus der Gruppe bestehend aus Methyl, Oxo, COCH₃, CONH₂, CH₂NEt₂, CH₂CH₂NMe₂, CH₂CO-Pyrrolidin, Pyridinyl, Isothiazolidinyl-1, 1-dioxid und Thiazolidinyl-1, 1-dioxid substituiert sein kann;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin **R**³ eine Gruppe der Formel **1c;** 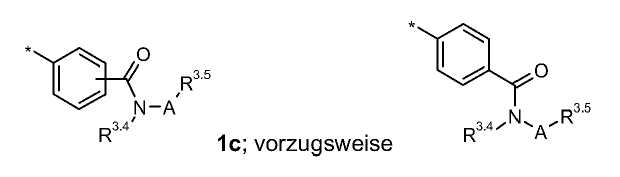 worin
- A: Bindung oder C₁₋₆-Alkyl, das gegebenenfalls mit Oxo oder NMe₂ substituiert sein kann;
- R^{3.4}: H oder C₁₋₆-Alkyl;
- R^{3.5}: ein Rest ausgewählt aus der Gruppe bestehend aus C₁₋₆-Alkyl, das gegebenenfalls durch einen Rest ausgewählt aus der Gruppe bestehend aus C₃₋₇Cycloalkyl, C₆₋₁₀-Aryl und einem C₅₋₁₀-Heterocyclus substituiert sein kann, welcher jeweils auch gegebenenfalls mit einem oder mehreren Resten ausgewählt aus der Gruppe aus Halogen, OH, Oxo, C₁₋₆-Alkyl, O-C₁₋₆-Alkyl, C₁₋₆-Haloalkyl, einem heterocyclischen C₃₋₁₀-Ring und einem bicyclischen Ring substituiert sein kann, wobei diese Reste jeweils gegebenenfalls mit einem oder mehreren Resten ausgewählt aus der Gruppe bestehend aus Oxo, C₁₋₆-Alkyl, OH, C₆₋₁₀-Aryl, einem heterocyclischen Ring, C₁₋₆-Alkylen-R^{3.5.1}, O-C₁₋₆-Alkylen-R^{3.5.1} und NH-C₁₋₆-Alkylen-R^{3.5.1} substituiert sein können,
- oder R^{3.5}: ein Rest ausgewählt aus der Gruppe bestehend aus heterocyclischer oder bicyclischer Ring, gegebenenfalls substituiert mit einem oder mehreren Resten unabhängig voneinander ausgewählt aus der Gruppe bestehend aus Oxo, C₁₋₆-Alkyl, OH, Aryl, ein heterocyclischer Ring, C₁₋₆-Alkylen-R^{3.5.1}, OC₁-₆-Alkylen-R^{3.5.1}, NH-C₁₋₆-Alkylen-R^{3.5.1};
wobei
R^{3.5.1} ein Rest ausgewählt aus der Gruppe bestehend aus C₆₋₁₀-Aryl und heterocyclischer C₃₋₁₀-Ring, der gegebenenfalls mit C₁₋₆-Alkyl substituiert sein kann ;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin **R**³ eine Gruppe der Formel **1c;** 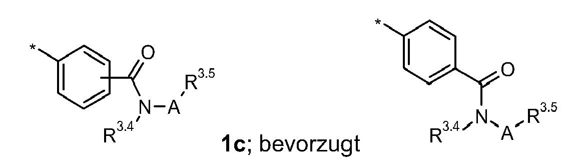 worin
- A: Bindung oder C₁₋₄-Alkyl, gegebenenfalls substituiert mit Oxo oder NMe₂;
- R^{3.4}: H oder Methyl;
- R^{3.5}: ein Rest ausgewählt aus der Gruppe bestehend aus Pyrrolidinyl, Piperidinyl, Piperazinyl, Morpholinyl, Cyclohexyl, Imidazolyl, Pyrazolyl, Phenyl, Pyridinyl, Benzimidazolyl, Imidazolidin-2-on, Pyrrolidin-2-on, Pyrrolidin-3-on, 1-Aza-bicyclo[2.2.2]octan, gegebenenfalls substituiert mit einem oder mehreren Resten unabhängig voneinander ausgewählt aus der Gruppe bestehend aus Methyl, Ethyl, OH, Phenyl, Pyridinyl, Pyrazolyl, Pyrrolidinyl, (CH₂)ₒ-R^{3.5.1}, O-(CH₂)ₒ-R^{3.5.1}, NH-(CH₂)ₒ-R^{3.5.1};
○ 0, 1 oder 2
R^{3.5.1} ein Rest ausgewählt aus der Gruppe bestehend aus Phenyl, Pyrrolidinyl, Piperidinyl und Imidazolidin-2-on, gegebenenfalls substituiert mit Methyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin **R**³ eine Gruppe der Formel **1d;** 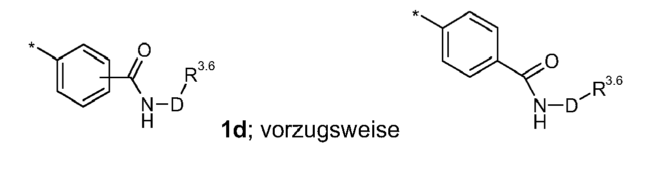 worin
- D: C₂₋₄-Alkinyl;
- R^{3.6}: Pyridinyl;
bedeutet, sowie pharmakologisch verträgliche Salze, Diastereomere, Enantiomere, Racemate, Hydrate oder Solvate davon.

Besonders bevorzugt sind die obigen Verbindungen der Formel **1,** worin X, R¹, R², R⁴, R⁵, R⁶, R⁷ und R⁸ die oben genannte Bedeutung haben und worin **R**³ eine Gruppe der Formel **1e;** 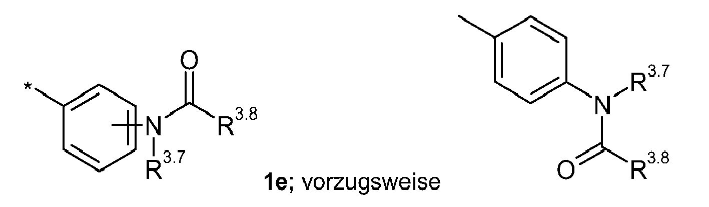 worin
- R^{3.7}: H, , C₁₋₆-Alkyl, C₂₋₆-Alkenyl;C₂₋₆-Alkinyl oder C₁₋₆-Haloalkyl;
- R^{3.8}: H, oder ein Rest ausgewählt aus der Gruppe bestehend aus C₁₋₆-Alkyl, C₂₋₆-Alkenyl;C₂₋₆-Alkinyl, C₁₋₆-Haloalkyl, O-C₁₋₆-Alkyl, C₆₋₁₀-Aryl, ein heterocyclischer C₃₋₁₀-Ring und ein bicyclischer Ring, der gegebenenfalls mit einem oder mehreren Resten ausgewählt aus der Gruppe aus Halogen, C₁₋₆-Alkyl, OH, C₁₋₆-Haloalkyl und O-C₁₋₆-Alkyl substituiert sein kann,
bedeutet, sowie pharmakologisch verträgliche Salze, Diastereomere, Enantiomere, Racemate, Hydrate oder Solvate davon.

Gegenstände der vorliegenden Erfindung sind ebenfalls die folgenden Synthese-Zwischenprodukte für die erfindungsgemäßen Verbindungen:
- Verbindungen nach Formel VIII nach Herstellungsweg B nach Schema 1
- Verbindungen nach Formel IV und V nach Herstellungsweg A nach Schema 1
- Verbindungen nach Formel I nach Schema 1
- Verbindungen nach Formel IV, VI, VII und VIII nach Schema 2
- Verbindungen nach Formel V, VII und VIII nach Schema 3
- Verbindungen nach Formel V, VII und IX nach Schema 4
- Verbindungen nach Formel V, VI, VII, VIII und IX nach Schema 5
- Verbindungen nach Formel V, VII, VIII und IX nach Schema 6
- Verbindungen nach Formel V, VII, VIII, XI und XII nach Schema 7
- Verbindungen nach Formel I und II nach Schema 8
- Verbindungen nach Formel II und III nach Schema 9
- Verbindungen nach Formel V, VI, VIII und X nach Schema 10
- Verbindungen nach Formel III und V nach Schema 11
- Verbindungen nach Formel III und V nach Schema 12
- Verbindungen nach Formel II, III, IV und V nach Schema 13
- Verbindungen nach Formel IV, V, VII, VIII, IX und X nach Schema 14,
worin R¹, R², R³, R⁴, R⁵, R⁶, R⁷, R⁸ und X die vorstehend definierten Bedeutungen haben und wobei R⁹, R¹⁰, R¹¹, R¹² jeweils unabhängig voneinander einen Rest ausgewählt aus der Gruppe bestehend aus H, Halogen, C₁₋₆-Alkyl, C₁₋₆-Haloalkyl, C₆₋₁₀-Aryl, C₃₋₇-Cycloalkyl, aromatischer oder nicht aromatischer C₃₋₁₀-Heterocyclus, C₆₋₁₀-Aryl-C₁₋₆-Alkylen, C₃₋₇-Cycloalkyl- C₁₋₆-Alkylen und C₃₋₁₀-Heterocyclus-C₁₋₆-Alkylen bedeuten, der gegebenenfalls mit einem oder mehreren Resten ausgewählt aus der Gruppe bestehend aus OH, Oxo, C₁₋₆-Alkyl, Phenyl C₃₋₇-Cyclloalkyl, C₃₋₇-Heterocydus und Halogen substituiert sein kann,
und worin R¹³ ausgewählt ist aus der Gruppe bestehend aus OH, Halogen, O-C₁₋₆-Alkyl, C₁₋₆-Alkyl, Phenyl und C₃₋₇-Cycloalkyl und C₃₋₇-Heterocyclus.

Bevorzugte Gegenstände der vorliegenden Erfindung sind die folgenden Synthese-Zwischenprodukte für die erfindungsgemäßen Verbindungen:
- Verbindungen nach Formel I nach Schema 1
- Verbindungen nach Formel VI, VII und VIII nach Schema 2
- Verbindungen nach Formel VII und VIII nach Schema 3
- Verbindungen nach Formel VII und IX nach Schema 4
- Verbindungen nach Formel VI, VII, VIII und IX nach Schema 5
- Verbindungen nach Formel VII, VIII und IX nach Schema 6
- Verbindungen nach Formel VII, VIII, XI und XII nach Schema 7
- Verbindungen nach Formel I und II nach Schema 8
- Verbindungen nach Formel II und III nach Schema 9
- Verbindungen nach Formel X nach Schema 10
- Verbindungen nach Formel III und V nach Schema 11
- Verbindungen nach Formel III und V nach Schema 12
- Verbindungen nach Formel II, III, IV und V nach Schema 13
- Verbindungen nach Formel VII, VIII, IX und X nach Schema 14

### VERWENDETE BEGRIFFE UND DEFINITIONEN

Soweit nicht anders angegeben, sind alle Substituenten voneinander unabhängig. Sollten an einer Gruppe z. B. mehrere C₁₋₆-Alkylgruppen als Substituenten sein, so könnte, im Fall von drei Substituenten C₁₋₆-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl **(I),** 4-Piperidinyl **(II),** 2-Tolyl **(III),** 3-Tolyl **(IV)** und 4-Tolyl **(V)** wie folgt dargestellt:

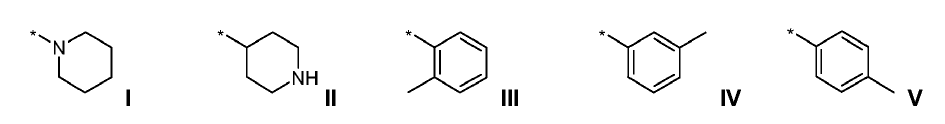

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen. So kann zum Beispiel **VI** die Bedeutung von 2-Tolyl, 3-Tolyl, 4-Tolyl und Benzyl haben.

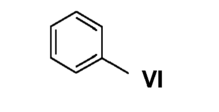

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n*-Propyl, *iso*-Propyl, *n-*Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatome. Beispielsweise werden hierfür genannt: Methylen, Ethylen, Propylen, 1-Methylethylen, Butylen, 1-Methylpropylen, 1, 1-Dimethylethylen, 1, 2-Dimethylethylen, Pentylen, 1, 1-Dimethylpropylen, 2, 2, -Dimethylpropylen, 1, 2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1, 1-Dimethylethylen, 1, 2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit folgende Beispiele der Ringe umfasst:

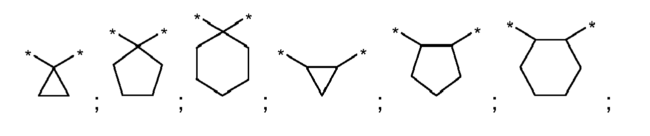

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1, 1-Dimethylethenylen, 1, 2-Dimethylethenylen, Pentenylen, 1, 1-Dimethylpropenylen, 2, 2, - Dimethylpropenylen, 1, 2-Dimethylpropenylen, 1, 3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1, 1-Dimethylethenylen, 1, 2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, - Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Alkylengruppen mit 1 bis 6 Kohlenstoffatomen verstanden, die mit einem aromatischen Ringsystem mit 6 oder 10 Kohlenstoffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf- oder sechsgliedrige heterocyclische Aromaten oder 5-10 gliedrige, bicyclische Heteroarylringe die ein, zwei oder drei Heteroatome, ausgewählt aus der Gruppe Sauerstoff, Schwefel und Stickstoff, enthalten können und so viele konjugierte Doppelbindungen enthalten, dass ein aromatisches Systeme gebildet wird. Als Beispiele für fünf- oder sechsgliedrige heterocyclische Aromaten, werden genannt:

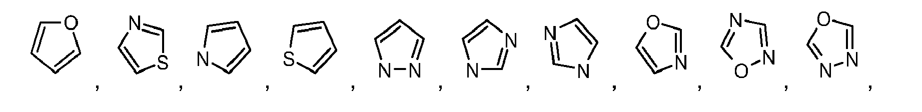

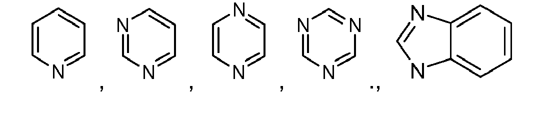

Soweit nicht anders beschreiben, können diese Heteroaryle substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Für die Heteroaryl-C₁₋₆-alkylene werden die folgenden Beispiele genannt:

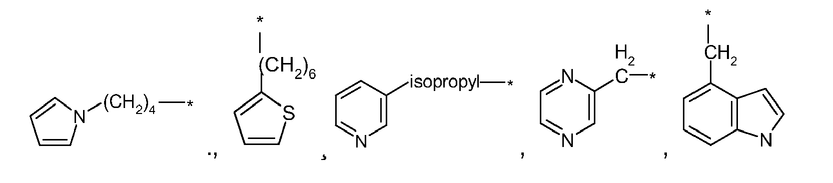

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "heterocyclische Ringe" oder auch "Heterocyclus" werden fünf-, sechs- oder siebengliedrige, gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "heterocyclische, nichtaromatische Ringe" fünf-, sechs- oder siebengliedrige ungesättigte Ringe. Als Beispiele werden genannt:

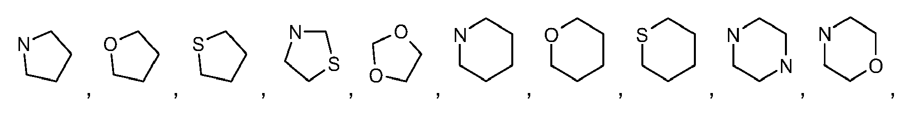

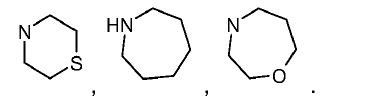

Obwohl unter dem Begriff "heterocyclische Ringe" oder "Heterocyclus" umfasst, definiert der Begriff "heterocyclische, aromatische Ringe" oder "Heteroaryl" fünf- oder sechsgliedrige heterocyclische Aromaten oder 5-10 gliedrige, bicyclische Heteroarylringe die ein, zwei oder drei Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Als Beispiele für fünf- oder sechsgliedrige heterocyclische Aromaten, werden genannt:

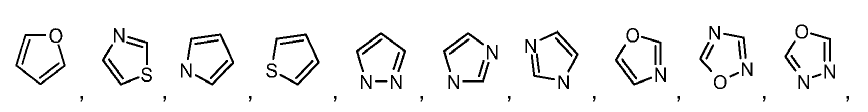

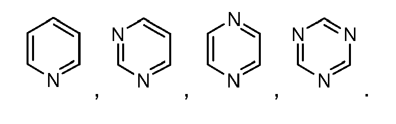

Soweit nicht anders erwähnt, kann ein heterocyclischer Ring (oder "Heterocyclus) mit einer Ketogruppe versehen sein. Als Beispiel hierfür werden genannt.

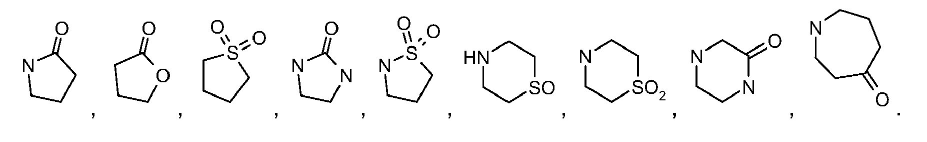

Unter dem Begriff "bicyclische Ringe" werden acht-, neun- oder zehngliedrige, bicyclische Ringe verstanden, die gegebenenfalls ein oder mehrere Heteroatome, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

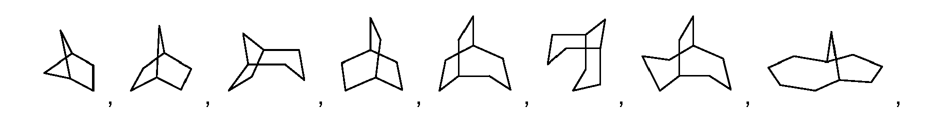

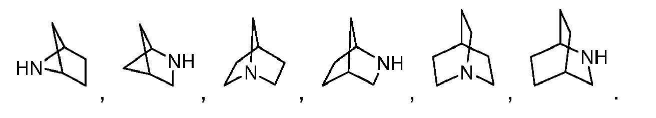

Obwohl unter dem Begriff "bicyclische Ringe" umfasst, definiert der Begriff "kondensierte bicyclische Ringe", bicyclische Ringe, in denen die die Ringe trennende Brücke eine direkte Einfachbindung bedeutet. Als Beispiel für einen kondensierten, bicyclischen Ring werden genannt:

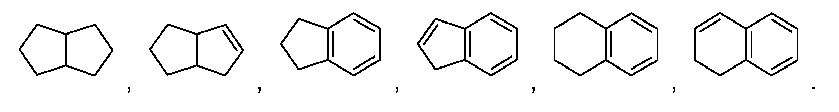

Obwohl unter dem Begriff "bicyclische Ringe" umfasst, definiert der Begriff "kondensierte, bicyclische Heteroringe", bicyclische 5-10 gliedrige Heteroringe die ein, zwei oder drei Heteroatome, ausgewählt aus der Gruppe Sauerstoff, Schwefel und Stickstoff enthalten und in denen die die Ringe trennende Brücke eine direkte Einfachbindung bedeutet. Beispielhaft werden genannt Pyrrolizin, Indol, Indolizin, Isoindol, Indazol, Purin, Chinolin, Isochinolin, Benzimidazol, Benzofuran, Benzopyran, Benzothiazol, Benzothiazol, Benzoisothiazol, Pyridopyrimidin, Pteridin, Pyrimidopyrimidin,

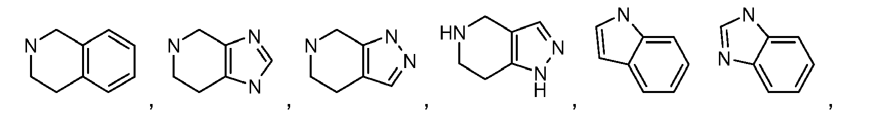

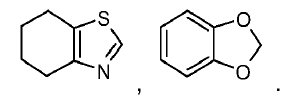

Unter dem Begriff "heterocyclische Spiroringe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

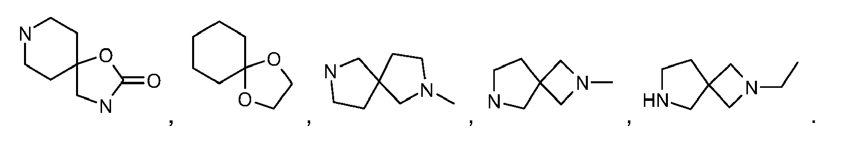

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Andererseits kann die Verbindung der Formel **1** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel (1) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure

Die erfindungsgemäßen Verbindungen können gegebenenfalls als Racemate vorliegen, sie können aber auch als reine Enantiomere, d.h. in (R)- oder (S)-Form gewonnen werden. Bevorzugt sind Verbindungen die als Racemate bzw. als (S)-Form vorliegen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### Synthesevorschriften:

### BEISPIELE

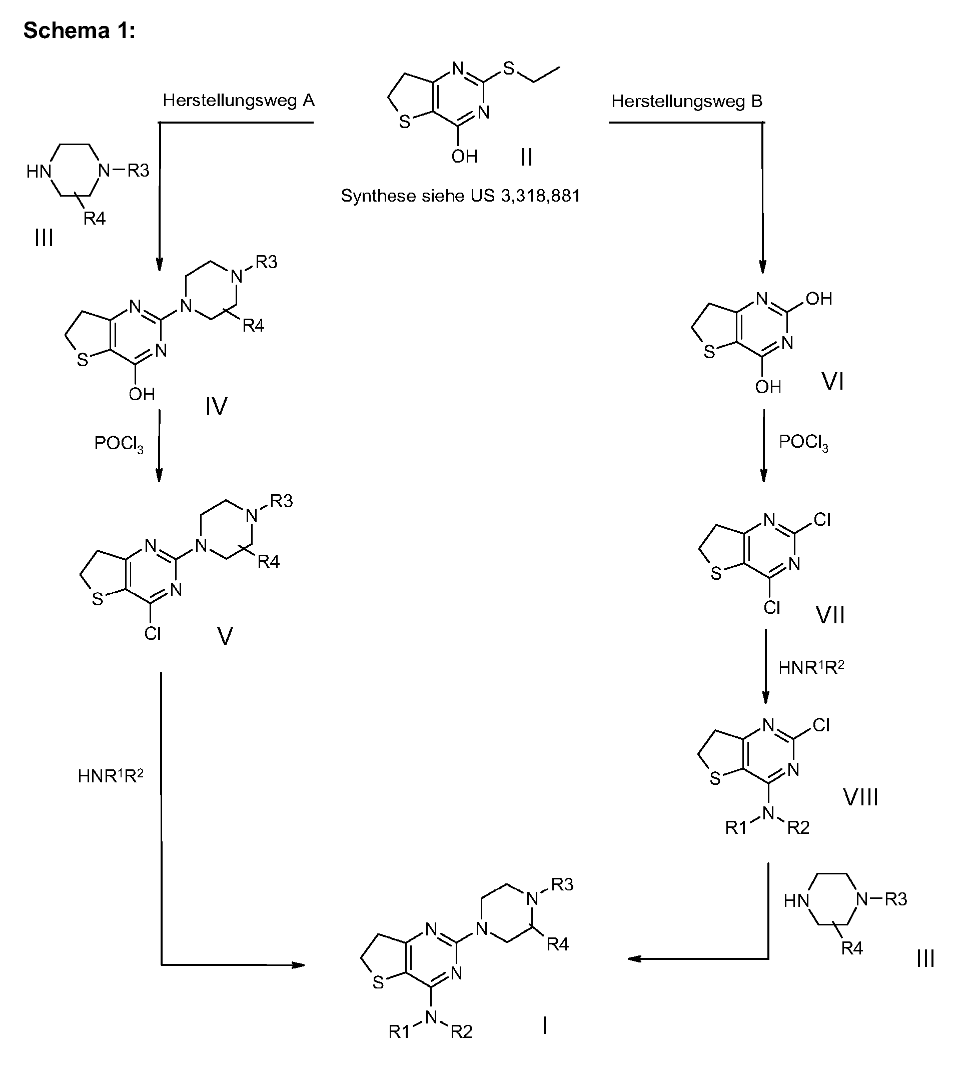

### SYNTHESE VON [2-(4-PHENYL-PIPERAZIN-1-YL)-6,7-DIHYDRO-THIENO[3,2-d]PYRIMIDIN-4-YL]-PROPYL-AMIN ÜBER SYNTHESEWEG A (I) (SCHEMA 1)

**2-(4-Phenyl-piperazin-1-yl)-6,7-dihydro-thieno[3,2-*d*]pyrimidin-4-ol (IV)**: 6,4 g (39 mmol) N-Phenylpiperazin (III) werden in 2,2 ml Eisessig vorgelegt, auf 125° C erhitzt., anschließend werden 3,2 g (15 mmol) 2-Ethylsulfanyl-6,7-dihydro-thieno[3,2-*d*] pyrimidin-4-ol (II) zugegeben, und auf 175° C erhitzt. Nach 1,5 Stunden wird der entstandene Feststoff mit Wasser verrührt, abgesaugt, mit Ethanol gewaschen und getrocknet. 4.3 g Produkt (91 %) werden als Pulver erhalten.

**4-Chlor-2-(4-phenyl-piperazin-1-yl)-6,7-dihydro-thieno[3,2-*d*]pyrimidin (V):** 4,3 g (13,7 mmol) 2-(4-Phenyl-piperazin-1-yl)-6,7-dihydro-thieno[3,2-*d*]pyrimidin-4-ol (IV)und 25 ml Phosphoroxychlorid werden vereint und 5 Stunden bei 120° C gerührt. Überschüssiges Phosphoroxychlorid wird eingedampft, der Rückstand mit Eiswasser und Dichlormethan versetzt. Die organische Phase wird abgetrennt und zur Trockene eingedampft. Der Rückstand wird mit Wasser verrieben, abgesaugt und getrocknet. 5,6 g des Produkts (100%) werden als Pulver erhalten.

**[2-(4-Phenyl-piperazin-1-yl)-6,7-dihydro-thieno[3,2-*d*]pyrimidin-4-yl]-propyl-amin (I) (Beispiel 27):** 0,83 g (2,5 mmol) 4-Chlor-2-(4-phenyl-piperazin-1-yl)-6,7-dihydro-thieno[3,2-*d*]pyrimidin (V) werden mit 8,3 ml n-Propylamin versetzt, 0,2 Stunden bei 130° C in der Mikrowelle umgesetzt. Anschließend wird das Reaktionsgemisch auf Wasser gegeben, mit Dichlormethan extrahiert. Die organische Phase wird eingedampft, der Rückstand kristallisiert. Die Wasserphase wird mit Ethylacetat extrahiert, die organische Phase zur Trockene eingedampft. Die Feststoffe werden vereint und mit Methanol ausgerührt. 0,68 g des Produkts (76%) werden als Pulver erhalten.

### SYNTHESE VON [2-(4-PHENYL-PIPERAZIN-1-YL)-6,7-DIHYDRO-THIENO[3,2-d]PYRIMIDIN-4-YL]-PROPYL-AMIN ÜBER SYNTHESEWEG B (SCHEMA 1)

**6,7-Dihydro-thieno[3,2-*d*]pyrimidin-2,4-diol (VI):** 3,5 g (16,33 mmol) 2-Ethylsulfanyl-6,7-dihydro-thieno[3,2-*d*]pyrimidin-4-ol (II) werden in 70 ml 1N Salzsäure vorgelegt, 0,75 Stunden bei 150° C in der Mikrowelle umgesetzt (14 x 0,250 g in jeweils 5 ml Salzsäure). Danach bleibt das Reaktionsgemisch 7 Stunden bei Raumtemperatur stehen. Es wird abgesaugt, mit Wasser gewaschen und getrocknet. 2,6 g des Produkts VI (95%) werden als Pulver erhalten.

**2,4-Dichlor-thieno[3,2-*d*]pyrimidin (VII):** 2,4 g (14,1 mmol) 6,7-Dihydro-thieno[3,2-*d*]pyrimidin-2,4-diol (VI) werden in 14 ml Phosphoroxychlorid suspendiert, mit 4,5 ml (28,2 mmol) Diethylanilin versetzt und 22 Stunden bei 80° C gerührt. Nach Abkühlen auf Raumtemperatur wird das Reaktionsgemisch auf Eiswasser gegeben, der ausgefallene Niederschlag abgesaugt und mit Wasser gewaschen. Der Niederschlag wird in Dichlormethan gelöst, vorhandenes Wasser mittels Phasenseparator abgetrennt. Die organische Phase wird zur Trockene eingedampft. 2,5 g des Produktes VII (85%) werden erhalten.

**(2-Chlor-6,7-dihydro-thieno[3,2-*d*]pyrimidin-4-yl)-propyl-amin (VIII):** 10,4 g (50,2 mmol) 2,4-Dichlor-thieno[3,2-*d*]pyrimidin (VII), 8,2 ml (100 mmol) N-Propylamin und 17,5 ml (100 mmol) N-Ethyldiisopropylamin werden in 100 ml Tetrahydrofuran vorgelegt und 20 Stunden bei Raumtemperatur gerührt. Die Suspension wird filtriert, das Filtrat eingedampft. Der Rückstand wird mit 100 ml Wasser versetzt und im Ultraschallbad behandelt. Festsubstanz wird abgesaugt, getrocknet und mit 50 ml Petrolether ausgerührt. 10,0 g des Produkts VIII (86%) werden als Pulver erhalten.

**[2-(4-Phenyl-piperazin-1-yl)-6,7-dihydro-thieno[3,2-*d*]pyrimidin-4-yl]-propyl-amin (Beispiel 27) (I) :** 9,7 g (42,3 mmol) 2-Chlor-6,7-dihydro-thieno[3,2-*d*]pyrimidin-4-yl)-propyl-amin (VIII) und 25,7 ml (169,2 mmol) 1-Phenylpiperazin (III) werden in 200 ml Dioxan vorgelegt (Durchführung in 10 Portionen), dann 1 Stunde bei 160° C in der Mikrowelle umgesetzt. Das Reaktionsgemisch wird eingedampft, der Rückstand mit 250 ml Wasser versetzt. Anschließend wird abgesaugt, mit Wasser gewaschen und getrocknet. Der Rückstand wird mit Acetonitril ausgerührt, dann aus Isopropanol umkristallisiert. 8,3 g des Produkts (I) (55%) werden als Pulver (Smp 121 °C) erhalten. ¹H NMR (400 MHz, DMSO): 7,22 (2H, t); 6,97 (2H, d); 6,79 (1 H, t); 6,43 (2H, t); 3,81-3,69 (4H, m); 3,37-3,19 (4H, m); 3,19-3,09 (4H, m); 2,99 (2H, t); 1,62-1,47 (2H, m); 0,87 (3H, t).

Folgende Beispiele werden über Syntheseweg A (Schema 1) analog oben beschrieben hergestellt (es werden jeweils nur der letzte Syntheseschritt, nämlich die Umsetzung von V mit einem Amin zum Produkt I beschrieben):

**Cyclohexyl-[2-(4-phenyl-piperazin-1-yl)-6,7-dihydro-thieno[3,2-*d*]pyrimidin-4-yl]-amin (Beispiel 16) (I) :** 0,300 g (0,90 mmol) 4-Chlor-2-(4-phenyl-piperazin-1-yl)-6,7-dihydro-thieno[3,2-*d*]pyrimidin (V) werden in 4 ml Cyclohexylamin vorgelegt, dann 0,1 Stunden bei 130° C in der Mikrowelle umgesetzt. Anschließend wird das Reaktionsgemisch eingedampft, der Rückstand mit Wasser verrührt und abgesaugt. 0,120 g des Produkts I (34%) werden als Pulver erhalten.
¹H NMR (400 MHz, DMSO): 7,22 (2H, t); 6,97 (2H, d); 6,79 (1 H, t); 6,08 (1 H, d); 3,93-3,81 (1 H, m); 3,80-3,69 (4H, m); 3,22 (2H, t); 3,18-3,11 (4H, m); 2,98 (2H, t); 1,92-1,79 (2H, m); 1,79-1,66 (2H, m); 1,66-1,55 (1 H, m); 1,37-1,21 (4H, m); 1,18-1,04 (1 H, m).

**(3-Chlor-phenyl)-[2-(4-phenyl-piperazin-1-yl)-6,7-dihydro-thieno[3,2-*d*]pyrimidin-4-yl]-amin (Beispiel 10) (I)** : 0,320 g (0,96 mmol) 4-Chlor-2-(4-phenyl-piperazin-1-yl)-6,7-dihydro-thieno[3,2-*d*]pyrimidin (V) 1,0 ml (9,5 mmol) 3-Chlor-phenylamin und 0,33 ml (1,92 mmol) Diisopropylethylamin werden zusammengegeben, 0,5 Stunden bei 130 °C in der Mikrowelle umgesetzt. Anschließend wird das Reaktionsgemisch mit Ethylacetat versetzt und sauer extrahiert. Die organische Phase wird getrocknet und zur Trockene eingedampft. Der Rückstand wird mit Acetonitril, Wasser und Trifluoressigsäure versetzt. Die das Produkt I enthaltende Phase, wird chromatographisch über RP-Säule mit einer HPLC gereinigt (Säule: Microsorb, RP-C18, 300Å, 10µm, 21,4*250 mm, Fließmittel: Acetonitril + 0,1 % Ameisensäure (A), Wasser + 0,13% Ameisensäure (B))

| Gradient: Minuten | % Fließmittel A | % Fließmittel B |
|---|---|---|
| 0 | 10 | 90 |
| 4,9 | 10 | 90 |
| 27 | 100 | 0 |
| 32 | 100 | 0 |
| 32,5 | 10 | 90 |
| 37,5 | 10 | 90 |

Entsprechende Fraktionen werden vereinigt und gefriergetrocknet. 0,101 g des Produkts werden als Pulver erhalten.
¹H NMR (400 MHz, DMSO): 8,63 (1H, s); 7,87 (1H, t); 7,60 (1H, dd); 7,31 (1H, t); 7,26-7,20 (2H, m); 7,05-7,01 (1H, m); 6,99 (2H, d); 7,80 (1H; t); 3,81-3,76 (4H, m); 3,22-3,15 (4H, m); 3,10 (2H, t).

**(3-Methoxy-phenyl)-[2-(4-phenyl-piperazin-1-yl)-6,7-dihydro-thieno[3,2-*d*]pyrimidin-4-yl]-amin (Beispiel 11) (I) :** 0,320 g (0,96 mmol) 4-Chlor-2-(4-phenyl-piperazin-1-yl)-6,7-dihydro-thieno[3,2-*d*]pyrimidin (V), 1,5 ml (13,1 mmol) 3-Methoxy-phenylamin und 0,33 ml (1,92 mmol) Diisopropylethylamin werden zusammengegeben, 0,5 Stunden bei 130 °C in der Mikrowelle umgesetzt. Anschließend wird das Reaktionsgemisch chromatographisch über RP-Säule mit einer HPLC gereinigt (Säule: Microsorb, RP-C18, 300Å, 10µm, 21,4*250 mm, Fließmittel: Acetonitril + 0,1 % Ameisensäure (A), Wasser + 0,13% Ameisensäure (B))

| Gradient: Minuten | % Fließmittel A | % Fließmittel B |
|---|---|---|
| 0 | 10 | 90 |
| 4,9 | 10 | 90 |
| 27 | 100 | 0 |
| 32 | 100 | 0 |
| 32,5 | 10 | 90 |
| 37,5 | 10 | 90 |

0,095 g des Produkts (23%) werden als Pulver (Smp 133-135°C) erhalten. ¹H NMR (400 MHz, DMSO): 8,37 (1H, s); 7,36 (1H, t); 7,26-7,14 (4H, m); 6,98 (2H, d); 6,80 (1H, t); 6,57 (1H, dd); 3,82-3,76 (4H, m); 3,75 (3H, s); 3,32-3,24 (2H, m); 3,21-3,15 (4H, m); 3,09 (2H, t).

**Phenyl-[2-(4-phenyl-piperazin-1-yl)-6,7-dihydro-thieno[3,2-*d*]pyrimidin-4-yl]-amin (Beispiel 18) (I) :** 0,300 g (0,90 mmol) 4-Chlor-2-(4-phenyl-piperazin-1-yl)-6,7-dihydro-thieno[3,2-*d*]pyrimidin (V) und 3 ml Phenylamin werden vereint, dann 0,3 Stunden bei 130° C in der Mikrowelle umgesetzt. Anschließend wird das Reaktionsgemisch mit Wasser versetzt und mit Ethylacetat extrahiert. Die organische Phase wird getrocknet und zur Trockene eingedampft. Der Rückstand wird mit Diethylether verrieben und abgesaugt. 0,18 g des Produkts I (51%) werden erhalten (Smp 174-175°C).
¹H-NMR (400 MHz, DMSO): 8,59 (1 H, s); 7,63 (2H, d); 7,31 (2H, t); 7,22 (2H, t); 7,03 (1 H, t); 6,99 (2H, d); 6,80 (1H, t); 3,83-3,73 (4H, m); 3,31 (2H, t); 3,23 (4H, m); 3,12 (2H, t).

Folgende Beispiele werden über Syntheseweg B (Schema 1) analog oben beschrieben hergestellt (es werden jeweils nur der letzte Syntheseschritt (Umsetzung von VIII mit III zum Produkt I beschrieben)::

**{2-[4-(4-Chlor-phenyl)-piperazin-1-yl]-6,7-dihydro-thieno[3,2-*d*]pyrimidin-4-yl}-propyl-amin (I) (Beispiel 37):** 0,250 g (1,1 mmol) 2-Chlor-6,7-dihydro-thieno[3,2-*d*]pyrimidin-4-yl)-propylamin (VIII), 0,700 g (3,6 mmol) 1-(4-Chlorphenyl)-piperazin (III) und 0,400 ml (2,3 mmol) Diisopropylethylamin werden in 4 ml Dioxan vorgelegt, dann 1,5 Stunden bei 160° C in der Mikrowelle umgesetzt. Das Reaktionsgemisch wird unter Eiskühlung mit Wasser versetzt, im Ultraschallbad behandelt. Der ausgefallene Niederschlag wird abgesaugt, mit Wasser und Petrolether gewaschen und getrocknet. 0,300 g des Produkts I (72%) werden als Pulver erhalten. ¹H NMR (400 MHz, DMSO): 7,24 (2H, d); 6,98 (2H, d); 6,42 (1 H, t); 3,79-3,70 (4H, m); 3,23 (2H, t); 3,19-3,11 (4H, m); 2,98 (2H, t); 1,61-1,47 (2H, m); 0,87 (4, 3H).

**{2-[4-(4-Hydroxy-phenyl)-piperazin-1-yl]-6,7-dihydro-thieno[3,2-*d*]pyrimidin-4-yl}-propyl-amin (I) (Beispiel 39) :** 0,400 g (1,7 mmol) 2-Chlor-6,7-dihydro-thieno[3,2-*d*]pyrimidin-4-yl)-propylamin (VIII), 1,55 g (8,7 mmol) 1-(4-Hydroxyphenyl)-piperazin (III) und 0,610 ml (3,5 mmol) Diisopropylethylamin werden in 4 ml Dimethylformamid vorgelegt, 2 Stunden bei 160° C in der Mikrowelle umgesetzt. Anschließend wird das Reaktionsgemisch mit Wasser versetzt und mit Dichlormethan extrahiert. Die organische Phase wird getrocknet und zur Trockene eingedampft. Der Rückstand wird chromatographisch über eine 10 g Kieselgelkartusche mit Petrolether/Essigester 1:1 gereinigt. 0,370 g des Produkts I (57%) werden als Pulver erhalten. ¹H NMR (400 MHz, DMSO): 8,80 (1H, s); 6,82 (2H, d); 6,66 (2H, d); 6,39 (1H, t); 3,76-3,69 (4H, m); 3,23 (2H, t); 3,02-2,92 (6H, m); 1,60-1,48 (2H, m); 0,86 (3H, t).

**((*1R*,*2R*)-2-Benzyloxy-cyclopentyl)-[2-(4-phenyl-piperazin-1-yl)-6,7-dihydro-thieno[3,2-*d*]pyrimidin-4-yl]-amin (chiral) (Beispiel 112) (I) :** 0,450 g (1,2 mmol) ((*1R*,*2R*)-2-Benzyloxy-cyclopentyl)-(2-chlor-6,7-dihydro-thieno[3,2-*d*]pyrimidin-4-yl)-amin (chiral) (VIII) und 0,760 ml (5,0 mmol) 1-Phenyl-piperazin (III) werden in 5 ml Dioxan vorgelegt, dann 1 Stunde bei 160° C in der Mikrowelle umgesetzt. Das Reaktionsgemisch wird eingedampft, der Rückstand mit Wasser versetzt und mit Dichlormethan extrahiert. Die organische Phase wird mittels Phasenseparator abgetrennt und zur Trockene eingedampft. Das Rohprodukt I wird chromatographisch über eine 100g Kieselgelkartusche mit Lösungsmittelgemisch Petrolether/Essigester 8/2 gereinigt. 0,540 g des Produkts (89%) werden erhalten (Smp 100-105°C).
¹H NMR (400 MHz, DMSO): 7,36-7,18 (7H, m); 6,91 (2H, d); 6,79 (1 H, t); 6,39 (1 H, d); 4,59-4,40 (3H, m); 3,95-3,89 (1 H, m); 3,79-3,69 (4H, m); 3,23 (2H, t); 3,11-3,03 (4H, m); 2,99 (2H, t); 2,11-2,00 (1 H, m); 1,93-1,79 (1 H, m); 1,77-1,50 (4H, m).

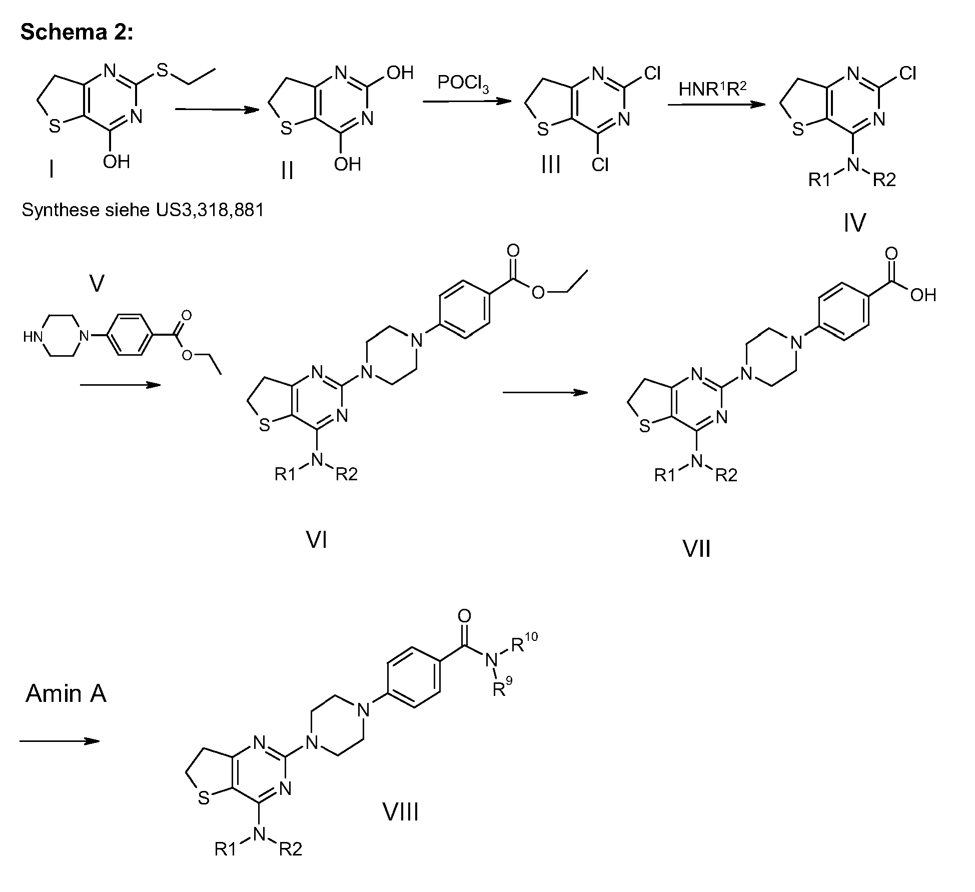

### SYNTHESE VON 4-[4-(4-CYCLOHEXYLAMINO-6,7-DIHYDRO-THIENO[3,2-d]PYRIMIDIN-2-YL)-PIPERAZIN-1-YL]-BENZOESÄURE (NACH SCHEMA 2)

**4-[4-(4-Cyclohexylamino-6,7-dihydro-thieno[3,2-*d*]pyrimidin-2-yl)-piperazin-1-yl]-benzoesäure-ethylester (VI):** 2,1 g (7,8 mmol) (2-Chlor-6,7-dihydro-thieno[3,2-*d*]pyrimidin-4-yl)-cyclohexyl-amin (IV), 3,1 g (13,2 mmol) 4-Piperazin-1-yl-benzoesäureethylester (V) und 2,9 ml (16,5 mmol) Diisopropylethylamin werden in 20 ml Dioxan vorgelegt, dann 4 Stunden bei 160° C in der Mikrowelle umgesetzt. Ausgefallene Substanz wird abgesaugt, die Mutterlauge eingedampft. Der Rückstand wird mit Wasser und Ethylacetat extrahiert, die vereinten organischen Phasen getrocknet und zur Trockene eingedampft. Das Rohprodukt VI wird mit Petrolether/Ethylacetat 9:1 kristallisiert. 2.3 g des Produkts (62%) werden erhalten.

**4-[4-(4-Cyclohexylamino-6,7-dihydro-thieno[3,2-*d*]pyrimidin-2-yl)-piperazin-1-yl]-benzoesäure (VII) (Beispiel 106):** 2,3 g (4,8 mmol) 4-[4-(4-Cyclohexylamino-6,7-dihydro-thieno[3,2-*d*]pyrimidin-2-yl)-piperazin-1-yl]-benzoesäure-ethylester (VI) und 26 ml (26 mmol) Natriumhydroxid werden in 15 ml Tetrahydrofuran und 15 ml Methanol vorgelegt, 2 Stunden unter Rückfluss gerührt. Anschließend wird das Reaktionsgemisch eingedampft, der Rückstand mit 2N Salzsäure angesäuert. Der ausfallende Niederschlag wird abgesaugt, mit Wasser gewaschen und getrocknet. 2.1 g des Produkts VII (100%) werden als Pulver erhalten.

**Standardvorschrift für die Synthese von Amiden:** Eine Lösung von 0,01 mmol der Säure (VII) in 500 µl Dimethylformamid wird mit 1,388 µl (0,01 mmol) Triethylamin und 3,21 mg (0,01 mmol) O-(1 H-Benzotriazol-1-yl)-N,N,N',N'-tetramethyluronium tetrafluoroborat [TBTU] versetzt und 15 min. bei Raumtemperatur geschüttelt. Zu diesem Reaktionsgemisch wird eine Lösung von 0,01 mmol des Amins (Amin A) in 500 µl Dimethylformamid gegeben und 12 h bei Raumtemperatur geschüttelt. Anschließend wird das Reaktionsgemisch zur Trockene eingeengt und durch präparative HPLC gereinigt (hierbei entsteht Produkt VIII).

Folgende Beispiele können analog dem oben gezeigten Syntheseweg hergestellt werden (nach Schema 2) (es werden jeweils nur die Umsetzungen der Säuren VII mit dem Amin A zu Produkt VIII beschrieben):

**4-[4-(4-Propylamino-6,7-dihydro-thieno[3,2-*d*]pyrimidin-2-yl)-piperazin-1-yl]-N-(4-pyrrolidin-1-ylmethyl-benzyl)-benzamid (VIII) (Beispiel 90):** 0,350 g (0,9 mmol) 4-[4-(4-Propylamino-6,7-dihydro-thieno[3,2-*d*]pyrimidin-2-yl)-piperazin-1-yl]-benzoesäure (VII) werden in 8 ml Dimethylsulfoxid vorgelegt, 0,15 ml (0,9 mmol) Diisopropylethylamin und 0,340 g (0,9 mmol) O-(7-Azabenzotriazol-1-yl-)-N,N,N',N'-tetramethyluronium-hexafluorophosphat (HATU) zugegeben. Es wird 0,1 Stunden bei Raumtemperatur gerührt, dann 0,170 g (0,9 mmol) 4-Pyrrolidin-1-ylmethyl-benzylamin (Amin A) zugegeben und noch 0,9 mmol Diisopropylethylamin zugefügt. Das Reaktionsgemisch wird 3 Stunden bei Raumtemperatur gerührt, dann über Alox abgesaugt und mit Dimethylsulfoxid nach gewaschen. Die Mutterlauge wird eingedampft, der Rückstand im Eisbad abgekühlt und mit Wasser versetzt. Ausgefallener Niederschlag wird abgesaugt und getrocknet. Anschließend wird dieser mit ca. 50 ml Petrolether im Ultraschallbad behandelt, danach wurde der Niederschlag abgesaugt. 0,374 g des Produkts VIII (75%) werden erhalten (Smp 157-158°C).
¹H NMR (400 MHz, DMSO): 8,71 (1H, t); 7,79 (2H, d); 7,23 (4H, s); 6,99 (2H, d); 6,43 (1H, t); 4,43 (2H, d); 3,79-3,71 (4H, m); 3,52 (2H, s); 3,23 (2H, t); 2,99 (2H, t); 2,44-2,34 (4H, m); 1,72-1,63 (4H, m); 1,61-1,48 (2H, m); 0,87 (3H, t).

**N-(1-Methyl-piperidin-4-yl)-4-[4-(4-propylamino-6,7-dihydro-thieno[3,2-*d*]pyrimidin-2-yl)-piperazin-1-yl]-benzamid (VIII) (Beispiel 87) :** 0,500 g (1,3 mmol) 4-[4-(4-Propylamino-6,7-dihydro-thieno[3,2-*d*]pyrimidin-2-yl)-piperazin-1-yl]-benzoesäure (VII) werden in 12 ml Dimethylsulfoxid vorgelegt, 0,716 g (1,9 mmol) O-(7-Azabenzotriazol-1-yl-)-N,N,N',N'-tetramethyluronium-hexafluoro-phosphat (HATU) in 6 ml Dimethylsulfoxid zugegeben. Es wird 1 Stunde bei Raumtemperatur gerührt. Anschließend werden 0,143 g (1,3 mmol) 1-Methyl-piperidin-4ylamin (Amin A) in 6 ml Dimethylsulfoxid zugegeben, danach noch 2,5 mmol Diisopropylethylamin. Das Reaktionsgemisch wird 16 Stunden bei Raumtemperatur gerührt, dann über Alox abgesaugt. Alox mit Dimethylsulfoxid nachgewaschen. Die Mutterlauge wird zur Trockene eingedampft. Der Rückstand wird mit Wasser ausgerührt, abgesaugt und getrocknet. 0,470 g des Produkts VIII (76%) werden als Pulver (Smp 229-233°C) erhalten.
¹H NMR (400 MHz, DMSO): 7,90 (1H, d); 7,75 (2H, d); 6,97 (2H, d); 6,43 (1H, d); 3,82-3,66 (5H, m); 3,23 (2H, t); 2,99 (2H, t); 2,80-2,70 (2H, m); 2,15 (3H, s); 1,92 (2H, t); 1,77-1,68 (2H, m); 1,64-1,49 (4H, m); 0,87 (3H, t).

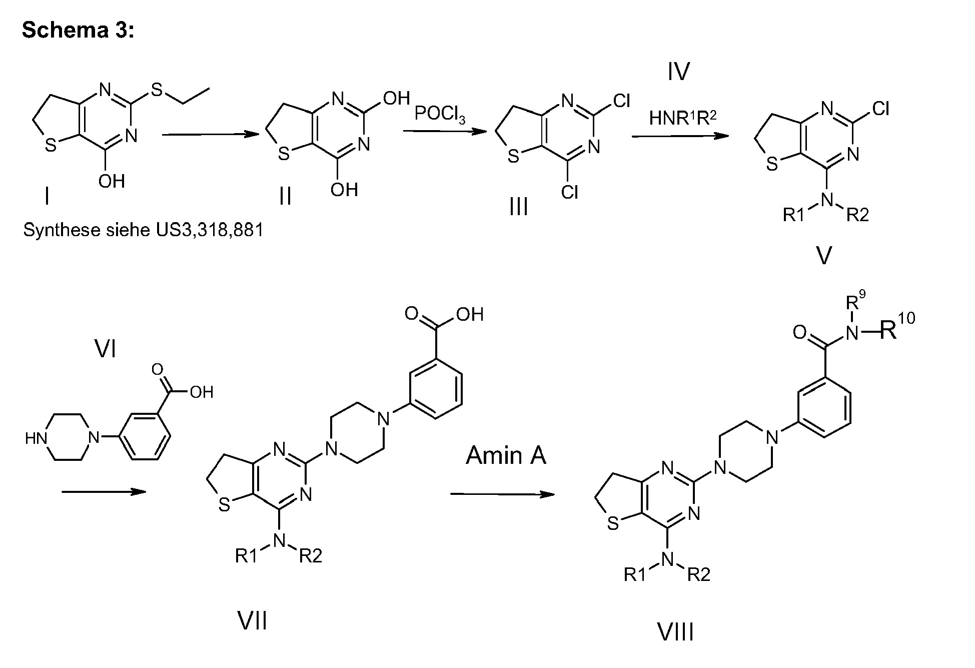

### SYNTHESE VON 3-[4-(4-PROPYLAMINO-6,7-DIHYDRO-THIENO[3,2-d]PYRIMIDIN-2-YL)-PIPERAZIN-1-YL]-BENZOESÄURE (SCHEMA 3) (es werden jeweils nur die Umsetzungen von V mit VI und mit dem Amin A zu Produkt VIII beschrieben):

**3-[4-(4-Propylamino-6,7-dihydro-thieno[3,2-*d*]pyrimidin-2-yl)-piperazin-1-yl]-benzoesäure (VIII) (Beispiel 66)** 0,900 g (3,9 mmol) (2-Chlor-6,7-dihydro-thieno[3,2-*d*]pyrimidin-4-yl)-propyl-amin (V) und 1,5 g (4,7 mmol) 3-Piperazin-1-yl-benzoesäure-triflat (VI) werden in 20 ml Dioxan vorgelegt, 5 ml (36,7 mmol) Diisopropylethylamin (Amin A) zugegeben. Die Suspension wird 24 Stunden bei 180° C in einem Glas-Druckbehälter gerührt, anschließend eingedampft. Der Rückstand wird in Acetonitril und Wasser gelöst, dann über RP-Säule mit einer HPLC gereinigt (Säule: XTerra, MS-C18, 5µm, 19*100 mm, Fließmittel: Wasser+ 0,1 % Trifluoressigsäure (A), Acetonitril + 0,1% Trifluoressigsäure (B))

| Gradient: Minuten | % Fließmittel A | % Fließmittel B |
|---|---|---|
| 0 | 90 | 10 |
| 2 | 90 | 10 |
| 11,5 | 0 | 100 |
| 13 | 0 | 100 |
| 13,5 | 90 | 10 |

Entsprechende Fraktionen werden vereinigt und gefriergetrocknet. 0,43 g des Produkts VIII (27%) werden erhalten.

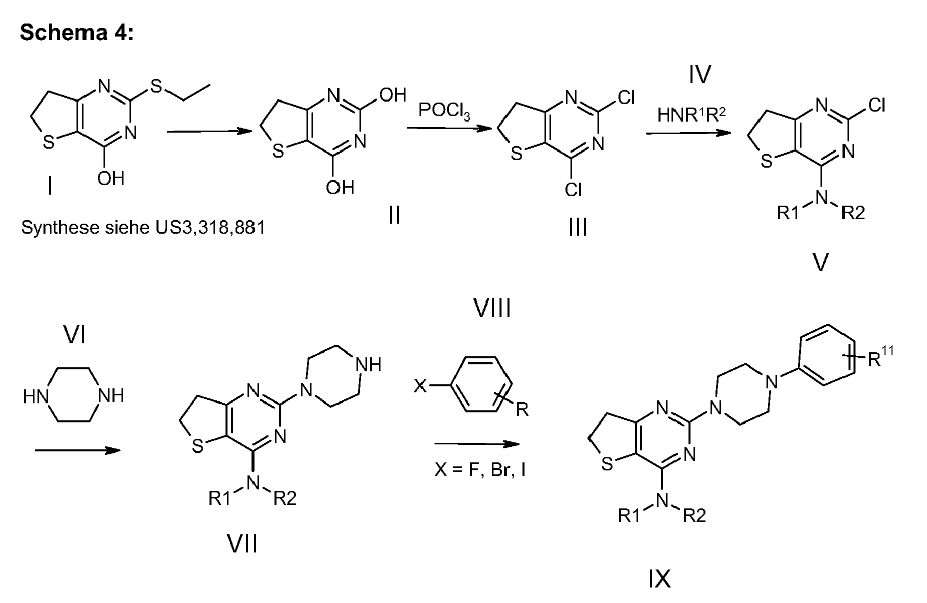

### SYNTHESE VON 4-[4-(4-PROPYLAMINO-6,7-DIHYDRO-THIENO[3,2-d]PYRIMIDIN-2-YL)-PIPERAZIN-1-YL]-BENZONITRIL (IX) (SCHEMA 4) (es werden nur die Umsetzungen von V mit VI zu VII und die von VII und VIII zu IX beschrieben):

**(2-Piperazin-1-yl-6,7-dihydro-thieno[3,2-*d*]pyrimidin-4-yl)-propyl-amin (VII)** : 1,0 g (4,4 mmol) (2-Chlor-6,7-dihydro-thieno[3,2-*d*]pyrimidin-4-yl)-propyl-amin (V) und 1,9 g (21,8 mmol) Piperazin (VI) werden in 10 ml Dioxan vorgelegt, dann 0,7 Stunden bei 150° C in der Mikrowelle umgesetzt. Anschließend wird das Reaktionsgemisch mit Wasser und Ethylacetat extrahiert, die organische Phase getrocknet und zur Trockene eingedampft. Der Rückstand wird mit Petrolether verrieben und abgesaugt. 0,97 g des Produkts VII (80%) werden als Pulver erhalten.

**4-[4-(4-Propylamino-6,7-dihydro-thieno[3,2-*d*]pyrimidin-2-yl)-piperazin-1-yl]-benzonitril (IX) (Beispiel 76) :** 0,150 g (0,5 mmol) (2-Piperazin-1-yl-6,7-dihydro-thieno[3,2-*d*]pyrimidin-4-yl)-propyl-amin (VII), 0,082 g (0,45 mmol) 4-Brom-benzonitril (VIII), 0,011 g (0,05 mmol) Palladiumacetat, 0,041 g (0,07 mmol) Xantphos und 0,204 g (0,6 mmol) Cäsiumcarbonat werden in 1 ml Toluol vorgelegt, dann 24 Stunden bei 80° C gerührt. Anschließend wird das Reaktionsgemisch mit Wasser versetzt und mit Ethylacetat extrahiert. Die organische Phase wird mit Wasser gewaschen, getrocknet und zur Trockene eingedampft. Der Rückstand wird chromatographisch über RP-Säule mit einer HPLC gereinigt (Säule: Microsorb, RP-C18, 300Å, 10µm, 21,4*250 mm, Fließmittel: Acetonitril + 0,1 % Ameisensäure (A), Wasser + 0,13% Ameisensäure (B)

| Gradient: Minuten | % Fließmittel A | % Fließmittel B |
|---|---|---|
| 0 | 10 | 90 |
| 4,9 | 10 | 90 |
| 27 | 100 | 0 |
| 32 | 100 | 0 |
| 32,5 | 10 | 90 |
| 37,5 | 10 | 90 |

entsprechende Fraktionen vereinigt und gefriergetrocknet. 0,08 g des Produkts IX (47%) werden als Pulver erhalten.
¹H NMR (400 MHz, DMSO): 7,59 (2H, d); 7,04 (2H, d); 6,44 (1H, t); 3,78-3,71 (4H, m); 3,43-3,36 (4H, m); 3,23 (2H, t); 2,99 (2H, t); 1,60-1,49 (2H, m); 0,86 (3H, t).

**{2-[4-(4-Nitro-phenyl)-piperazin-1-yl]-6,7-dihydro-thieno[3,2-*d*]pyrimidin-4-yl}-propylamin (IX) (Beispiel 71):** 0,100 g (0,358 mmol) (2-Piperazin-1-yl-6,7-dihydro-thieno[3,2-*d*]pyrimidin-4-yl)-propyl-amin (VII) (cf. vorherige Vorschrift), 0,038 ml (0,358 mmol) 1-Fluoro-4-nitro-benzol (VIII) und 0,148 g (1,07 mmol) Kaliumcarbonat werden in 4 ml Tetrahydrofuran vorgelegt, dann 16 Stunden bei Raumtemperatur und 8 Stunden bei 65° C gerührt und weitere 16 Stunden bei Raumtemperatur stehen gelassen. Anschließend wird die Lösung mit Wasser versetzt und mit Ethylacetat extrahiert. Die organische Phase wird getrocknet und zur Trockene eingedampft. Der Rückstand wird mit Wasser und Acetonitril versetzt, wobei ein Niederschlag ausfällt. Dieser wird abgesaugt, gewaschen und getrocknet. 0,023 g des Produkts IX (16%) werden als Pulver erhalten. ¹H NMR (400 MHz, DMSO): 8,07 (2H, d); 7,04 (2H, d); 6,49-6,43 (1H, m); 3,81-3,73 (4H, m); 3,58-3,50 (4H, m); 3,24 (2H, t); 2,99 (2H, t); 1,61-1,49 (2H, m); 0,87 (3H, t).

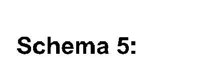

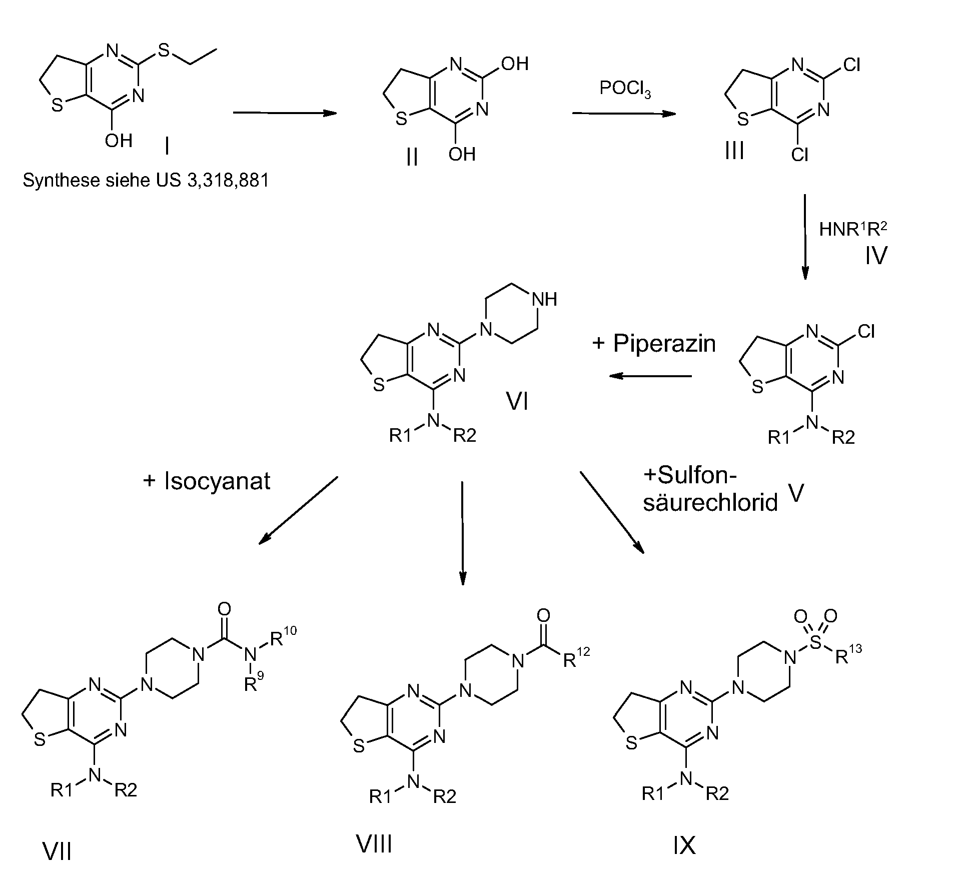

### STANDARDVORSCHRIFTE FÜR DIE SYNTHESE VON AMIDEN, HARNSTOFFE UND SULFONAMIDEN AUS (2-PIPERAZIN-1-YL-6,7-DIHYDRO-THIENO[3,2-d]PYRIMIDIN-4-YL)-PROPYLAMIN (SCHEMA 5)

**Standardvorschrift für die Synthese von Amiden (VIII):** siehe oben

**Standardvorschrift für die Synthese von Sulfonamiden (IX):** Eine Lösung von 0,063 mmol des Amins (VI) in 1 ml Dichlormethan wird mit 30 µl Diisopropylamin und 0,065 mmol Sulfonsäurechlorid, gelöst in 1 ml Dichlormethan, versetzt und 1 h bei Raumtemperatur gerührt. Anschließend wird das Reaktionsgemisch zur Trockene eingeengt und durch präparative HPLC gereinigt.

**Standardvorschrift für die Synthese von Harnstoffen (VII):** Eine Lösung von 0,063 mmol des Amins (VI) in 1 ml Tetrahydrofuran wird mit 30 µl Diisopropylamin und 0,065 mmol Isocyanat, gelöst in 1 ml Tetrahydrofuran, versetzt und 1 h bei Raumtemperatur gerührt. Anschließend wird das Reaktionsgemisch zur Trockene eingeengt und durch präparative HPLC gereinigt.

Standardbedingungen für präparative HPLC Reinigung: Säule: XTerra, MS-C18, 5µm, 19*100 mm, Fließmittel: Wasser + 0,1 % Trifluoressigsäure (A), Acetonitril + 0,1% Trifluoressigsäure (B)

| Gradient: Minuten | % Fließmittel A | % Fließmittel B |
|---|---|---|
| 0 | 90 | 10 |
| 2 | 90 | 10 |
| 11,5 | 0 | 100 |
| 13 | 0 | 100 |
| 13,5 | 90 | 10 |

Entsprechende Fraktionen werden vereinigt und gefriergetrocknet. Folgende Beispiele werden über den gleichen Syntheseweg nach Schema 5 hergestellt:

**4-(4-Cyclohexylamino-6,7-dihydro-thieno[3,2-*d*]pyrimidin-2-yl)-piperazin-1-carbonsäure-phenylamid (VII) (Beispiel 102)** : 0,330 g (1,0 mmol) Cyclohexyl-(2-piperazin-1-yl-6,7-dihydro-thieno[3,2-*d*]pyrimidin-4-yl)-amin (VI) und 0,115 ml (1,0 mmol) Isocyansaürephenylester werden in 12,5 ml Tetrahydrofuran vorgelegt, dann 1 Stunde bei Raumtemperatur gerührt. Anschließend wird das Reaktionsgemisch eingedampft. Der Rückstand wird mit Wasser verrührt, abgesaugt, mit Wasser und Petrolether gewaschen, dann getrocknet. Das Rohprodukt wird chromatographisch über eine 25g Kieselgelkartusche mit Lösungsmittelgemisch Petrolether/ Essigester 1/1 gereinigt. 0,240 g des Produkts VII (53%) werden als Pulver (Smp 207-210°C) erhalten.
¹H NMR (400 MHz, DMSO): 8,51 (1H, s); 7,47 (2H, d); 7,23 (2H, t); 6,92 (1H, t); 6,11 (1H, d); 3,91-3,78 (1H, m); 3,69-3,59 (4H, m); 3,53-3,44 (4H, m); 3,22 (2H, t); 2,98 (2H, t); 1,92-1,78 (2H, m); 1,79-1,67 (2H, m); 1,65-1,56 (1H, m); 1,37-1,21 (4H, m); 1,18-1,04 (1H, m).

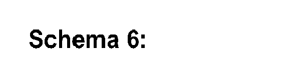

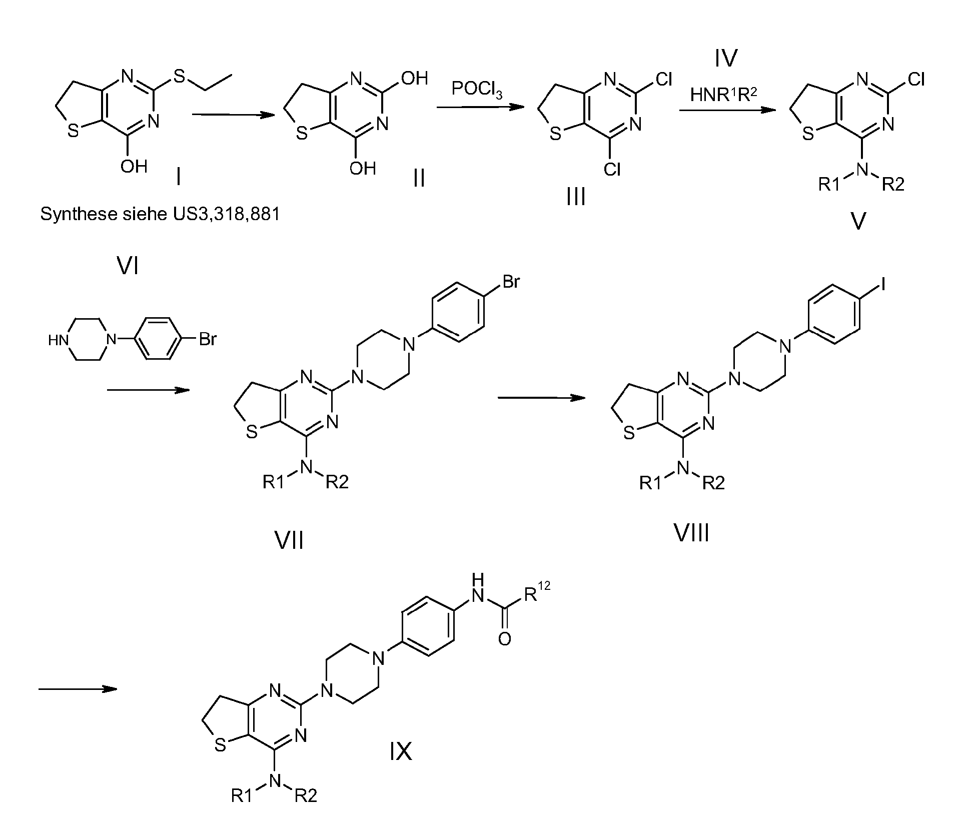

### SYNTHESE VON N-{4-[4-(4-PROPYLAMINO-6,7-DIHYDRO-THIENO[3,2-d]PYRIMIDIN-2-YL)-PIPERAZIN-1-YL]-PHENYL}-ISONICOTINAMID (IX) (NACH SCHEMA 6)

**{2-[4-(4-Brom-phenyl)-piperazin-1-yl]-6,7-dihydro-thieno[3,2-*d*]pyrimidin-4-yl}-propyl-amin (VII):** 1,0 g (4,4 mmol) (2-Chlor-6,7-dihydro-thieno[3,2-*d*]pyrimidin-4-yl)-propyl-amin (V), 4,2 g (17,4 mmol) 1-(4-Brom-phenyl)-piperazin (VI) und 2,0 ml (11,5 mmol) Diisopropylethylamin werden in 12 ml Dioxan vorgelegt, 2,5 Stunden bei 160° C in der Mikrowelle umgesetzt. Anschließend wird das Reaktionsgemisch mit Wasser und Dichlormethan versetzt und extrahiert. Die organische Phase wird mit Wasser gewaschen, getrocknet und zur Trockene eingedampft. Der Rückstand wird über Kieselgel mit Petrolether/Essigester (8/2) filtriert. Entsprechende Fraktionen wurden eingedampft. 1,9 g des Produkts VII (100%) werden erhalten.

**{2-[4-(4-Iod-phenyl)-piperazin-1-yl]-6,7-dihydro-thieno[3,2-*d*]pyrimidin-4-yl}-propylamin (VIII):** 1,55 g (3,6 mmol) {2-[4-(4-Brom-phenyl)-piperazin-1-yl]-6,7-dihydro-thieno[3,2-*d*]pyrimidin-4-yl}-propyl-amin (VII), 1,1 g (7,2 mmol) Natriumjodid und 0,036 g (0,19 mmol) Kupferjodid werden vorgelegt, unter Argon-Atmosphäre werden 0,060 ml (0,38 mmol) trans-N,N-dimethyl-1,2-cyclohexandiamin und 7 ml wasserfreies und entgastes Dioxan zugegeben. Das Reaktionsgemisch wird 1,5 Stunden bei 140° C in der Mikrowelle erhitzt, anschließend mit Dioxan verdünnt und über Alox abgesaugt. Das Dioxan wurde eingedampft. 1,70 g des Produkts VIII (81 %) werden als Pulver erhalten.

**N-{4-[4-(4-Propylamino-6,7-dihydro-thieno[3,2-*d*]pyrimidin-2-yl)-piperazin-1-yl]-phenyl}-isonicotinamid (IX) (Beispiel 237) :** 0,170 g (0,4 mmol) {2-[4-(4-lod-phenyl)-piperazin-1-yl]-6,7-dihydro-thieno[3,2-*d*]pyrimidin-4-yl}-propyl-amin (VIII), 0,054 g (0,4 mmol) Isonicotinamid, 0,100 g (0,7 mmol) Kaliumcarbonat und 0,004 g (0,02 mmol) Kupferjodid werden vorgelegt, unter Argon-Atmosphäre 1 ml wasserfreies/entgastes Dioxan und 0,006 ml (0,04 mmol) trans-N,N-dimethyl-1,2-cyclohexandiamin zugegeben. Das Reaktionsgemisch wird 2 Stunden bei 140° C in der Mikrowelle umgesetzt, danach über Kieselgel mit Petrolether/Essigester (1/1) und dann Essigester/Methanol (8/2) filtriert und eingedampft. Der Rückstand wird chromatographisch gereinigt (Säule: Microsorb, RP-C18, 300Å, 10µm, 21,4*250 mm, Fließmittel: Acetonitril + 0,1 % Ameisensäure (A), Wasser + 0,13% Ameisensäure (B))

| Gradient: Minuten | % Fließmittel A | % Fließmittel B |
|---|---|---|
| 0 | 10 | 90 |
| 4,9 | 10 | 90 |
| 10,5 | 30 | 70 |
| 20 | 30 | 70 |
| 21 | 100 | 0 |
| 25 | 100 | 0 |
| 26,5 | 10 | 90 |
| 31,5 | 10 | 90 |

0,08 g des Produkts IX (50%) werden als Pulver erhalten.
¹H NMR (400 MHz, DMSO): 10,29 (1H, s); 8,77 (2H, d); 7,85 (2H, d); 7,64 (2H, d); 7,01 (2H, d); 3,86-3,75 (4H, m); 3,23-3,07 (6H, m); 1,64-1,50 (2H, m); 0,88 (3H, t).

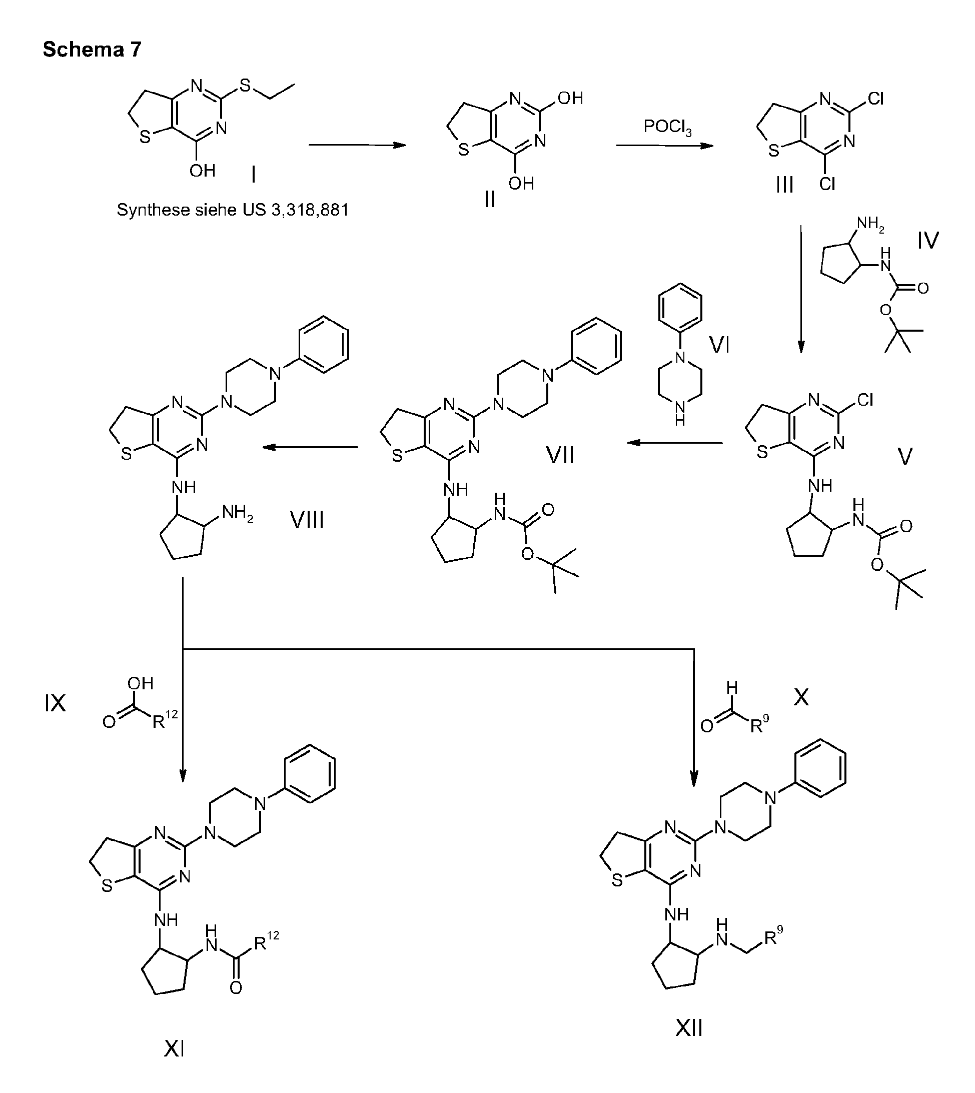

### SYNTHESE VON ISOXAZOLE-5-CARBONSÄURE-{(1S,2S)-2-[2-(4-PHENYL-PIPERAZIN-1-YL)-6,7-DIHYDRO-THIENO[3,2-d]PYRIMIDIN-4-YLAMINO]-CYCLOPENTYL}-AMID TRIFLAT (XI) (CHIRAL) (NACH SCHEMA 7)

**[(*1S*,*2S*)-2-(2-Chlor-6,7-dihydro-thieno[3,2-*d*]pyrimidin-4-ylamino)-cyclopentyl]-carbarninsäure-tert-butylester (V) (chiral):** 0,600 g (2,9 mmol) 2,4-Dichlor-6,7-dihydro-thieno[3,2-*d*]pyrimidin (III), 0,580 g (2,9 mmol) (2-Amino-cyclopentyl)-carbaminsäure-tert-butylester (IV) und 2,5 ml (14,5 mmol) Diisopropylethylamin werden in 30 ml Tetrahydrofuran vorgelegt, 2 Stunden bei Raumtemperatur und 72 Stunden bei 80° C gerührt. Das Reaktionsgemisch wird eingedampft und roh weiter umgesetzt.

**{(*1S*,*2S*)-2-[2-(4-Phenyl-piperazin-1-yl)-6,7-dihydro-thieno[3,2-*d*]pyrimidin-4-ylamino]-cyclopentyl}-carbaminsäure-tert-butylester (VII) (chiral):** 1,08 g (2,9 mmol) [(*1S*,*2S*)-2-(2-Chlor-6,7-dihydro-thieno[3,2-*d*]pyrimidin-4-ylamino)-cyclopentyl]-carbaminsäure-tert-butylester (V) (chiral) und 9,9 mmol 1-Phenyl-Piperazin (VI) in 14 ml Dioxan vorgelegt, dann 2,3 Stunden auf 160° C erhitzt. Das Reaktionsgemisch wird eingedampft, der Rückstand dann über RP-Säule mit einer HPLC gereinigt (Säule: XTerra, MS-C18, 5µm, 19*100 mm
Fließmittel: Wasser + 0,1 % Trifluoressigsäure (A), Acetonitril + 0,1% Trifluoressigsäure (B))

| Gradient: Minuten | % Fließmittel A | % Fließmittel B |
|---|---|---|
| 0 | 90 | 10 |
| 2 | 90 | 10 |
| 11,5 | 0 | 100 |
| 13 | 0 | 100 |
| 13,5 | 90 | 10 |

Entsprechende Fraktionen werden vereinigt und gefriergetrocknet. 0,97 g des Produkts VII (67%) werden erhalten.

**(*1S*,*2S*)-N-[2-(4-Phenyl-piperazin-1-yl)-6,7-dihydro-thieno[3,2-*d*]pyrimidin-4-yl]-cyclopentane-1,2-diamin ditriflat (VIII) (chiral):** 0,960 g (1,9 mmol) {(*1S*,*2S*)-2-[2-(4-Phenyl-piperazin-1-yl)-6,7-dihydro-thieno[3,2-*d*]pyrimidin-4-ylamino]-cyclopentyl}-carbaminsäure-tert-butylester (VII) (chiral) werden in 40 ml 25% Trifluoressigsäure in Dichlormethan gelöst, 1 Stunde bei Raumtemperatur gerührt. Anschließend wird das Reaktionsgemisch eingedampft, der Rückstand mit Acetonitril und Wasser versetzt und gefriergetrocknet. 1,2 g des Produkts VIII (100%) werden als Pulver erhalten.

**Isoxazole-5-carbonsäure-{(*1S*,*2S*)-2-[2-(4-phenyl-piperazin-1-yl)-6,7-dihydro-thieno[3,2-*d*]pyrimidin-4-ylamino]-cyclopentyl}-amid triflat (chiral) (XI) (Beispiel 277)** 0,004 g (0,03 mmol) Isoxazol-5-carbonsäure (IX), 0,012 g (0,03 mmol) O-(7-Azabenzotriazol-1-yl)-N,N,N',N'-tetramethyluronium-hexafluoro-phosphat (HATU) und 0,017 ml (0,10 mmol) Diisopropylethylamin werden in 1 ml Dimethylformamid vorgelegt, 0,1 Stunden bei Raumtemperatur gerührt. Anschließend werden 0,020 g (0,03 mmol) N-[2-(4-Phenyl-piperazin-1-yl)-6,7-dihydro-thieno[3,2-d]pyrimidin-4-yl]-cyclopentane-1,2-diamin ditriflat (VIII) (chiral) in 1 ml Dimethylformamid zugegeben. Es wird 1 Stunde bei Raumtemperatur geschüttelt. Danach wird das Reaktionsgemisch direkt dann über RP-Säule mit einer HPLC gereinigt (Säule: XTerra, MS-C18, 5µm, 19*100 mm, Fließmittel: Wasser + 0,1 % Trifluoressigsäure (A), Acetonitril + 0,1% Trifluoressigsäure (B))

| Gradient: Minuten | % Fließmittel A | % Fließmittel B |
|---|---|---|
| 0 | 90 | 10 |
| 2 | 90 | 10 |
| 11,5 | 0 | 100 |
| 13 | 0 | 100 |
| 13,5 | 90 | 10 |

Entsprechende Fraktionen werden vereinigt und gefriergetrocknet. 0,003 g des Produkts XI (18%) werden erhalten.

### SYNTHESE VON (1S,2S)-N-(1-METHYL-1H-PYRROL-2-YLMETHYL)-N'-[2-(4-PHENYL-PIPERAZIN-1-YL)-6,7-DIHYDRO-THIENO[3,2-d]PYRIMIDIN-4-YL]-CYCLOPENTANE-1,2-DIAMIN TRIFLAT (CHIRAL) (BEISPIEL 310) (XII) (nach SCHEMA 7)

0,015 g (0,02 mmol) N-[2-(4-Phenyl-piperazin-1-yl)-6,7-dihydro-thieno[3,2-*d*]pyrimidin-4-yl]-cyclopentan-1,2-diaminditriflat (VIII) (chiral), 0,015 ml (0,14 mmol) 1-Methyl-1H-pyrrole-2-carbaldehyd (X), 0,005 ml (0,09 mmol) Eisessig und Molekularsieb werden in 2 ml Dimethylformamid vorgelegt, 0,5 Stunden bei Raumtemperatur gerührt. Anschließend werden erst 0,070 g (0,23 mmol) Polyaminharz, dann nach weiteren 0,5 Stunden 0,030 g (0,14 mmol) Natriumtriacetoxyborhydrid zugegeben. Das Reaktionsgemisch wird 16 Stunden bei Raumtemperatur geschüttelt, filtriert und über RP-Säule mit HPLC gereinigt (Säule: XTerra, MS-C18, 5µm, 19*100 mm, Fließmittel: Wasser + 0,1% Trifluoressigsäure (A), Acetonitril + 0,1% Trifluoressigsäure (B)). Entsprechende Fraktionen werden vereinigt und lyophylisiert. 0,009 g des Produkts XII (59%) werden erhalten.
¹H NMR (400 MHz, DMSO): 7,28-7,21 (2H, m); 7,00-6,95 (2H, m); 6,85-6,78 (2H, m); 6,21-6,18 (1H, m); 6,01-5,98 (1H, m); 4,74-4,67 (1H, m); 4,19-4,12 (2H, m); 3,81-3,74 (4H, m); 3,74-3,64 (1H, m); 3,59 (3H, t); 3,20-3,13 (4H, m); 3,11-3,03 (3H, m); 2,19-2,03 (3H, m); 1,78-1,70 (4H, m); 1,67-1,54 (1H, m).

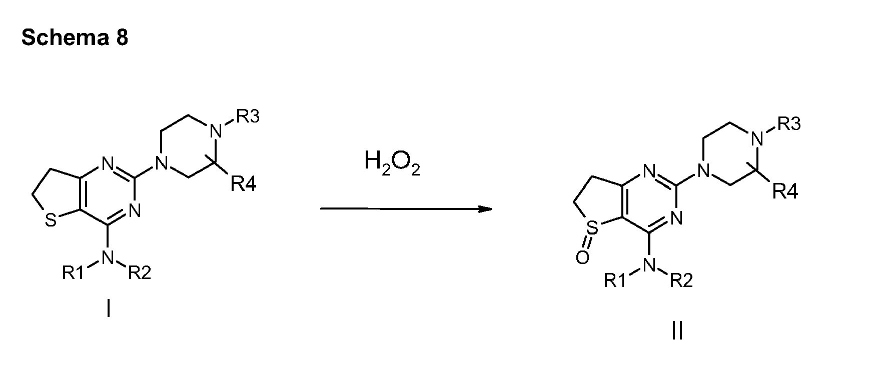

### SYNTHESE VON CYCLOHEXYL-[5-OXO-2-(4-PHENYL-PIPERAZIN-1-YL)-6,7-DIHYDRO-5H-5λ⁴-THIENO[3,2-d]PYRIMIDIN-4-YL]-AMIN (BEISPIEL 297) (II) (NACH SCHEMA 8)

0,120 g (0,3 mmol) Cyclohexyl-[2-(4-phenyl-piperazin-1-yl)-6,7-dihydro-thieno[3,2-*d*]pyrimidin-4-yl]-amin (I) werden in 4 ml Essigsäure gelöst, 0,27 ml 35%ige Wasserstoffperoxidlösung zugegeben. Es wird 16 Stunden bei Raumtemperatur gerührt, dann Wasser zugegeben. Das Reaktionsgemisch wird basisch gestellt und mit Ethylacetat extrahiert. Die organische Phase wird getrocknet und zur Trockene eingedampft. Der Rückstand wird chromatographisch gereinigt, das Produkt mit Diethylether verrieben und abgesaugt. 0,060 g des Produkts II (48%) werden erhalten.
¹H NMR (400 MHz, DMSO): 7,40 (1 H, d); 7,23 (2H, t); 6,98 (2H, d); 6,80 (1 H, t); 4,01-3,86 (5H, m); 3,48-3,37 (1 H, m); 3,28-3,13 (5H, m); 3,00-2,82 (2H, m); 1,92-1,81 (2H, m); 1,80-1,69 (2H, m); 1,67-1,57 (1 H, m); 1,43-1,21 (4H, m); 1,20-1,05 (1 H, m).

Die Enantiomeren wurden über analytischen chiralen HPLC getrennt (Säule: Chiralpak Diacel AD-H, 5 µM, 250*4,6 mm, Flußrate: 1,0 ml/min, Fließmittel: Hexan/iPrOH (90/10)): Rt = 14.6 min: Enantiomer 1 (Beispiel 367) [α]_{D}²⁰ + 183 (CH₂Cl₂); Rt = 16,4 min: Enantiomer 2 (Beispiel 368) , [α]_{D}²⁰ -189 (CH₂Cl₂).

### SYNTHESE VON [5-OXO-2-(4-PHENYL-PIPERAZIN-1-YL-6,7-DIHYDRO-5H-5λ⁴-THIENO[3,2-d]PYRIMIDIN-4-YL]-PROPYL-AMIN (BEISPIEL 298) (II) (NACH SCHEMA 8)

0,240 g (0,68 mmol) [2-(4-Phenyl-piperazin-1-yl)-6,7-dihydro-thieno[3,2-*d*]pyrimidin-4-yl]-propylamin (I) werden in 5 ml Essigsäure gelöst, 0,60 ml 35%ige Wasserstoffperoxidlösung unter Kühlung zugegeben. Es wird 16 Stunden bei Raumtemperatur gerührt, dann Wasser zugegeben. Das Reaktionsgemisch wird basisch gestellt und mit Dichlormethan extrahiert. Die organische Phase wird getrocknet und zur Trockene eingedampft. Der Rückstand wird chromatographisch gereinigt, das Produkt mit Diethylether verrieben und abgesaugt.0,130 g des Produkts II (52%) werden erhalten.
¹H NMR (400 MHz, DMSO): 7,69 (1 H, t); 7,23 (2H, t); 6,98 (2H, d); 6,80 (1 H, t); 3,95-3,87 (4H, m); 3,49-3,19 (4H, m); 3,22-3,13 (4H, m); 3,01-2,82 (2H, m); 1,64-1,52 (2H, m); 0,88 (3H, t).

### SYNTHESE VON [5-OXO-2-(4-PHENYL-PIPERAZIN-1-YL)-6,7-DIHYDRO-5H-5λ⁴-THIENO[3,2-d]PYRIMIDIN-4-YL]-PHENYLAMIN (II) (BEISPIEL 299) (NACH SCHEMA 8)

0,190 g (0,49 mmol) Phenyl-[2-(4-phenyl-piperazin-1-yl)-6,7-dihydro-thieno[3,2-*d*]pyrimidin-4-yl]-amin (I) werden in 5 ml Essigsäure gelöst, 0,43 ml 35%ige Wassersto ffperoxidlösung unter Kühlung zugegeben. Es wird 16 Stunden bei Raumtemperatur gerührt, dann Wasser zugegeben. Das Reaktionsgemisch wird basisch gestellt und mit Dichlormethan extrahiert. Die organische Phase wird getrocknet und zur Trockene eingedampft. Der Rückstand wird chromatographisch gereinigt, das Produkt mit Diethylether verrieben und abgesaugt. 0,100 g des Produkts II (51%) werden erhalten.
¹H NMR (400 MHz, DMSO): 9,54 (1H, s); 7,66 (2H, d); 7,35 (2H, t); 7,23 (2H, t); 7,09 (1H, t); 6,98 (2H, d); 6,80 (1H, t); 3,96-3,85 (4H, m); 3,60-3,47 (1H, m); 3,32-3,15 (4H, m); 3,12-3,02 (1H, m); 3,01-2,92 (1H, m).

Folgende Beispiele werden analog oben beschrieben nach Schema 8 hergestellt:

### (3-METHOXY-PHENYL)-[5-OXO-2-(4-PHENYL-PIPERAZIN-1-YL)-6,7-DIHYDRO-5H-5λ⁴-THIENO[3,2-d]PYRIMIDIN-4-YL]-AMIN (Beispiel 302) (II nach SCHEMA 8)

0,072 g des Produkts II (17%) werden als Pulver (Smp 217°-220° C) erhalten.

(3-CHLOR-PHENYL)-[5-OXO-2-(4-PHENYL-PIPERAZIN-1-YL)-6,7-DIHYDRO-5*H*-5λ⁴-THIENO[3,2-d]PYRIMIDIN-4-YL]-AMIN (BEISPIEL 300 ) (II NACH SCHEMA 8) 4,50 g des Produkts II (73%) werden als Pulver (Smp 230°-231 °C) erhalten.

Die Enantiomeren wurden über semipräparativen chiralen HPLC getrennt (Säule: Chiralpak Diacel IA, 5 µM, 200*25 mm, Flußrate: 12 ml/min, Fließmittel: TBME/EtOH (75/25)): Rt = 16 min: Enantiomer 1 (BEISPIEL 370), [α]_{D}²⁰ + 221.3 (c 2,06, CH₂Cl₂).; Rt = 19 min: Enantiomer 2 (BEISPIEL 371), [α]_{D}²⁰ - 207.9 (c 2.24, CH₂Cl₂).

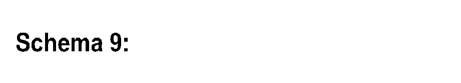

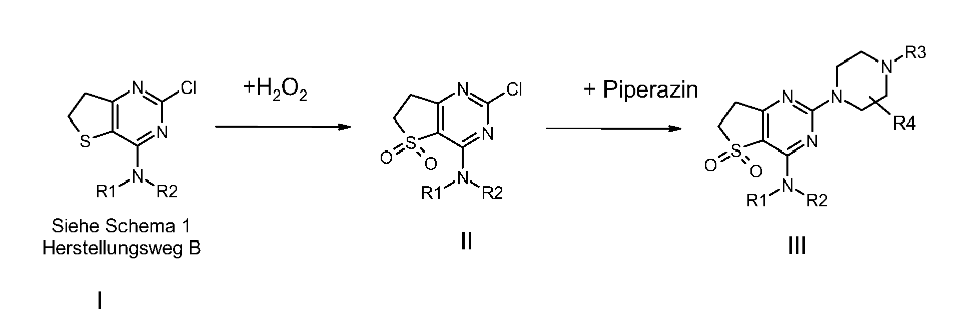

### SYNTHESE VON {2-[4-(4-CHLOR-PHENYL)-PIPERAZIN-1-YL]-5,5-DIOXO-6,7-DIHYDRO-5H-5λ⁶-THIENO[3,2-d]PYRIMIDIN-4-YL}-PROPYL-AMIN (BEISPIEL 377) (III NACH SCHEMA 9)

**(2-Chlor-5,5-dioxo-6,7-dihydro-5*H*-5λ⁶-thieno[3,2-*d*]pyrimidin-4-yl)-propyl-amin (II):** 0,805 g (3,50 mmol) (2-Chlor-6,7-dihydro-thieno[3,2-*d*]pyrimidin-4-yl)-propyl-amin (I) (siehe Schema 1, Herstellungsweg B) werden bei Raumtemperatur in 14 ml Trifluoressigsäure vorgelegt, dann mit 0,82 ml (8,40 mmol) Wasserstoffperoxid (35% in Wasser) versetzt. Das Reaktionsgemisch wird 16 Stunden bei Raumtemperatur und 2 Stunden bei 45° C gerührt. Nach Zugabe von 0,5 eq Wasserstoffperoxid wird noch 0,5 Stunden bei 45° C gerührt, dann in Eiswasser gerührt und mit Ammoniaklösung basisch gestellt. Ausfallender Niederschlag wird abgesaugt, gewaschen und getrocknet. 0,720 g des Produkts II (79%) werden als Pulver erhalten.

**{2-[4-(4-Chlor-phenyl)-piperazin-1-yl]-5,5-dioxo-6,7-dihydro-5*H*-5λ⁶-thieno[3,2-*d*]yrimidin-4-yl}-propyl-amin (III):** 0,262 g (1,00 mmol) (2-Chlor-5,5-dioxo-6,7-dihydro-5*H*-5λ⁶-thieno[3,2-*d*]pyrimidin-4-yl)-propyl-amin (II) und 0,433 g (2,20 mmol) 1-(4-Chlorphenyl)-piperazin werden in 4,50 ml Dioxan vorgelegt, dann in der Mikrowelle 0,75 Stunden auf 150°C erhitzt. Anschließend wird das Reaktionsgemisch eingedampft, der Rückstand mit Wasser behandelt. Es wird abgesaugt, mit Wasser gewaschen und getrocknet. Das noch unsaubere Produkt wird mittels Chromatographie gereinigt (Säule: 10 g Chromabond SiOH Kartusche, Lösungsmittel: Petrolether/Ether 1:1). Entsprechende Fraktionen werden vereint und eingedampft. 0,295 g des Produkts III (70%) werden als Pulver (Smp 243°-245° C) erhalten.

Folgende Beispiele werden analog oben beschrieben nach Schema 9 hergestellt:

### (3-CHLOR-PHENYL)-[5,5-DIOXO-2-(4-PHENYL-PIPERAZIN-1-YL)-6,7-DIHYDRO-5H-5λ⁶-THIENO[3,2-d]PYRIMIDIN-4-YL]-AMIN (BEISPIEL 375) (III nach SCHEMA 9)

0,163 g des Produkts III (36%) werden als Pulver (Smp 234° C) erhalten.

### (3-CHLOR-PHENYL)-{2-[4-(4-CHLORO-PHENYL)-PIPERAZIN-1-YL]-5,5-DIOXO-6,7-DIHYDRO-5H-5λ⁶-THIENO[3,2-d]PYRIMIDIN-4-YL}-AMIN (BEISPIEL 376) (III NACH SCHEMA 9)

0,173 g des Produkts III (35%) werden als Pulver (Smp 246° C) erhalten.

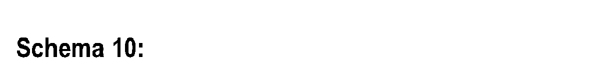

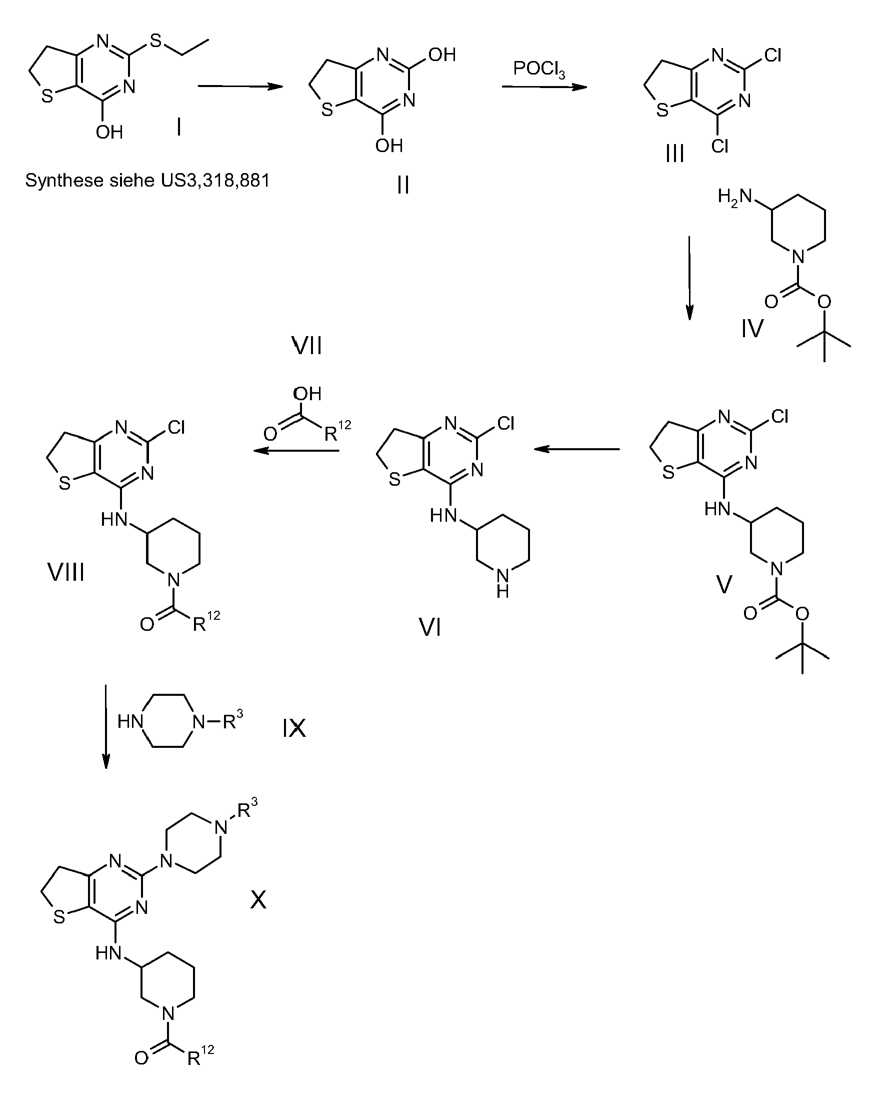

### SYNTHESE VON (1-METHYL-1 H-PYRROL-2-YL)-{3-[2-(4-(4-CHLOR-PHENYL)-PIPERAZIN-1-YL]-6,7-DIHYDRO-THIENO[3,2-d]PYRIMIDIN-4-YLAMINO]-PIPERIDIN-1-YL}-METHANON (X NACH SCHEMA 10)

**3-(2-Chlor-6,7-dihydro-thieno[3,2-*d*]pyrimidin-4-ylamino)-piperidine-1-carbonsäure tert-butylester (V):** 2,07 g (10,0 mmol) 2,4-Dichlor-6,7-dihydro-thieno[3,2-*d*]pyrimidin (III), 2,0 g (10,0 mmol) 3-Amino-piperidin-1-carbonsäure-tert-butylester (IV) und 3,4 ml (19,3 mmol) Diisopropylethylamin werden in 40 ml Tetrahydrofuran vorgelegt, dann 40 Stunden bei Raumtemperatur gerührt. Anschließend wird das Reaktionsgemisch abgesaugt, die Mutterlauge eingedampft. Der Rückstand wird mit Wasser versetzt und mit Dichlormethan extrahiert. Die organische Phase wird mittels Phasenseparator abgetrennt und zur Trockene eingedampft. Das Rohprodukt wird chromatographisch über eine Biotage Kieselgel Kartusche 40M mit Petrolether/Essigester 9:1 gereinigt. 1,77 g des Produkts V (48%) werden erhalten.

**(2-Chlor-6,7-dihydro-thieno[3,2-*d*]pyrimidin-4-yl)-piperidin-3-yl-amin hydrochlorid (VI):** 1,77 g (4,8 mmol) 3-(2-Chloro-6,7-dihydro-thieno[3,2-d]pyrimidin-4-ylamino)-piperidin-1-carbonsäure-tert-butylester (V) werden in 21,5 ml einer 4%igen Salzsäurelösung in Dioxan vorgelegt, Methanol zugegeben. Die Lösung wird 0,5 Stunden bei Raumtemperatur gerührt, dabei fällt ein Niederschlag aus. Dieser wird abgesaugt, mit Diethylether gewaschen und getrocknet. 1,33 g des Produkts (VI) 91%) werden erhalten.

**(1-methyl-1*H*-pyrrol-2-yl)-[3-(2-Chlor-6,7-dihydro-thieno[3,2-*d*]pyrimidin-4-ylamino)-piperidin-1-yl]-methanon (VIII):** 0,462 g (3,7 mmol) 1-Methyl-1H-pyrrol-2-carbonsäure (VII), 1,4 g (9,7 mmol) O-(7-Azabenzotriazol-1-yl-)-N,N,N',N'-tetramethyluronium-hexafluoro-phosphat (HATU) und 0,640 ml (3,7 mmol) Diisopropylethylamin werden in 10 ml Dimethylsulfoxid vorgelegt, dann 0,1 Stunden bei Raumtemperatur gerührt. 0,814 g (3,0 mmol) (2-Chlor-6,7-dihydro-thieno[3,2-d]pyrimidin-4-yl)-piperidin-3-yl-amin hydrochlorid (VI) und 3,7 mmol Diisopropylethylamin werden zugegeben, dann 16 Stunden bei Raumtemperatur gerührt. Anschließend wird das Reaktionsgemisch über Alox abgesaugt, die Mutterlauge eingedampft. Der Rückstand wird mit 1 N Natronlauge versetzt und mit Dichlormethan extrahiert. Die organisch Phase wird mittels Phasenseparator abgetrennt und zur Trockene eingedampft. Das Rohprodukt wird chromatographisch über eine Biotage Kieselgel Kartusche 40s mit Petrolether/Essigester 1:1 gereinigt. 0,970 g des Produkts VIII (85%) werden erhalten.

**(1-methyl-1*H*-pyrrol-2-yl)-{3-[2-(4-(4-Chlor-phenyl)-piperazin-1-yl]-6,7-dihydro-thieno[3,2-*d*]pyrimidin-4-ylamino]-piperidin-1-yl}-methanon (X nach Schema 10) (Beispiel 230) :** 0,250 g (0,7 mmol) (1-methyl-1*H*-pyrrol-2-yl)-[3-(2-Chlor-6,7-dihydro-thieno[3,2-*d*]pyrimidin-4-ylamino)-piperidin-1-yl]-methanon (VIII), 0,503 g (1,9 mmol) 1-(4-Chloro-phenyl)-piperazin dihydrochlorid (IX) und 0,430 ml (2,5 mmol) Diisopropylethylamin werden in 3,5 ml Dioxan vorgelegt, dann 2,25 Stunden bei 160° C in der Mikrowelle umgesetzt. Anschließend wird das Reaktionsgemisch mit Wasser versetzt. Ausfallender Niederschlag wird abgesaugt, mit Wasser gewaschen und getrocknet. 0,167 g des Produkts X (47%) werden als beiges Pulver erhalten.
¹H NMR (400 MHz, DMSO): 7,25 (2H, d); 6,97 (2H, d); 6,84 (1 H, t); 6,34-6,31 (1 H, m), 5,96 (1 H, m); 4,46-4,34 (1 H, m); 4,22-4,12 (1 H, m); 4,04-4,92 (1 H, m); 3,73-3,64 (4H, m); 3,67 (3H, s); 3,24 (2H, t); 3,16-3,09 (4H, m); 3,05-2,84 (4H, m); 1,97-1,88 (1 H, m); 1,82-1,65 (2H, m); 1,55-1,43 (1 H, m).

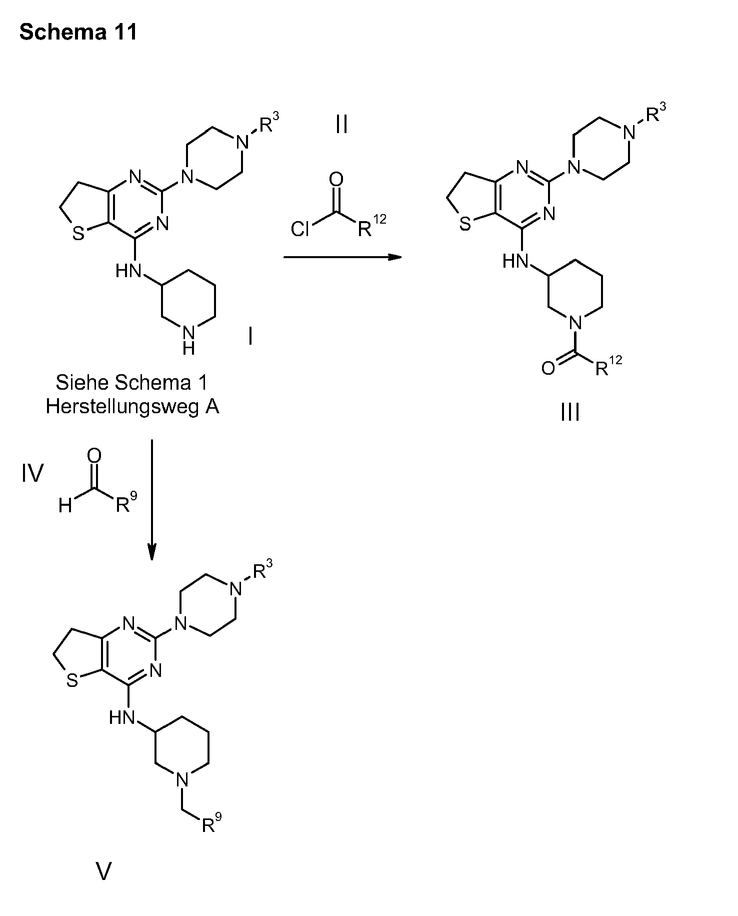

### SYNTHESE VON (3-{2-[4-(4-CHLOR-PHENYL)-PIPERAZIN-1-YL]-6,7-DIHYDRO-THIENO[3,2-d]PYRIMIDIN-4-YLAMINO}-PIPERIDIN-1-YL)-MORPHOLIN-4-YL-METHANON TRIFLAT (BEISPIEL 357) (III NACH SCHEMA 11):

0,135 g (0,31 mmol) {2-[4-(4-Chlor-phenyl)-piperazin-1-yl]-6,7-dihydro-thieno[3,2-*d*]pyrimidin-4-yl}-piperidin-3-yl-amin (I) (siehe Schema 1), 0,037 ml (0,31 mmol) Morpholine-4-carbonyl chlorid (II), 0,119 g (0,31 mmol) O-(7-Azabenzotriazol-1-yl-)-N,N,N',N'-tetramethyluronium-hexafluoro-phosphat (HATU) und 0,218 ml (1,25 mmol) Diisopropylethylamin werden in 6 ml Dimethylformamid 18 Stunden bei Raumtemperatur gerührt. Das Reaktionsgemisch wird eingedampft, der Rückstand chromatographisch (RP-HPLC) gereinigt. 0,040 g des Produktes III (19%) werden nach Gefriertrocknung erhalten.
¹H NMR (400 MHz, DMSO): 7,26 (2H, d); 7,00 (2H, d); 4,13-3,95 (m); 3,87-3,76 (m); 3,68-3,60 (m); 3,59-3,43 (m); 3,43-3,34 (m); 3,31-3,09 (m); 2,88-2,77 (m); 1,95-1,83 (1 H, m); 1,78-1,60 (2H, m); 1,57-1,40 (m).

### [2-(4-PHENYL-PIPERAZIN-1-YL)-6,7-DIHYDRO-THIENO[3,2-d]PYRIMIDIN-4-YL]-(1-PYRIDIN-3-YLMETHYL-PIPERIDIN-3-YL)-AMIN DITRIFLAT (BEISPIEL363) (V nach SCHEMA 11)

0,015 g (0,024 mmol) {2-[4-(4-Chlor-phenyl)-piperazin-1-yl]-6,7-dihydro-thieno[3,2-*d*]pyrimidin-4-yl}-piperidin-3-yl-amin (I) (siehe Schema 1), 0,015 ml (0,158 mmol) Pyridin-3-aldehyd (IV) und 0,005 ml (0,087 mmol) Eisessig werden in 2 ml DMF 0,5 Stunde bei Raumtemperatur über Molekularsieb gerührt. 0,026 mg (0,123 mmol) Natriumtriacetoxyborhydrid werden dazu gegeben und das Reaktionsgemisch 12 Stunden bei Raumtemperatur weiter gerührt. Das Reaktionsgemisch wird chromatographisch (RP-HPLC) gereinigt. 0,009 g des Produkts V (50%) werden nach Gefriertrocknung erhalten. ¹H NMR (400 MHz, DMSO): 8,76-8,70 (1 H, m); 8,65-8,58 (1 H, m); 8,02-7,94 (1 H, m); 7,56-7,49 (1 H, m); 7,33-7,21 (3H, m); 7,04-6,97 (2H, m); 6,89-6,81 (1 H, m); 4,57-3,78 (m); 3,75-3,40 (m); 3,37-3,28 (2H, m); 3,20-3,03 (m); 2,99-2,79 (1 H, m); 2,78-2,59 (1 H, m); 2,07-1,71 (3H, m); 1,69-1,52 (1 H, m).

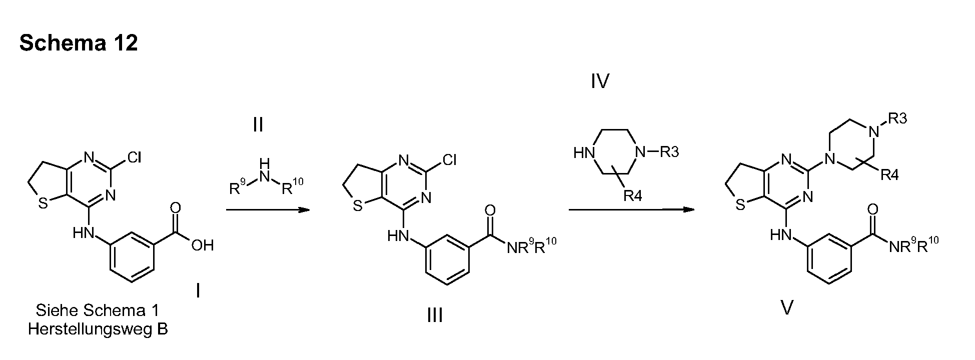

### SYNTHESE VON (4-METHYL-PIPERAZIN-1-YL)-(3-{2-[4-(4-CHLOR-PHENYL)-PIPERAZIN-1-YL]-6,7-DIHYDRO-THIENO[3,2-d]PYRIMIDIN-4-YLAMINO}-PHENYL)-METHANON (V NACH SCHEMA 12)

**3-(2-Chlor-6,7-dihydro-thieno[3,2-*d*]pyrimidin-4-ylamino)-benzoesäure (I):** 0,200 g (0,93 mmol) 2,4-Dichlor-6,7-dihydro-thieno[3,2-*d*]pyrimidin, 0,127 g (0,93 mmol) 3-Aminobenzoesäure und 0,323 ml (1,85 mmol) Diisopropylethylamin werden in 4 ml Tetrahydrofuran vorgelegt, 48 Stunden bei Raumtemperatur und 48 Stunden bei 70° C gerührt. Anschließend wird das Reaktionsgemisch mit Wasser versetzt, mit 1 N Salzsäure angesäuert. Ausfallender Niederschlag wird abgesaugt und getrocknet. 0,110 g des Produkts I (39%) werden erhalten.

**(4-methyl-piperazin-1-yl)-[3-(2-Chlor-6,7-dihydro-thieno[3,2-*d*]pyrimidin-4-ylamino)-phenyl]-methanon (III):** 0,600 g (1,95 mmol) 3-(2-Chlor-6,7-dihydro-thieno[3,2-*d*]pyrimidin-4-ylamino)-benzoesäure (I), 0,741 g (1,95 mmol) O-(7-Azabenzotriazol-1-yl-)-N,N,N',N'-tetramethyluronium-hexafluoro-phosphat (HATU) und 0,680 ml (3,90 mmol) Diisopropylethylamin werden in 10 ml Dimethylsulfoxid vorgelegt, dann 0,5 Stunden bei Raumtemperatur gerührt. 0,220 ml (1,95 mmol) Methylpiperazin (II) werden zugegeben, dann 3 Stunden bei Raumtemperatur gerührt. Anschließend wird das Reaktionsgemisch eingedampft, der Rückstand mit Wasser versetzt. Ausfallender Niederschlag wird abgesaugt und getrocknet. Das Rohprodukt wird chromatographisch über eine 50 g Kieselkartusche mit Essigester/Methanol 8:2 gereinigt. 0,250 g des Produkts III (33%) werden erhalten.

**(4-methyl-piperazin-1-yl)-(3-{2-[4-(4-Chlor-phenyl)-piperazin-1-yl]-6,7-dihydro-thieno[3,2-*d*]pyrimidin-4-ylamino}-phenyl)-methanon (V) (Beispiel 236) :** 0,100 g (0,26 mmol) (4-methyl-piperazin-1-yl)-[3-(2-Chlor-6,7-dihydro-thieno[3,2-*d*]pyrimidin-4-ylamino)-phenyl]-methanon (III), 0,207 g (0,77 mmol) 1-(4-Chlor-phenyl)-piperazin (IV) und 0,180 ml (1,02 mmol) Diisopropylethylamin werden in 3,5 ml Dioxan vorgelegt, dann insgesamt 2 Stunden bei 160° C in der Mikrowelle umgesetzt. Anschließend wird das Reaktionsgemisch mit Wasser versetzt und mit Ethylacetat extrahiert. Die organische Phase wird getrocknet und zur Trockene eingedampft. Der Rückstand wird chromatographisch über RP-Säule mit einer HPLC gereinigt (Säule: Microsorb, RP-C18, 300Å, 10µm, 21,4*250 mm, Fließmittel: Acetonitril + 0,1 % Ameisensäure (A), Wasser + 0,13% Ameisensäure (B))

| Gradient: Minuten | % Fließmittel A | % Fließmittel B |
|---|---|---|
| 0 | 10 | 90 |
| 4,9 | 10 | 90 |
| 27 | 100 | 0 |
| 32 | 100 | 0 |
| 32,5 | 10 | 90 |
| 37,5 | 10 | 90 |

Entsprechende Fraktionen vereinigt und gefriergetrocknet. 0,014 g des Produkts V (10%) werden erhalten.
¹H NMR (400 MHz, DMSO): 8,60 (1 H, s); 7,76-7,70 (2H, m); 7,37 (1 H, t); 7,25 (2H, d); 7,03 (1H, d); 6,98 (2H, d); 3,81-3,72 (5H, m); 3,72-3,37 (3H, m); 3,21-3,14 (4H, m); 3,10 (3H, t); 2,97-2,62 (4H, m).

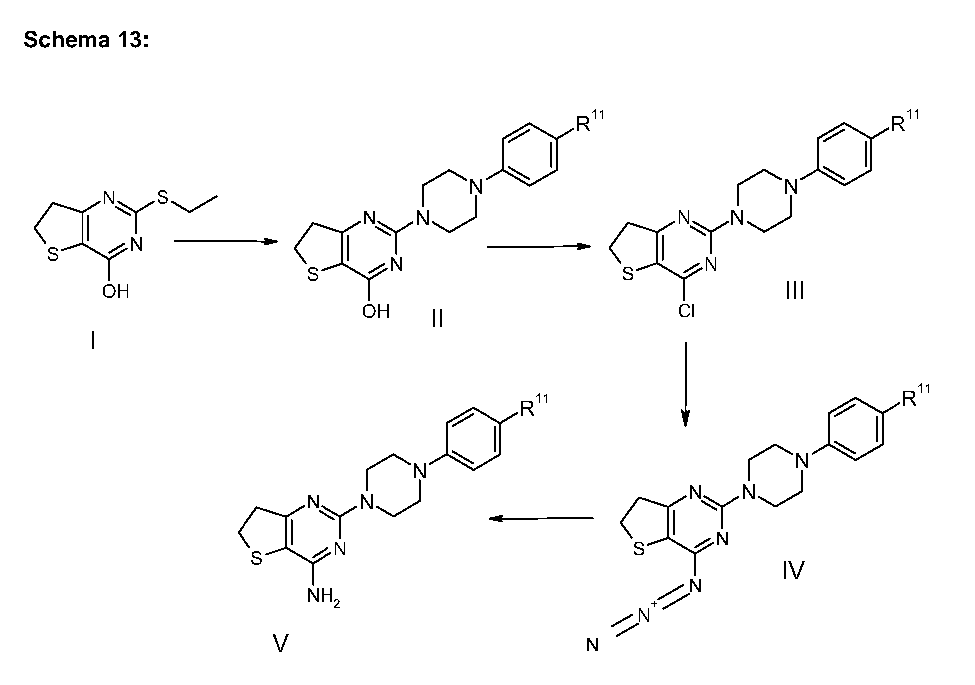

### SYNTHESE VON 4-[4-(4-AMINO-6,7-DIHYDRO-THIENO[3,2-d]PYRIMIDIN-2-YL)-PIPERAZIN-1-YL]-PHENOL (V NACH SCHEMA 13)

**2-[4-(4-Hydroxy-phenyl)-piperazin-1-yl]-6,7-dihydro-thieno[3,2-*d*]pyrimidin-4-ol (II):** 1,70 g (9.54 mmol) 4-Piperazin-1-yl-phenol werden in 0,55 ml (9,62 mmol) Eisessig vorgelegt und im Heizblock auf 180° C erwärmt. 0,800 g (3,73 mmol) 2-Ethylsulfanyl-6,7-dihydro-thieno[3,2-*d*]pyrimidin-4-ol (I) werden zugegeben, dann 1,5 Stunden bei 180° C und 16 Stunden bei Raumtemperatur stehen lassen. Anschließend wird das Reaktionsgemisch mit Wasser versetzt und im Ultraschallbad behandelt. Niederschlag wird abgesaugt, gewaschen und getrocknet. 1.1 g des Produkts II werden als Pulver erhalten.

**4-[4-(4-Chlor-6,7-dihydro-thieno[3,2-*d*]pyrimidin-2-yl)-piperazin-1-yl]-phenol (III):** 1,11 g (3,36 mmol) 2-[4-(4-Hydroxy-phenyl)-piperazin-1-yl]-6,7-dihydro-thieno[3,2-*d*]pyrimidin-4-ol (II) werden in 5 ml Phosphoroxychlorid vorgelegt, dann 4 Stunden bei 120° C gerührt. Danach wird überschüssiges Phosphoroxychlorid abdestilliert, der Rückstand mit Wasser versetzt. Ausfallender Niederschlag wird abgesaugt, mit viel Wasser gewaschen und getrocknet. 1.18 g des Produkts III werden als braunes Pulver erhalten.

**4-[4-(4-Azido-6,7-dihydro-thieno[3,2-*d*]pyrimidin-2-yl)-piperazin-1-yl]-phenol (IV):** 1,18 g (2,75 mmol) 4-[4-(4-Chlor-6,7-dihydro-thieno[3,2-*d*]pyrimidin-2-yl)-piperazin-1-yl]-phenol (III) werden in 25 ml Dimethylformamid vorgelegt, 1,20 g (18,46 mmol) Natriumazid zugegeben. Das Reaktionsgemisch wird 4,5 Stunden bei 100° C gerührt. Anschließend wird eingedampft, der Rückstand im Eisbad gekühlt und mit Wasser versetzt. Ausfallender Niederschlag wird abgesaugt, gewaschen und getrocknet. 0,800 g des Produkts IV werden erhalten und als Rohprodukt weiter verwendet.

**4-[4-(4-Amino-6,7-dihydro-thieno[3,2-*d*]pyrimidin-2-yl)-piperazin-1-yl]-phenol (V) (Beispiel 218) :** 0,800 g (1,80 mmol) 4-[4-(4-Azido-6,7-dihydro-thieno[3,2-*d*]pyrimidin-2-yl)-piperazin-1-yl]-phenol (IV) werden in 10 ml Tetrahydrofuran vorgelegt, 0,500 g Molsieb zugegeben. 3,80 ml (3,80 mmol.) Lithiumaluminiumhydrid (1 M Lösung in Tetrahydrofuran) werden langsam zugetropft. Das Reaktionsgemisch wird 3 Stunden bei Raumtemperatur gerührt. Anschließend werden 0,55 ml 1N Natronlauge und 0,50 ml Wasser zugegeben, 0,3 Stunden gekocht, dann wieder abgekühlt. Es wird filtriert, das Filtrat wird getrocknet und zur Trockene eingedampft. Der Rückstand wird mit Essigester und 1 N Salzsäure extrahiert, die Wasserphase basisch gestellt und mit Essigester extrahiert. Die organische Phase wird mit Wasser gewaschen, getrocknet und zur Trockene eingedampft. 0,280 g des Produkts V werden als hellbrauner Schaum erhalten. ¹H NMR (400 MHz, DMSO): 8,80 (1H, s); 6,82 (2H, d); 6,66 (2H, d); 6,26 (2H, s); 3,77-3,66 (4H, m); 3,21 (2H, t); 2,98 (2H, t); 2,98-2,90 (4H, m).

### SYNTHESE VON 2-[4-(4-CHLOR-PHENYL)-PIPERAZIN-1-YL]-6,7-DIHYDRO-THIENO[3,2-d]PYRIMIDIN-4-YLAMIN (V NACH SCHEMA 13)

**2-[4-(4-Chlor-phenyl)-piperazin-1-yl]-6,7-dihydro-thieno[3,2-*d*]pyrimidin-4-ol (II):** 1,9 g (9,66 mmol) 1-(4-Chlorphenyl)piperazin werden in 0,55 ml (9,62 mmol) Eisessig vorgelegt und im Heizblock auf 180° C erwärmt. 0,800 g (3,73 mmol) 2-Ethylsulfanyl-6,7-dihydro-thieno[3,2-*d*]pyrimidin-4-ol (I) werden zugegeben, dann 1,5 Stunden bei 180° C und 16 Stunden bei Raumtemperatur stehen lassen. Anschließend wird das Reaktionsgemisch mit Wasser versetzt und im Ultraschallbad behandelt. Niederschlag wird abgesaugt, gewaschen und getrocknet. 1,25 g 2 des Produkts II werden erhalten und roh weiter verwendet.

**4-Chlor-2-[4-(4-chlor-phenyl)-piperazin-1-yl]-6,7-dihydro-thieno[3,2-*d*]pyrimidin (III):**1,25 g (3,05 mmol) 2-[4-(4-Chlor-phenyl)-piperazin-1-yl]-6,7-dihydro-thieno[3,2-*d*]pyrimidin-4-ol (II) werden in 4,50 ml Phosphoroxychlorid vorgelegt, dann 4 Stunden bei 120° C gerührt. Danach wird überschüssiges Phosphoroxychlorid abdestilliert, der Rückstand mit Wasser versetzt. Ausfallender Niederschlag wird abgesaugt, mit viel Wasser gewaschen und getrocknet, danach mit Methanol ausgerührt. 0,960 g des Produkts III (85%) werden erhalten.

**4-Azido-2-[4-(4-chlor-phenyl)-piperazin-1-yl]-6,7-dihydro-thieno[3,2-*d*]pyrimidin (IV):** 0,950 g (2,59 mmol) 4-Chlor-2-[4-(4-chlor-phenyl)-piperazin-1-yl]-6,7-dihydro-thieno[3,2-*d*]pyrimidin (III) werden in 20 ml Dimethylformamid vorgelegt, 0,900 g (13,84 mmol) Natriumazid zugegeben. Das Reaktionsgemisch wird 4 Stunden bei 100° C gerührt. Anschließend wird eingedampft, der Rückstand im Eisbad gekühlt und mit Wasser versetzt. Ausfallender Niederschlag wird abgesaugt, gewaschen und getrocknet. 0,920 g des Produkts IV (76%) werden erhalten und roh weiter eingesetzt.

**2-[4-(4-Chlor-phenyl)-piperazin-1-yl]-6,7-dihydro-thieno[3,2-*d*]pyrimidin-4-ylamin (Beispiel 219) (V):** 0,820 g (1,76 mmol) 4-Azido-2-[4-(4-chlor-phenyl)-piperazin-1-yl]-6,7-dihydro-thieno[3,2-*d*]pyrimidin (IV) werden in 10 ml Tetrahydrofuran vorgelegt, 3,80 ml (3,80 mmol.) Lithiumaluminiumhydrid (1 molare Lösung in Tetrahydrofuran) werden langsam zugetropft. Das Reaktionsgemisch wird 4 Stunden bei Raumtemperatur gerührt. Anschließend werden 0,55 ml 1N Natronlauge und 0,50 ml Wasser zugegeben, 0,3 Stunden gekocht, dann wieder abgekühlt. Es wird filtriert, das Filtrat wird getrocknet und zur Trockene eingedampft. Der Rückstand wird mit Ethylacetat und 1 N Salzsäure extrahiert. Das Produkt fällt aus, wird abgesaugt, dann mit Ethylacetat und 1 N Natronlauge extrahiert. Die organische Phase wird mit Wasser gewaschen, getrocknet und zur Trockene eingedampft. Der Rückstand wird mit Petrolether ausgerührt. 0,513 g des Produkts V (84%) werden als Feststoff (Smp 176-178°C) erhalten.
¹H NMR (400 MHz, DMSO): 7,24 (2H, d); 6,98 (2H, d); 6,30 (2H, s); 3,77-3,68 (4H, m); 3,22 (2H, t); 3,17-3,09 (4H, m); 2,99 (2H, t).

### SYNTHESE VON SULFURIC ACID MONO-{4-[4-(4-PROPYLAMINO-6,7-DIHYDRO-THIENO[3,2-d]PYRIMIDIN-2-YL)-PIPERAZIN-1-YL]-PHENYL} ESTER (V NACH SCHEMA 13) (BEISPIEL 291)

0,100 g (0,269 mmol) {2-[4-(4-Hydroxy-phenyl)-piperazin-1-yl]-6,7-dihydro-thieno[3,2-*d*]pyrimidin-4-yl}-propyl-amin werden bei Raumtemperatur in 3 ml Pyridin gelöst, 0,009 g (0,0540 mmol) Kaliumjodid zugegeben. Dann werden 0,089 ml (1,34 mmol) Chlorsulfonsäure unter Eiskühlung zugetropft. Nach der Zugabe wird das Reaktionsgemisch fest, es werden 3 ml Tetrahydrofuran zugegeben. Anschließend wird mit Wasser und Dichlormethan versetzt, ausfallender Niederschlag wird abgesaugt und getrocknet. 0,049 g des Produkts (33%) werden als Pulver erhalten.
¹H NMR (400 MHz, DMSO+DCI): 9,01-8,92 (1 H, m); 8,21-8,10 (1 H, m); 7,90-7,80 (1 H, m); 7,79-7,70 (1 H, m); 7,40-7,31 (1 H, m); 7,02-6,92 (1 H, m); 4,49-4,27 (4H, m); 3,82-3,64 (4H, m); 3,57-3,30 (6H, m); 1,65-1,50 (2H, m); 0,96-0,77 (3H, m).

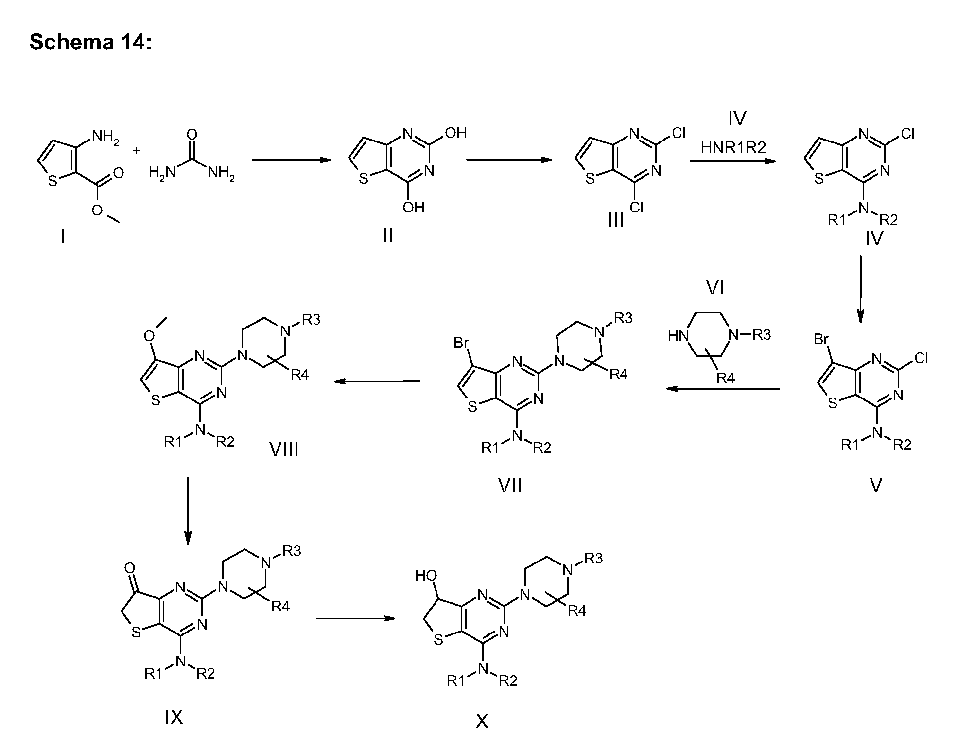

### SYNTHESE VON 2-[4-(4-CHLOR-PHENYL)-PIPERAZIN-1-YL]-4-PROPYLAMINO-6,7-DIHYDRO-THIENO[3,2-d]PYRIMIDIN-7-OL TRIFLAT ((BEISPIEL 378) (X nach Schema 14)

**Thieno[3,2-*d*]pyrimidin-2,4-diol (II):** 10,0 g (64,0 mmol) 3-Aminothiophen-2-carbonsäure-methylester (I) und 19,0 g (31,60 mmol) Harnstoff werden vermischt und 2 Stunden bei 200° C geschmolzen. Nach Abkühlen wird das Reaktionsgemisch in 1 molarer Natronlauge gelöst und mit Aktivkohle entfärbt. Es wird filtriert, das Filtrat abgekühlt und mit 4molarer Salzsäure sauer gestellt. Der ausfallende Niederschlag wird abgesaugt und getrocknet. 8,03 g des Produkts II (75%) werden als Pulver erhalten.

**2,4-Dichlor-thieno[3,2-*d*]pyrimidin (III):** 7,97 g (47,0 mmol) Thieno[3,2-*d*]pyrimidin-2,4-diol (II) werden in 50 ml (54,5 mmol) Phosphoroxychlorid vorgelegt, dann 14 Stunden unter Rückfluss gerührt. Anschließend wird eingedampft, der Rückstand mit Eiswasser versetzt. Der ausfallende Niederschlag wird abgesaugt und getrocknet. 9,00 g der Produkts III (93%) werden als Pulver erhalten.

**(2-Chlor-thieno[3,2-*d*]pyrimidin-4-yl)-propyl-amin (IV):** 3,95 g (19,26 mmol) 2,4-Dichlor-thieno[3,2-*d*]pyrimidin (III), 1,90 ml (23,11 mmol) Propylamin und 6,71 ml (38,52 mmol) Diisopropylethylamin werden in 40 ml Tetrahydrofuran 16 Stunden bei Raumtemperatur gerührt. Anschließend wird das Reaktionsgemisch eingedampft, der Rückstand mit Wasser versetzt. Der ausfallende Niederschlag wird abgesaugt und getrocknet. 4,03 g des Produkts IV (92%) werden als Pulver erhalten.

**(7-Brom-2-chlor-thieno[3,2-*d*]pyrimidin-4-yl)-propyl-amin (V):** 8,34 g (36,63 mmol) (2-Chlor-thieno[3,2-*d*]pyrimidin-4-yl)-propyl-amin (IV) werden in 60 ml Acetonitril gelöst, 7,89 g (44,32 mmol) N-Bromsuccinimid zugegeben. Das Reaktionsgemisch wird 16 Stunden bei Raumtemperatur und 6 Stunden bei 50° C gerührt. Anschließend wird eingedampft, der Rückstand mit Wasser versetzt. Ausfallender Niederschlag wird abgesaugt, gewaschen und getrocknet. 7,25 g des Produkts V (61%) werden als Pulver erhalten.

**{7-Brom-2-[4-(4-chlor-phenyl)-piperazin-1-yl]-thieno[3,2-*d*]pyrimidin-4-yl}-propylamin (VII):** 2,00 g (6,20 mmol) (7-Brom-2-chlor-thieno[3,2-*d*]pyrimidin-4-yl)-propyl-amin (V), 4,64 g (23,59 mmol) 1-(4-Chlor-phenyl)-piperazin (VI) und 2,13 ml (12,39 mmol) Diisopropylethylamin werden in 15 ml Dioxan vorgelegt, in der Mikrowelle 0,75 Stunden auf 100°C erhitzt. Da nur zu 50% umgesetzt, wird das Reaktionsgemisch noch 1,5 Stunden in der Mikrowelle auf 160° C erhitzt. Anschließend wird ausgefallener Niederschlag abgesaugt, mit Wasser gewaschen und mit Petrolether verrührt. Nach erneutem Absaugen werden 3,19 g des Produkts VII (100%) erhalten.

**{2-[4-(4-Chlor-phenyl)-piperazin-1-yl]-7-methoxy-thieno[3,2-*d*]pyrimidin-4-yl}-propylamin (VIII):** 2,00 g (4,28 mmol) {7-Brom-2-[4-(4-chlor-phenyl)-piperazin-1-yl]-thieno[3,2-*d*]pyrimidin-4-yl}-propyl-amin (VII) werden in 15 ml Methanol vorgelegt und etwas abgekühlt. Erst werden 0,995 g (18,42 mmol) Natriummethoxid, dann 0,187 g (2,36 mmol) Cupfer(II)oxid und 0,040 g (0,27 mmol) Natriumiodid zugegeben. Das Reaktionsgemisch wird in der Mikrowelle 0,75 Stunden auf 160° C erhitzt. Anschließend wird über Kieselgel abgesaugt, die Mutterlauge eingedampft. Der Rückstand wird gelöst, Unlösliches abgesaugt. Weitere Reinigung erfolgt über präparative HPLC. Dabei werden 0,23 g des Produkts VIII (20%) erhalten.

**2-[4-(4-Chlor-phenyl)-piperazin-1-yl]-4-propylamino-thieno[3,2-*d*]pyrimidin-7-ol triflat (IX):** 0,430 g (1,03 mmol) {2-[4-(4-Chlor-phenyl)-piperazin-1-yl]-7-methoxy-thieno[3,2-*d*]pyrimidin-4-yl}-propyl-amin (VIII) werden in 4 ml Dichlormethan gelöst und etwas abgekühlt. In Stickstoffatmosphäre werden 1,13 ml (1,13 mmol) Bortribromidlösung (1 molar in Heptan) zugetropft. Das Reaktionsgemisch wird 48 Stunden bei Raumtemperatur gerührt. Nach Zugabe von 0,50 ml Bortribromidlösung wird noch 24 Stunden bei Raumtemperatur gerührt. Anschließend wird gesättigte Kaliumcarbonatlösung zugegeben und 0,4 Stunden gerührt. Nach Zugabe von Dichlormethan wird die organische Phase über Phasenseparator abgetrennt und zur Trockene eingedampft. Der Rückstand wird chromatographisch (HPLC) gereinigt, entsprechende Fraktionen lyophylisiert. 0,150 g des Produkts IX (28%) werden erhalten.

**2-[4-(4-Chlor-phenyl)-piperazin-1-yl]-4-propylamino-6,7-dihydro-thieno[3,2-*d*]pyrimidin-7-ol triflat (X):** 0,050 g (0,12 mmol) 2-[4-(4-Chlor-phenyl)-piperazin-1-yl]-4-propylamino-thieno[3,2-*d*]pyrimidin-7-ol triflat (IX) werden in 1 ml Methanol gelöst, 0,020 g (0,53 mmol) Natriumborhydrid zugegeben. Das Reaktionsgemisch wird 16 Stunden bei Raumtemperatur gerührt, anschließend eingedampft. Der Rückstand wird chromatographisch über RP-Säule mit einer HPLC gereinigt (Säule: Microsorb, RP-C18). Entsprechende Fraktionen werden vereinigt und lyophylisiert. 0,013 g des Produkts X (20%) werden als Pulver erhalten.
¹H NMR (400 MHz, DMSO): 7,26 (2H, d); 7,00 (2H, d); 5,15-5,09 (1H, m); 3,86-3,79 (4H, m), 3,63-3,55 (2H, m); 3,42-3,33 (2H, m); 3,28-3,22 (4H, m); 3,18-3,11 (1H, m); 1,63-1,52 (2H, m); 0,88 (3H, t).

Die folgenden Beispiele können analog den oben gezeigten Synthesevorschriften nach Schema 1 bis 14 herstellt werden (je nach Kennzeichnung). Die Verbindungen sind als PDE4-Inhibitoren geeignet und besitzen IC₅₀-Werte kleiner oder gleich 1 µmol.

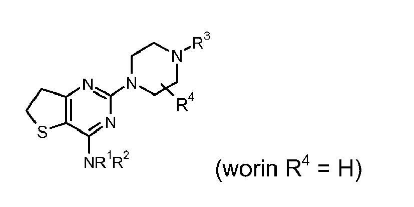

| **#** | **R¹** | **R²** | **R³** | **Herstellung** |
|---|---|---|---|---|
| 1. | H | 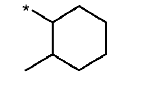 | 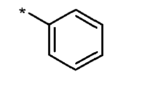 | Schema 1 |
| 2. | H | 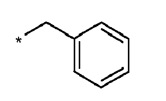 | 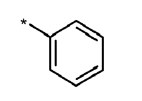 | Schema 1 |
| 3. | H | 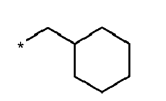 | 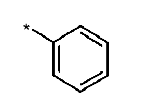 | Schema 1 |
| 4. | H | 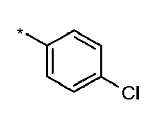 | 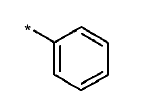 | Schema 1 |
| 5. | H | 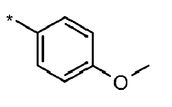 | 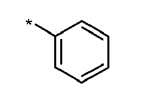 | Schema 1 |
| 6. | H | 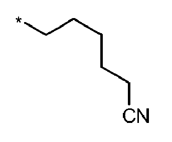 | 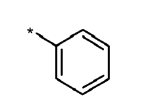 | Schema 1 |
| 7. | H | 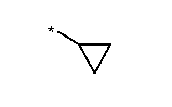 | 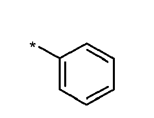 | Schema 1 |
| 8. | H | 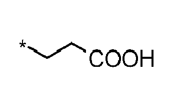 | 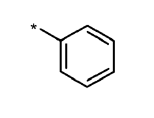 | Schema 1 |
| 9. | H | 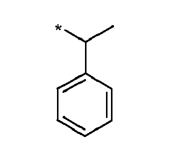 | 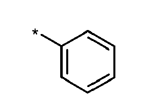 | Schema 1 |
| 10. | H | 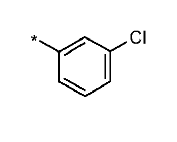 | 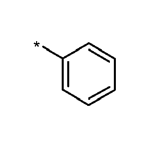 | Schema 1 |
| 11. | H | 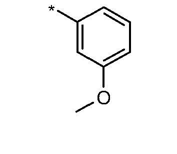 | 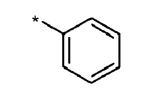 | Schema 1 |
| 12. | H | 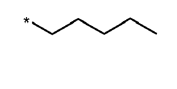 | 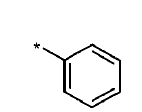 | Schema 1 |
| 13. | H | 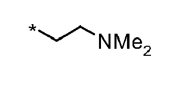 | 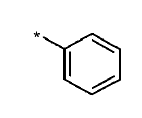 | Schema 1 |
| 14. | H | 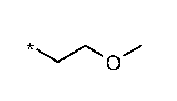 | 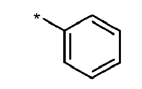 | Schema 1 |
| 15. | H | 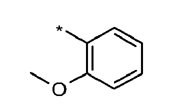 | 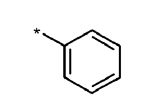 | Schema 1 |
| 16. | H | 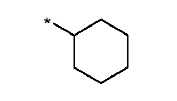 | 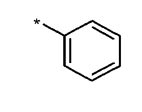 | Schema 1 |
| 17. | H | 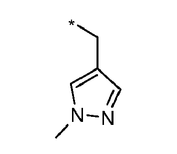 | 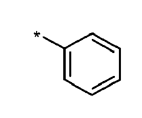 | Schema 1 |
| 18. | H | 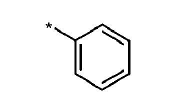 | 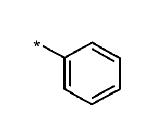 | Schema 1 |
| 19. | H | 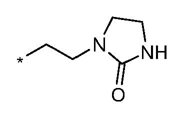 | 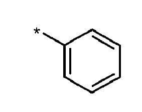 | Schema 1 |
| 20. | H | 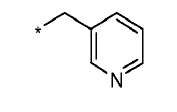 | 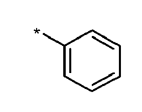 | Schema 1 |
| 21. | H | 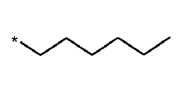 | 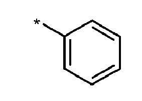 | Schema 1 |
| 22. | H | 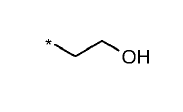 | 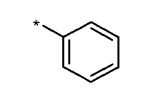 | Schema 1 |
| 23. | H | 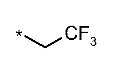 | 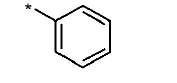 | Schema 1 |
| 24. | H | 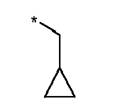 | 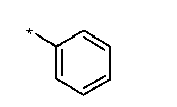 | Schema 1 |
| 25. | H | 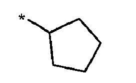 | 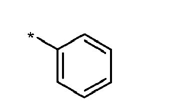 | Schema 1 |
| 26. | H | 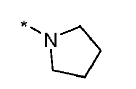 | 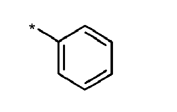 | Schema 1 |
| 27. | H | 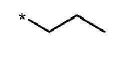 | 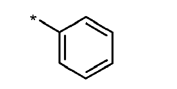 | Schema 1 |
| 28. | H | 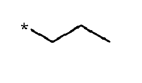 | 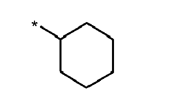 | Schema1 |
| 29. | H | 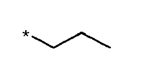 | 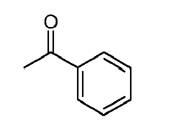 | Schema 1 |
| 30. | H | 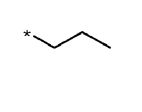 | 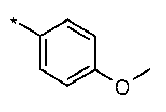 | Schema 1 |
| 31. | H | 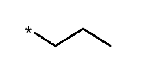 | 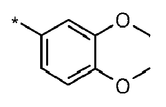 | Schema 1 |
| 32. | H | 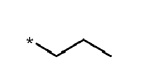 | 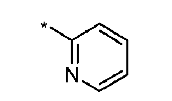 | Schema 1 |
| 33. | H | 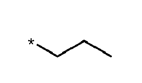 | 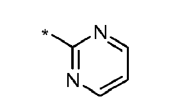 | Schema 1 |
| 34. | H | 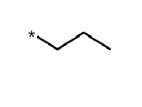 | 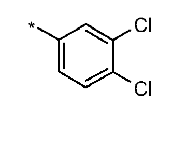 | Schema 1 |
| 35. | H | 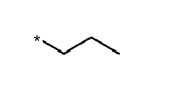 | 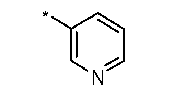 | Schema 1 |
| 36. | H | 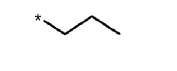 | 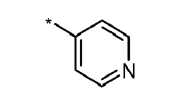 | Schema 1 |
| 37. | H | 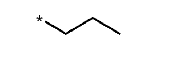 | 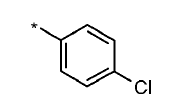 | Schema 1 |
| 38. | H | 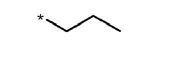 | 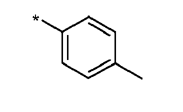 | Schema 1 |
| 39. | H | 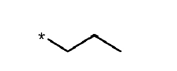 | 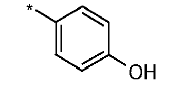 | Schema 1 |
| 40. | H | 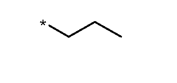 | 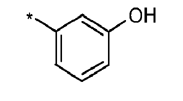 | Schema 1 |
| 41. | H | 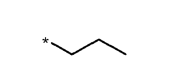 | 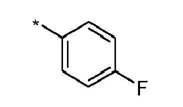 | Schema 1 |
| 42. | H | 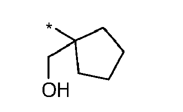 | 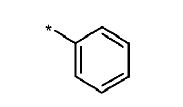 | Schema 1 |
| 43. | H | 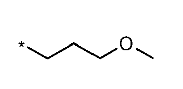 | 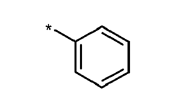 | Schema 1 |
| 44. | H | 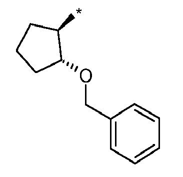 | 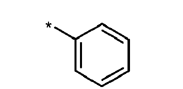 | Schema 1 |
| 45. | H | 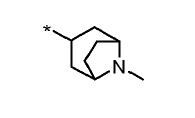 | 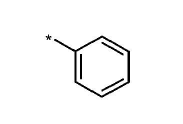 | Schema 1 |
| 46. | H | 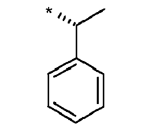 | 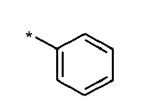 | Schema 1 |
| 47. | H | 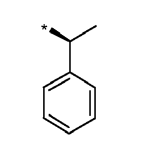 | 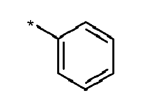 | Schema 1 |
| 48. | H | 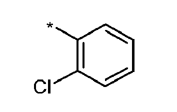 | 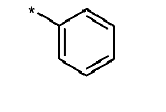 | Schema 1 |
| 49. | H | 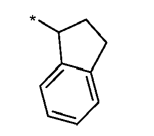 | 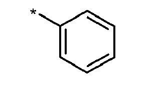 | Schema 1 |
| 50. | H | 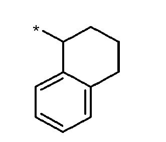 | 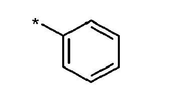 | Schema 1 |
| 51. | H | 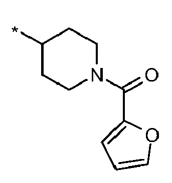 | 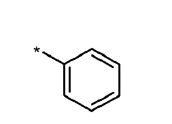 | Schema 1 |
| 52. | H | 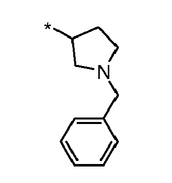 | 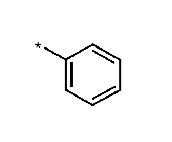 | Schema 1 |
| 53. | H | 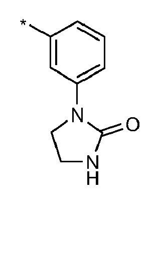 | 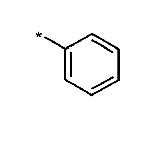 | Schema 1 |
| 54. | H | 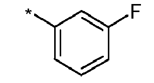 | 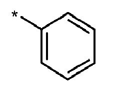 | Schema 1 |
| 55. | H | 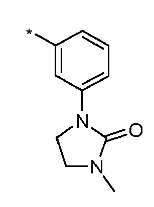 | 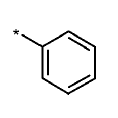 | Schema 1 |
| 56. | H | 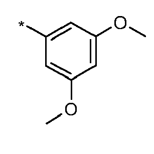 | 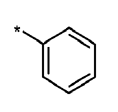 | Schema 1 |
| 57. | H | 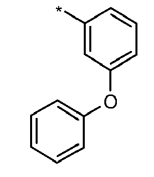 | 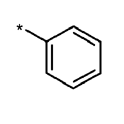 | Schema 1 |
| 58. | H | 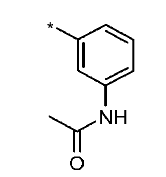 | 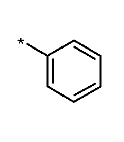 | Schema 1 |
| 59. | H | 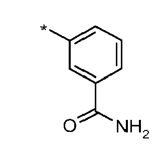 | 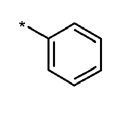 | Schema 1 |
| 60. | H | 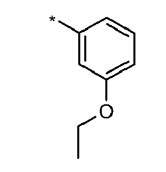 | 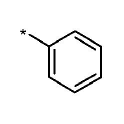 | Schema 1 |
| 61. | H | 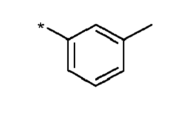 | 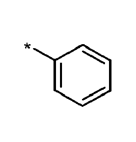 | Schema 1 |
| 62. | H | 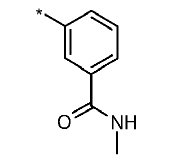 | 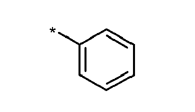 | Schema 1 |
| 63. | H | 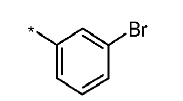 | 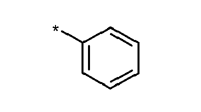 | Schema 1 |
| 64. | H | 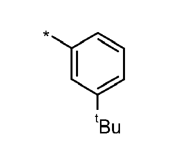 | 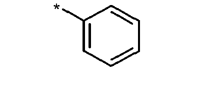 | Schema 1 |
| 65. | H | 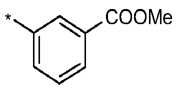 | 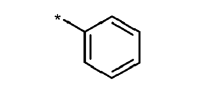 | Schema 1 |
| 66. | H | 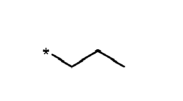 | 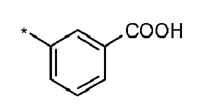 | Schema 1 |
| 67. | H | 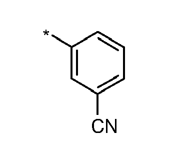 | 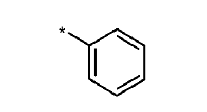 | Schema 1 |
| 68. | H | 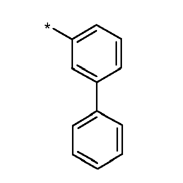 | 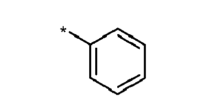 | Schema 1 |
| 69. | H | 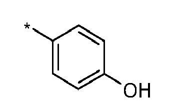 | 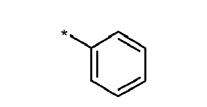 | Schema 1 |
| 70. | H | 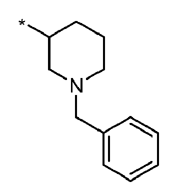 | 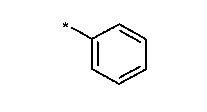 | Schema 1 |
| 71. | H | 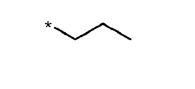 | 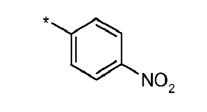 | Schema 1 |
| 72. | H | 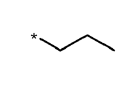 | 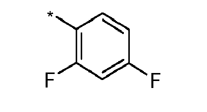 | Schema 1 |
| 73. | H | 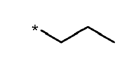 | 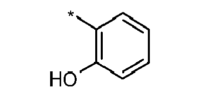 | Schema 1 |
| 74. | H | 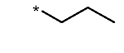 | 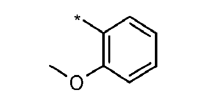 | Schema 1 |
| 75. | H | 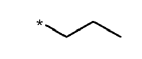 | 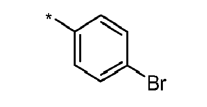 | Schema 1 |
| 76. | H | 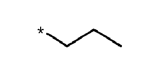 | 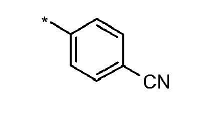 | Schema 1 |
| 77. | H | 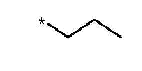 | 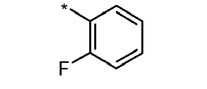 | Schema 1 |
| 78. | H | 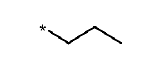 | 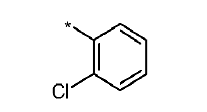 | Schema 1 |
| 79. | H | 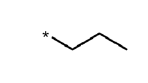 | 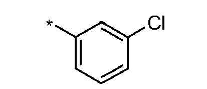 | Schema 1 |
| 80. | H | 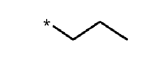 | 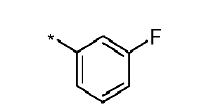 | Schema 1 |
| 81. | H | 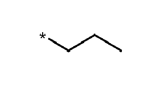 | 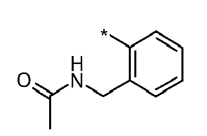 | Schema 1 |
| 82. | H | 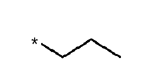 | 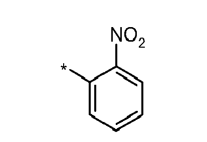 | Schema 1 |
| 83. | H | 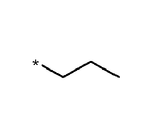 | 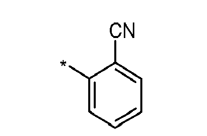 | Schema 1 |
| 84. | H | 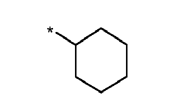 | 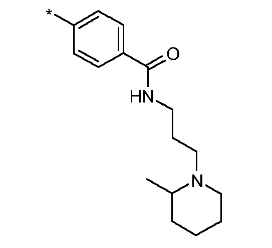 | Schema 2 |
| 85. | H | 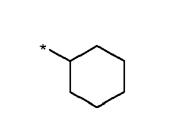 | 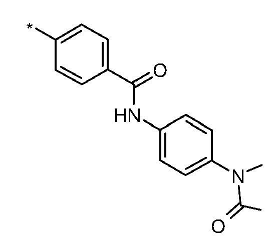 | Schema 2 |
| 86. | H | 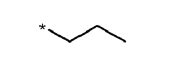 | 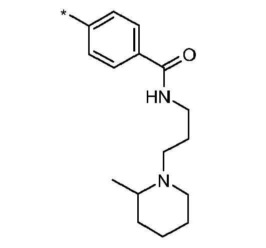 | Schema 2 |
| 87. | H | 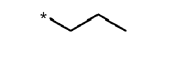 | 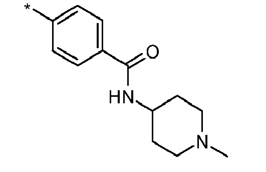 | Schema 2 |
| 88. | H | 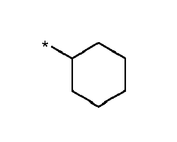 | 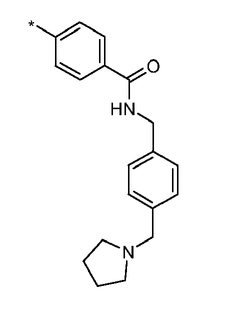 | Schema 2 |
| 89. | H | 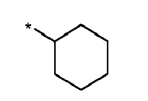 | 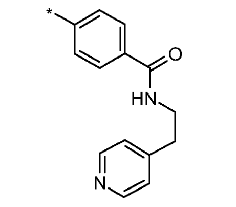 | Schema 2 |
| 90. | H | 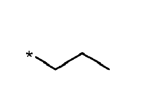 | 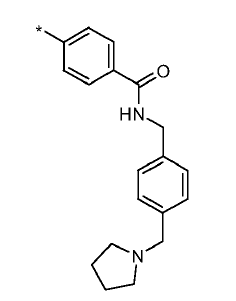 | Schema 2 |
| 91. | H | 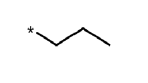 | 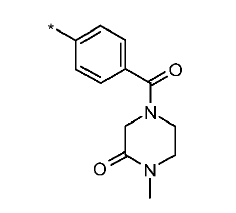 | Schema 2 |
| 92. | H | 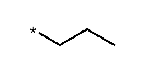 | 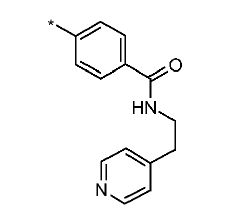 | Schema 2 |
| 93. | H | 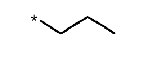 | 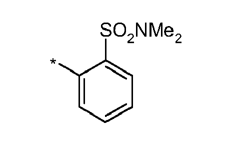 | Schema 1 |
| 94. | H | 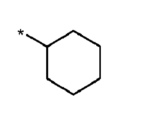 | 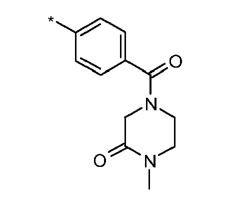 | Schema 2 |
| 95. | H | 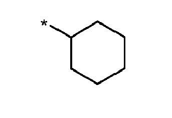 | 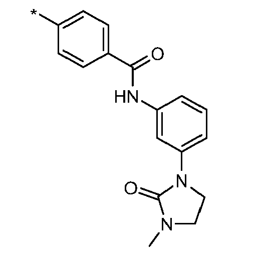 | Schema 2 |
| 96. | H | 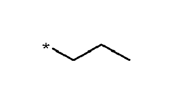 | 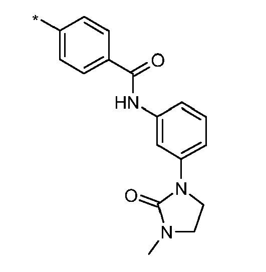 | Schema 2 |
| 97. | H | 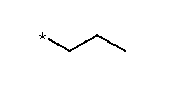 | 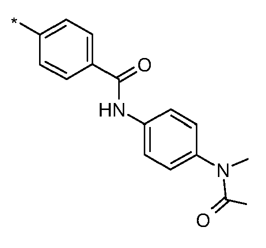 | Schema 2 |
| 98. | H | 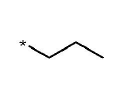 | 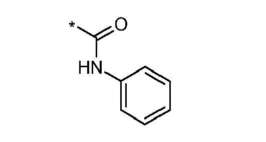 | Schema 5 |
| 99. | H | 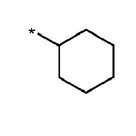 | 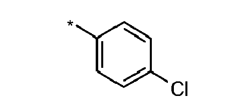 | Schema 1 |
| 100. | H | 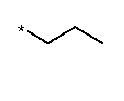 | 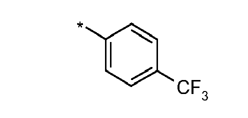 | Schema 1 |
| 101. | H | 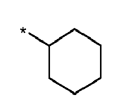 | 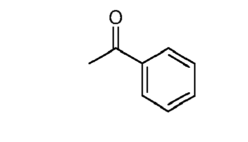 | Schema 5 |
| 102. | H | 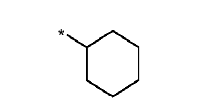 | 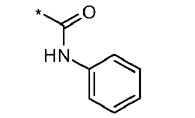 | Schema 5 |
| 103. | H | 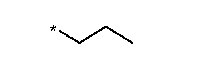 | 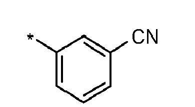 | Schema 1 |
| 104. | H | 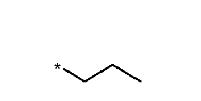 | 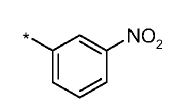 | Schema 1 |
| 105. | H | 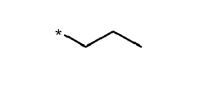 | 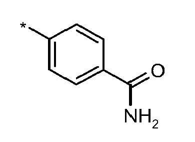 | Schema 1 |
| 106. | H | 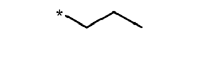 | 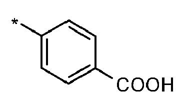 | Schema 1 |
| 107. | H | 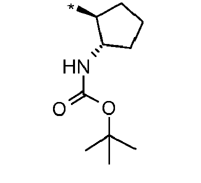 | 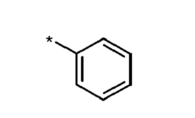 | Schema 7 |
| 108. | H | 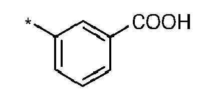 | 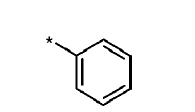 | Schema 1 |
| 109. | H | 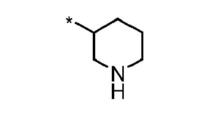 | 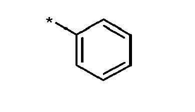 | Schema 11 |
| 110. | H | 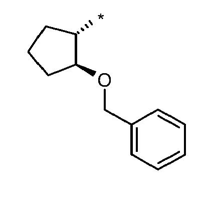 | 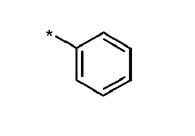 | Schema 1 |
| 111. | H | 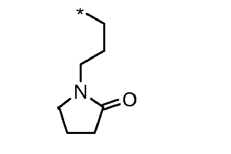 | 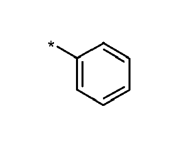 | Schema 1 |
| 112. | H | 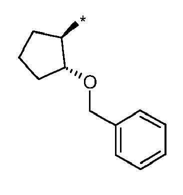 | 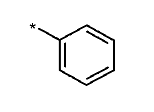 | Schema 1 |
| 113. | H | 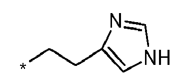 | 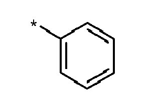 | Schema 1 |
| 114. | H | 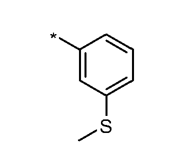 | 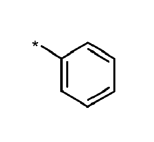 | Schema 1 |
| 115. | H | 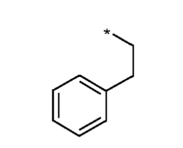 | 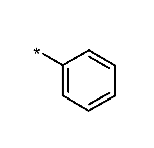 | Schema 1 |
| 116. | H | 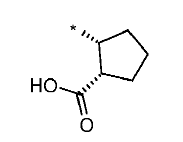 | 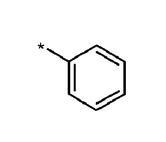 | Schema 1 |
| 117. | H | 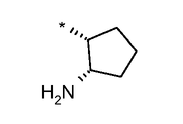 | 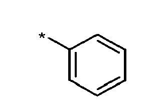 | Schema 1 |
| 118. | H | 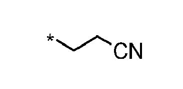 | 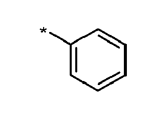 | Schema 1 |
| 119. | H | 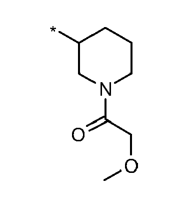 | 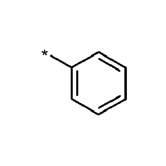 | Schema 10 |
| 120. | H | 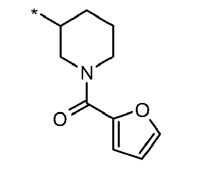 | 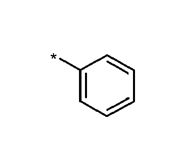 | Schema 10 |
| 121. | H | 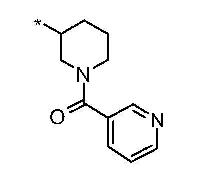 | 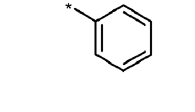 | Schema 10 |
| 122. | H | 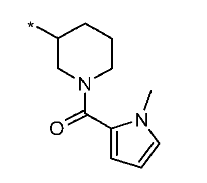 | 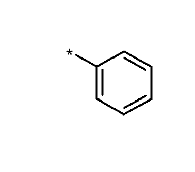 | Schema 10 |
| 123. | H | 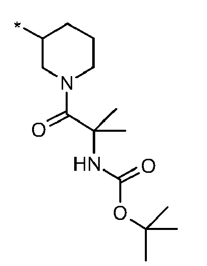 | 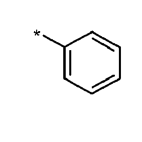 | Schema 10 |
| 124. | H | 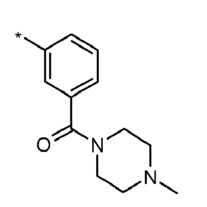 | 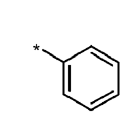 | Schema 12 |
| 125. | H | 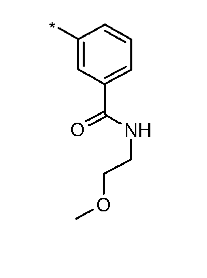 | 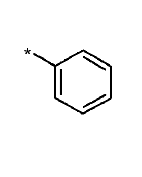 | Schema 12 |
| 126. | H | 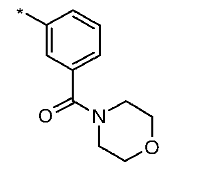 | 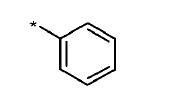 | Schema 12 |
| 127. | H | 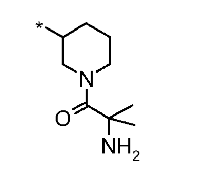 | 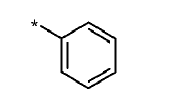 | Schema 10 |
| 128. | H | 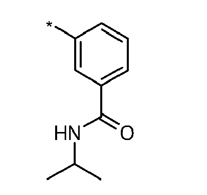 | 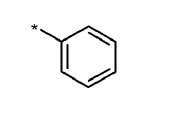 | Schema 12 |
| 129. | H | 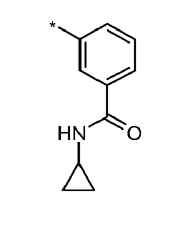 | 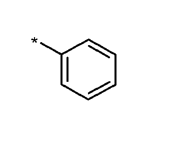 | Schema 12 |
| 130. | H | 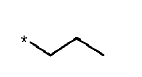 | 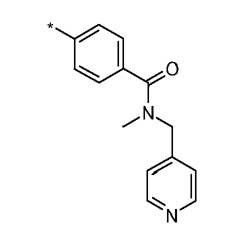 | Schema 2 |
| 131. | H | 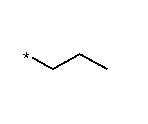 | 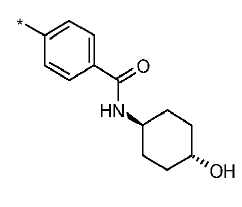 | Schema 2 |
| 132. | H | 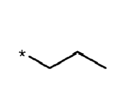 | 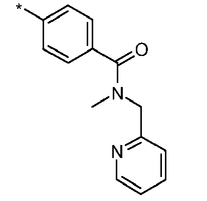 | Schema 2 |
| 133. | H | 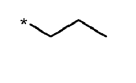 | 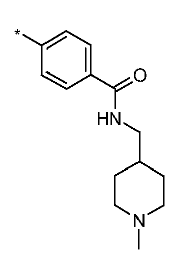 | Schema 2 |
| 134. | H | 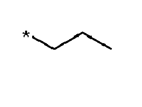 | 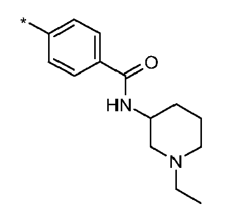 | Schema 2 |
| 135. | H | 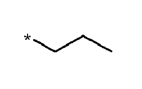 | 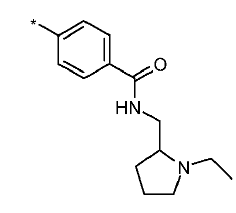 | Schema 2 |
| 136. | H | 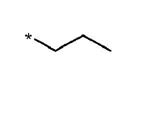 | 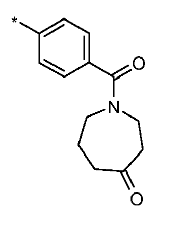 | Schema 2 |
| 137. | H | 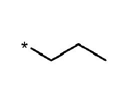 | 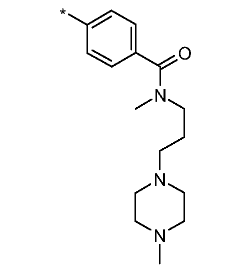 | Schema 2 |
| 138. | H | 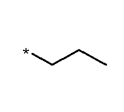 | 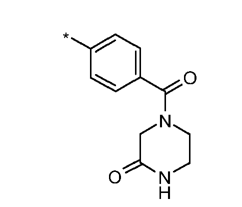 | Schema 2 |
| 139. | H | 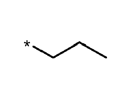 | 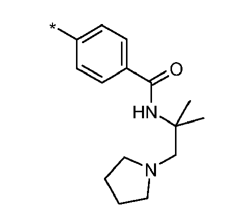 | Schema 2 |
| 140. | H | 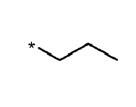 | 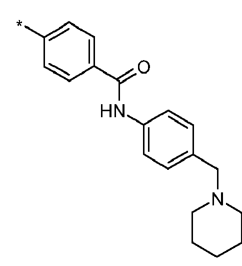 | Schema 2 |
| 141. | H | 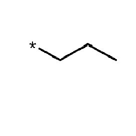 | 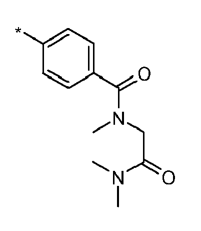 | Schema 2 |
| 142. | H | 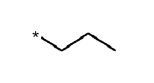 | 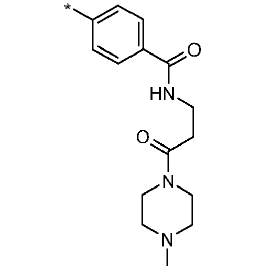 | Schema 2 |
| 143. | H | 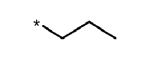 | 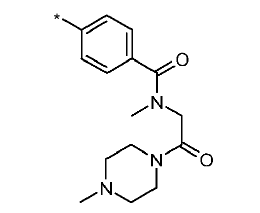 | Schema 2 |
| 144. | H | 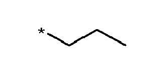 | 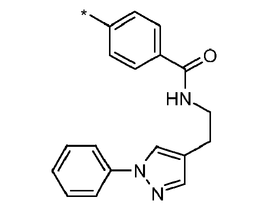 | Schema 2 |
| 145. | H | 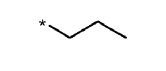 | 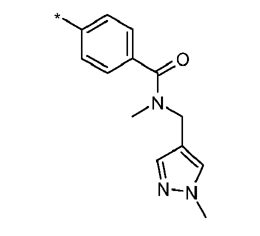 | Schema 2 |
| 146. | H | 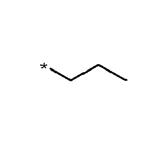 | 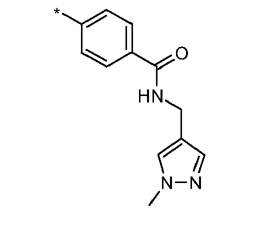 | Schema 2 |
| 147. | H | 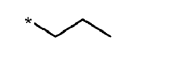 | 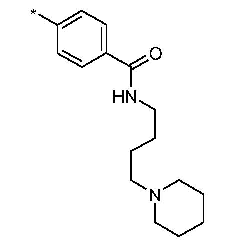 | Schema 2 |
| 148. | H | 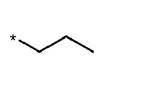 | 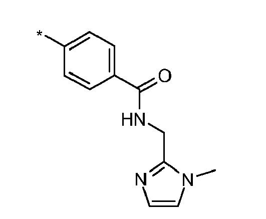 | Schema 2 |
| 149. | H | 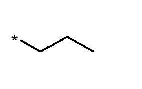 | 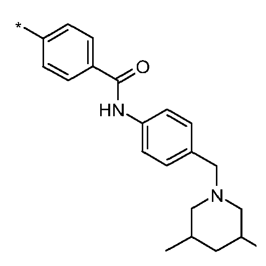 | Schema 2 |
| 150. | H | 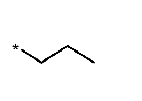 | 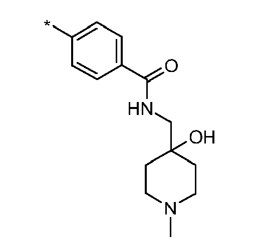 | Schema 2 |
| 151. | H | 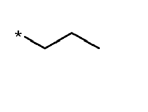 | 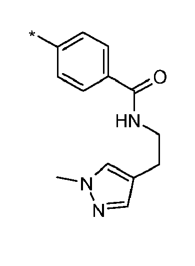 | Schema 2 |
| 152. | H | 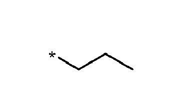 | 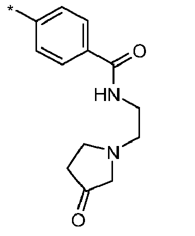 | Schema 2 |
| 153. | H | 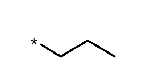 | 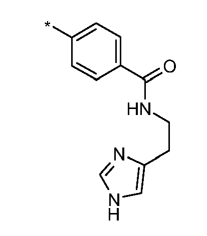 | Schema 2 |
| 154. | H | 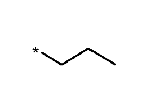 | 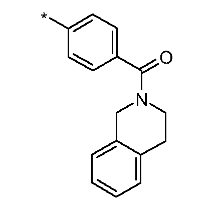 | Schema 2 |
| 155. | H | 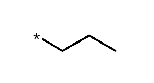 | 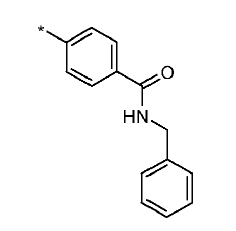 | Schema 2 |
| 156. | H | 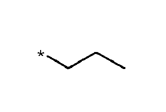 | 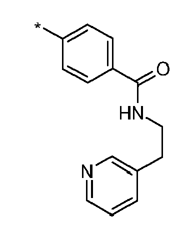 | Schema 2 |
| 157. | H | 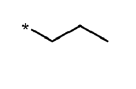 | 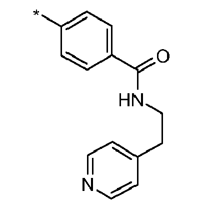 | Schema 2 |
| 158. | H | 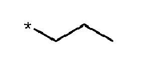 | 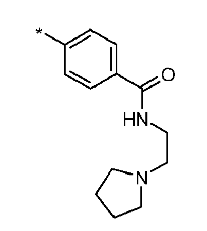 | Schema 2 |
| 159. | H | 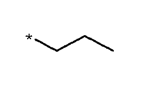 | 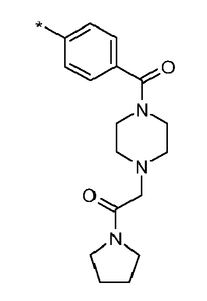 | Schema 2 |
| 160. | H | 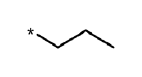 | 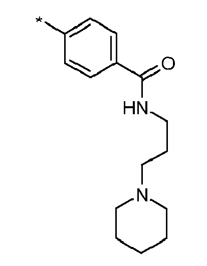 | Schema 2 |
| 161. | H | 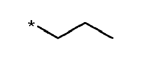 | 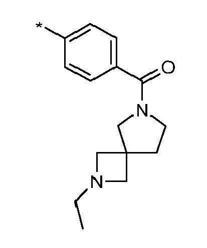 | Schema 2 |
| 162. | H | 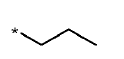 | 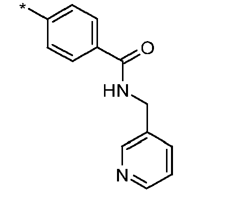 | Schema 2 |
| 163. | H | 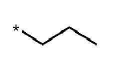 | 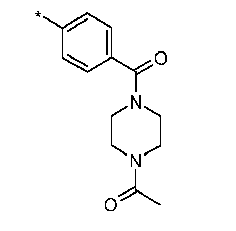 | Schema 2 |
| 164. | H | 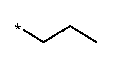 | 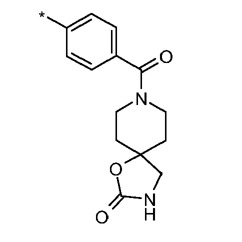 | Schema 2 |
| 165. | H | 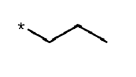 | 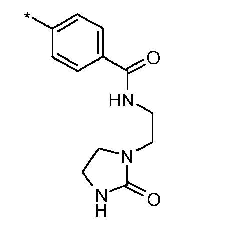 | Schema 2 |
| 166. | H | 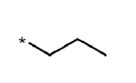 | 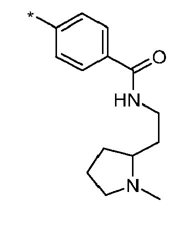 | Schema 2 |
| 167. | H | 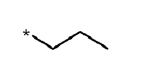 | 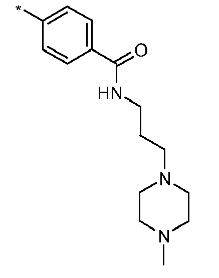 | Schema 2 |
| 168. | H | 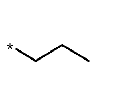 | 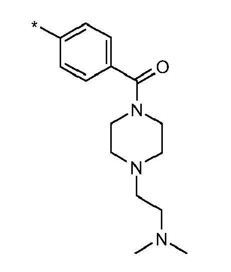 | Schema 2 |
| 169. | H | 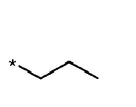 | 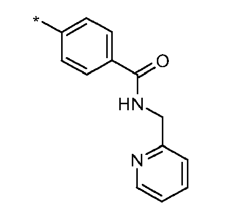 | Schema 2 |
| 170. | H | 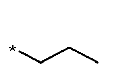 | 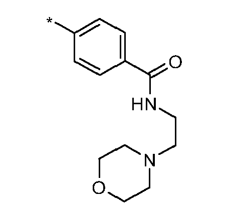 | Schema 2 |
| 171. | H | 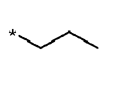 | 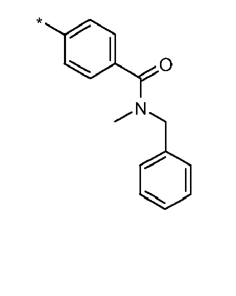 | Schema 2 |
| 172. | H | 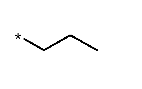 | 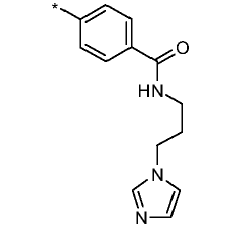 | Schema 2 |
| 173. | H | 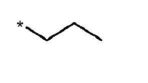 | 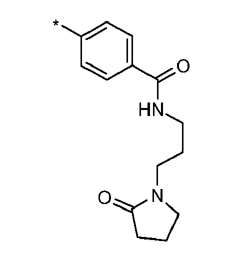 | Schema 2 |
| 174. | H | 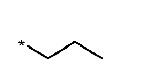 | 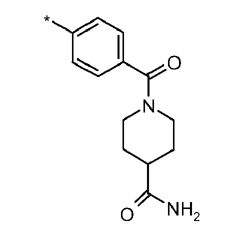 | Schema 2 |
| 175. | H | 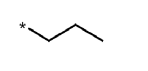 | 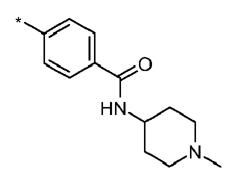 | Schema 2 |
| 176. | H | 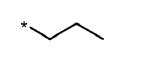 | 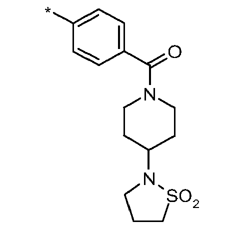 | Schema 2 |
| 177. | H | 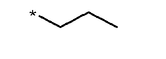 | 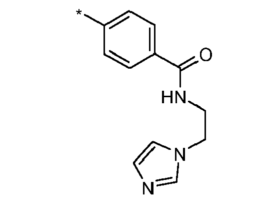 | Schema 2 |
| 178. | H | 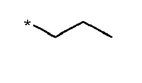 | 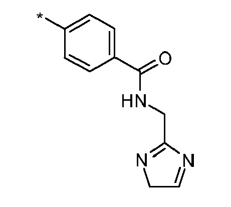 | Schema 2 |
| 179. | H | 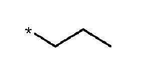 | 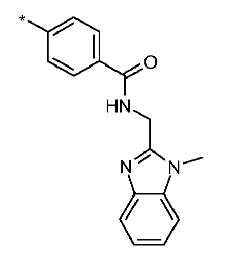 | Schema 2 |
| 180. | H | 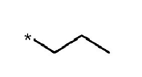 | 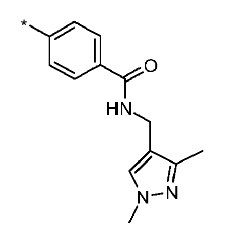 | Schema 2 |
| 181. | H | 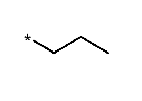 | 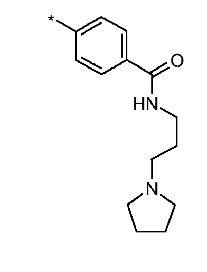 | Schema 2 |
| 182. | H | 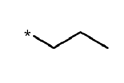 | 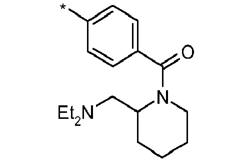 | Schema 2 |
| 183. | H | 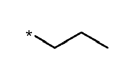 | 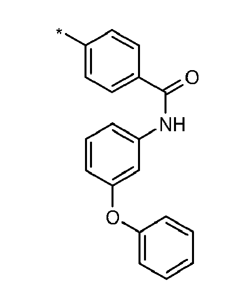 | Schema 2 |
| 184. | H | 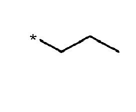 | 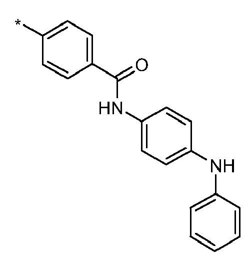 | Schema 2 |
| 185. | H | 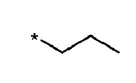 | 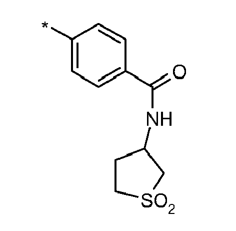 | Schema 2 |
| 186. | H | 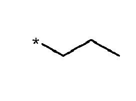 | 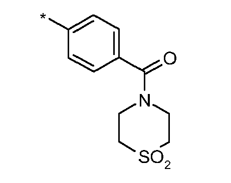 | Schema 2 |
| 187. | H | 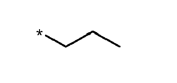 | 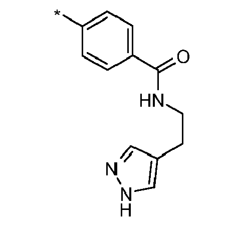 | Schema 2 |
| 188. | H | 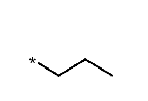 | 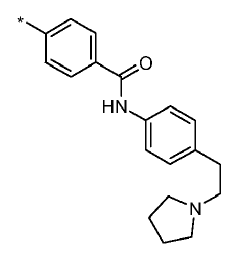 | Schema 2 |
| 189. | H | 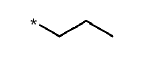 | 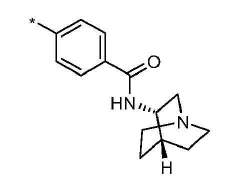 | Schema 2 |
| 190. | H | 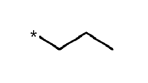 | 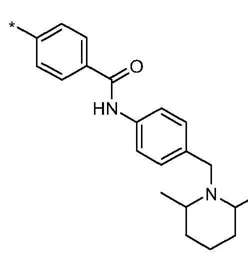 | Schema 2 |
| 191. | H | 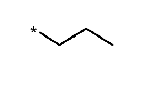 | 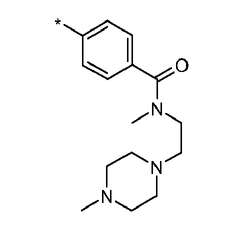 | Schema 2 |
| 192. | H | 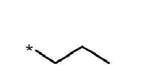 | 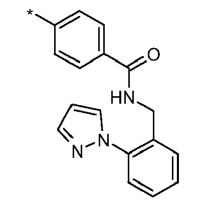 | Schema 2 |
| 193. | H | 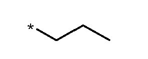 | 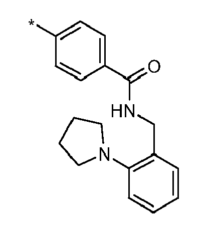 | Schema 2 |
| 194. | H | 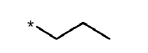 | 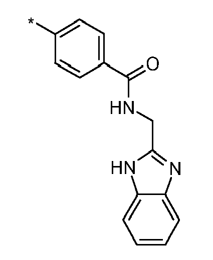 | Schema 2 |
| 195. | H | 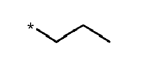 | 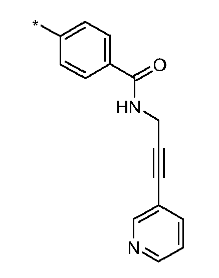 | Schema 2 |
| 196. | H | 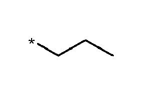 | 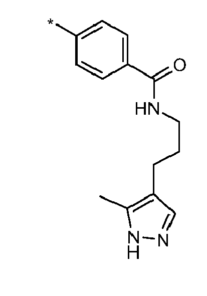 | Schema 2 |
| 197. | H | 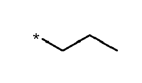 | 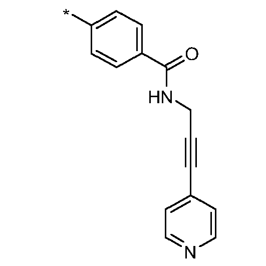 | Schema 2 |
| 198. | H | 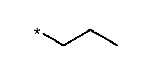 | 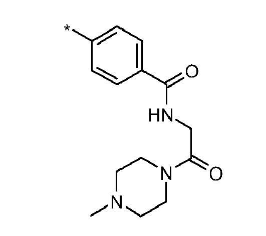 | Schema 2 |
| 199. | H | 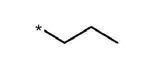 | 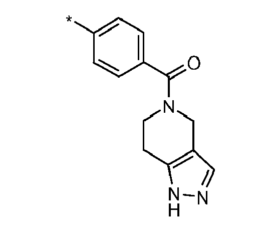 | Schema 2 |
| 200. | H | 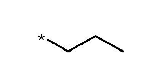 | 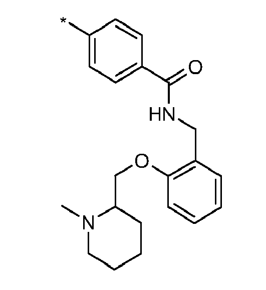 | Schema 2 |
| 201. | H | 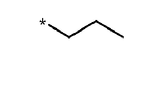 | 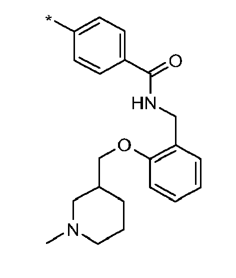 | Schema 2 |
| 202. | H | 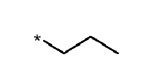 | 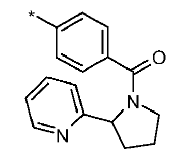 | Schema 2 |
| 203. | H | 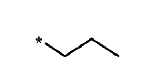 | 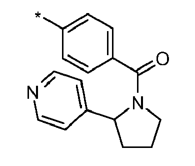 | Schema 2 |
| 204. | H | 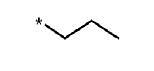 | 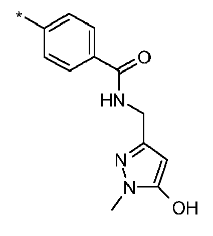 | Schema 2 |
| 205. | H | 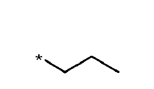 | 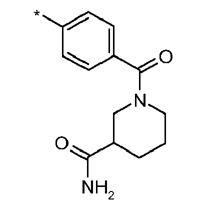 | Schema 2 |
| 206. | H | 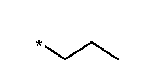 | 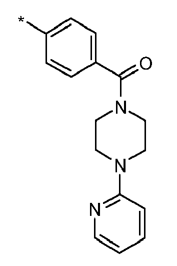 | Schema 2 |
| 207. | H | 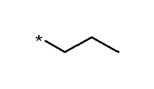 | 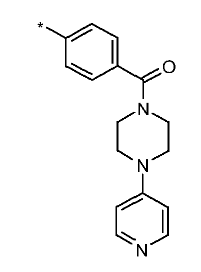 | Schema 2 |
| 208. | H | 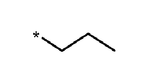 | 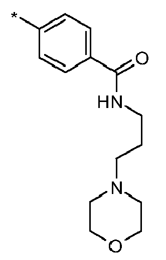 | Schema 2 |
| 209. | H | 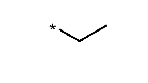 | 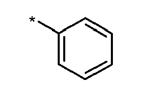 | Schema 1 |
| 210. | H | 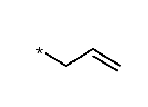 | 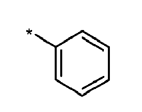 | Schema 1 |
| 211. | H | 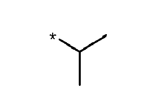 | 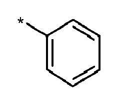 | Schema 1 |
| 212. | H | 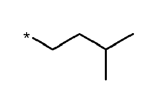 | 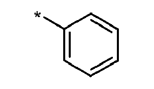 | Schema 1 |
| 213. | H | 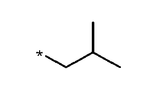 | 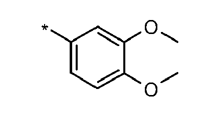 | Schema 1 |
| 214. | H | 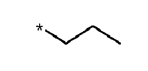 | 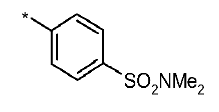 | Schema 1 |
| 215. | H | 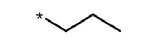 | 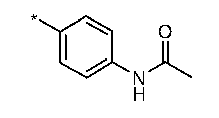 | Schema 4 |
| 216. | H | 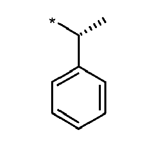 | 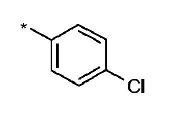 | Schema 1 |
| 217. | H | 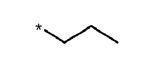 | 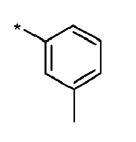 | Schema 1 |
| 218. | H | H | 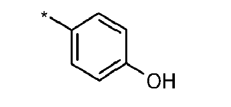 | Schema 1 |
| 219. | H | H | 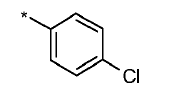 | Schema 1 |
| 220. | H | 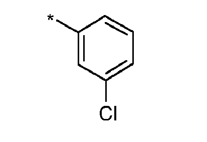 | 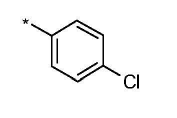 | Schema 1 |
| 221. | H | 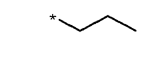 | 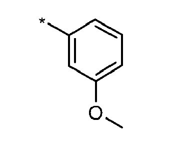 | Schema 1 |
| 222. | H | 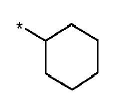 | 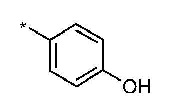 | Schema 1 |
| 223. | H | 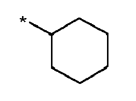 | 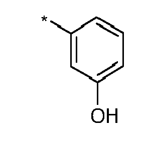 | Schema 1 |
| 224. | H | 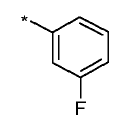 | 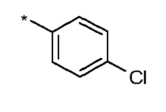 | Schema 1 |
| 225. | H | 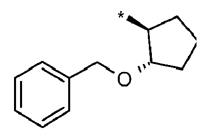 | 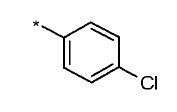 | Schema 1 |
| 226. | H | 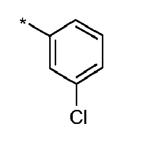 | 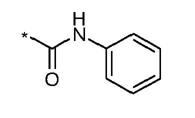 | Schema 5 |
| 227. | H | 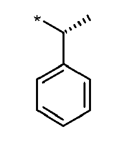 | 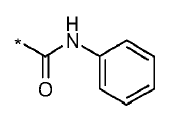 | Schema 5 |
| 228. | H | 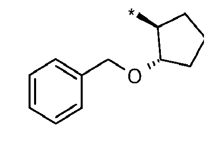 | 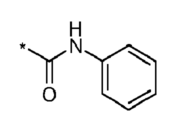 | Schema 5 |
| 229. | H | 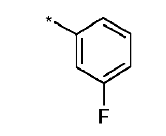 | 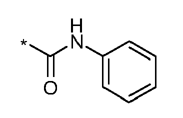 | Schema 5 |
| 230. | H | 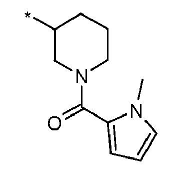 | 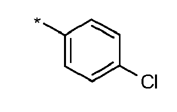 | Schema 10 |
| 231. | H | 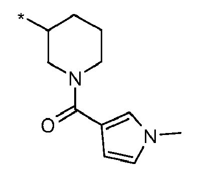 | 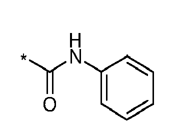 | Schema 5 |
| 232. | H | 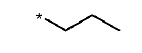 | 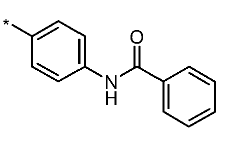 | Schema 5 |
| 233. | H | 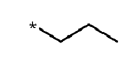 | 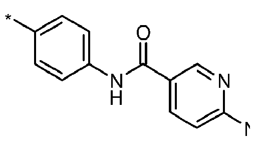 | Schema 5 |
| 234. | H | 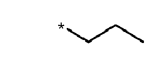 | 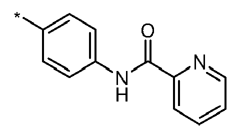 | Schema 5 |
| 235. | H | 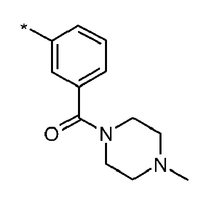 | 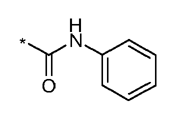 | Schema 5 |
| 236. | H | 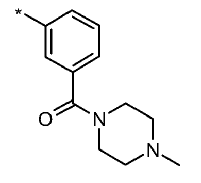 | 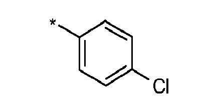 | Schema 12 |
| 237. | H | 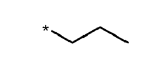 | 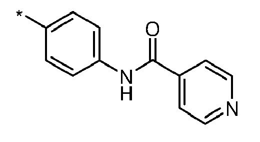 | Schema 4 |
| 238. | H | 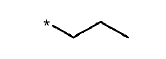 | 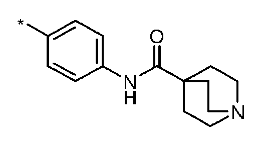 | Schema 4 |
| 239. | H | 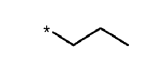 | 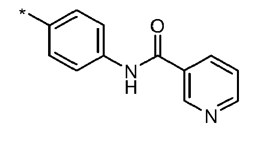 | Schema 4 |
| 240. | H | 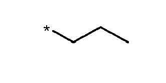 | 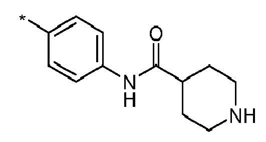 | Schema 4 |
| 241. | H | 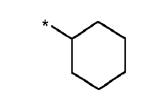 | 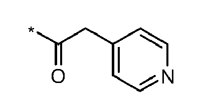 | Schema 5 |
| 242. | H | 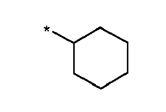 | 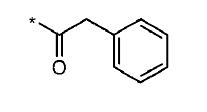 | Schema 5 |
| 243. | H | 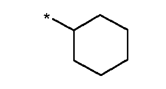 | 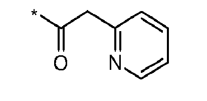 | Schema 5 |
| 244. | H | 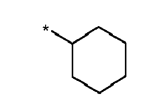 | 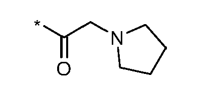 | Schema 5 |
| 245. | H | 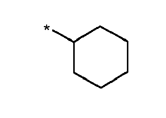 | 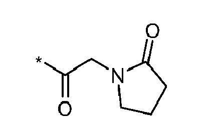 | Schema 5 |
| 246. | H | 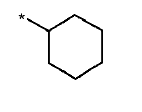 | 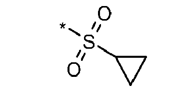 | Schema 5 |
| 247. | H | 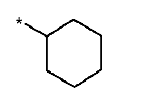 | 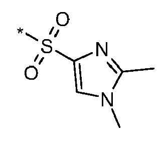 | Schema 5 |
| 248. | H | 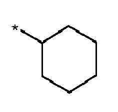 | 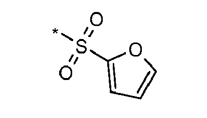 | Schema 5 |
| 249. | H | 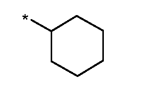 | 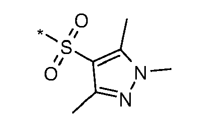 | Schema 5 |
| 250. | H | 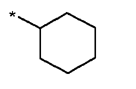 | 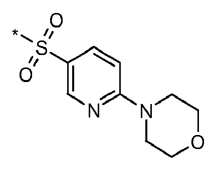 | Schema 5 |
| 251. | H | 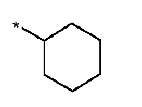 | 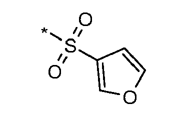 | Schema 5 |
| 252. | H | 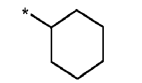 | 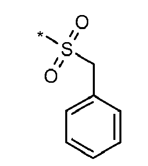 | Schema 5 |
| 253. | H | 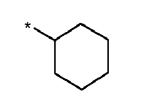 | 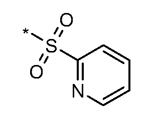 | Schema 5 |
| 254. | H | 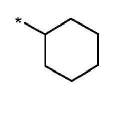 | 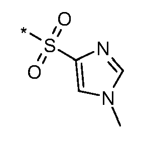 | Schema 5 |
| 255. | H | 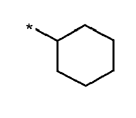 | 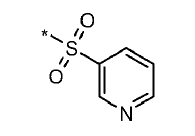 | Schema 5 |
| 256. | H | 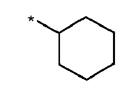 | 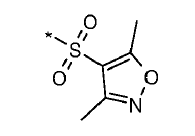 | Schema 5 |
| 257. | H | 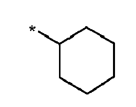 | 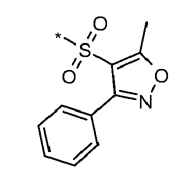 | Schema 5 |
| 258. | H | 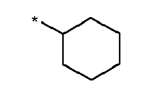 | 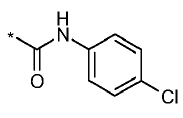 | Schema 5 |
| 259. | H | 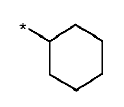 | 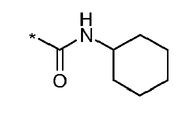 | Schema 5 |
| 260. | H | 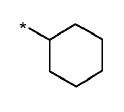 | 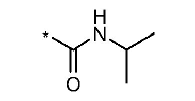 | Schema 5 |
| 261. | H | 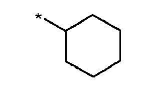 | 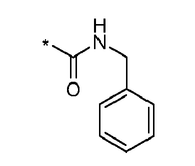 | Schema 5 |
| 262. | H | 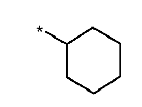 | 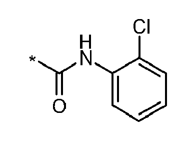 | Schema 5 |
| 263. | H | 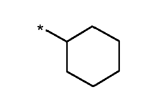 | 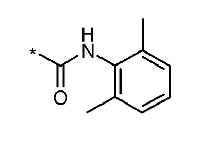 | Schema 5 |
| 264. | H | 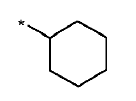 | 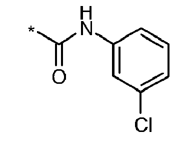 | Schema 5 |
| 265. | H | 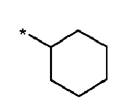 | 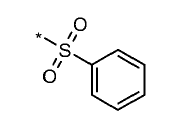 | Schema 5 |
| 266. | H | 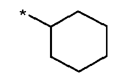 | 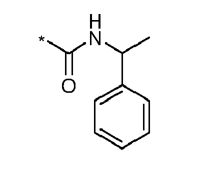 | Schema 5 |
| 267. | H | 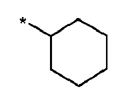 | 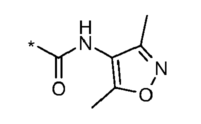 | Schema 5 |
| 268. | H | 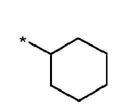 | 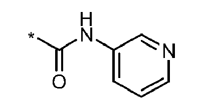 | Schema 5 |
| 269. | H | 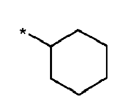 | 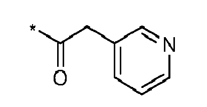 | Schema 5 |
| 270. | H | 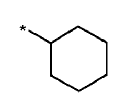 | 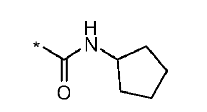 | Schema 5 |
| 271. | H | 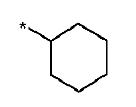 | 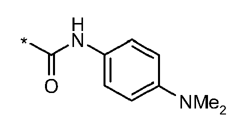 | Schema 5 |
| 272. | H | 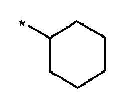 | 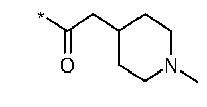 | Schema 5 |
| 273. | H | 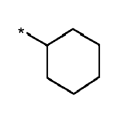 | 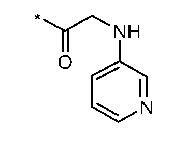 | Schema 5 |
| 274. | H | 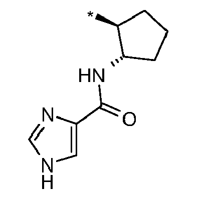 | 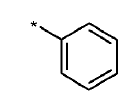 | Schema 7 |
| 275. | H | 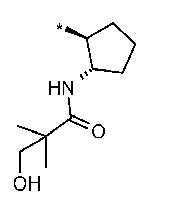 | 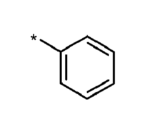 | Schema 7 |
| 276. | H | 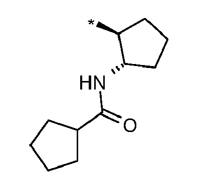 | 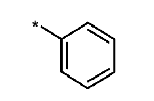 | Schema 7 |
| 277. | H | 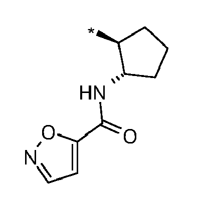 | 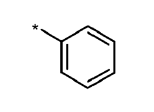 | Schema 7 |
| 278. | H | 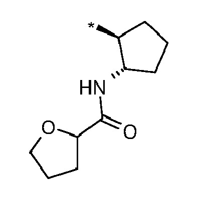 | 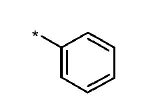 | Schema 7 |
| 279. | H | 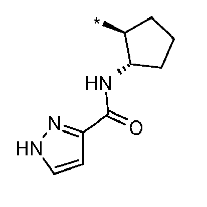 | 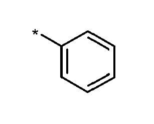 | Schema 7 |
| 280. | H | 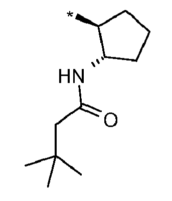 | 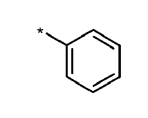 | Schema 7 |
| 281. | H | 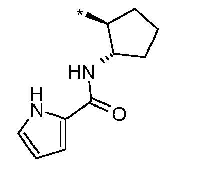 | 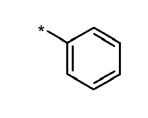 | Schema 7 |
| 282. | H | 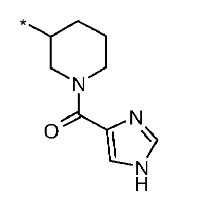 | 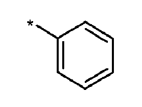 | Schema 10 |
| 283. | H | 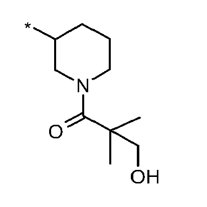 | 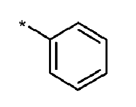 | Schema 10 |
| 284. | H | 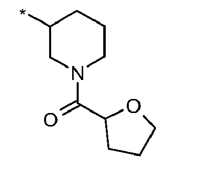 | 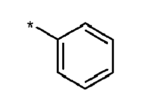 | Schema 10 |
| 285. | H | 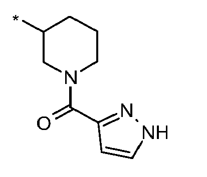 | 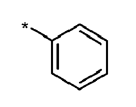 | Schema 10 |
| 286. | H | 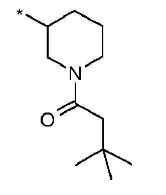 | 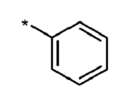 | Schema 10 |
| 287. | H | 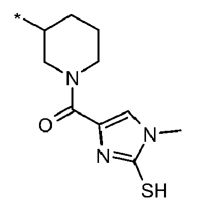 | 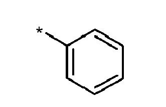 | Schema 10 |
| 288. | H | 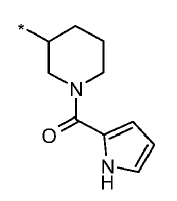 | 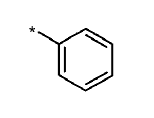 | Schema 10 |
| 289. | H | 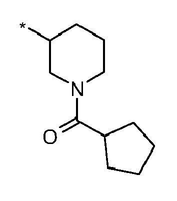 | 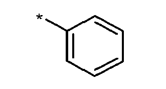 | Schema 10 |
| 290. | H | 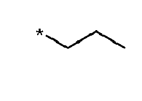 | 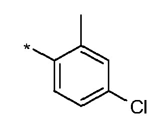 | Schema 1 |
| 291. | H | 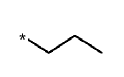 | 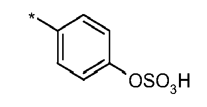 | Schema 1 |
| 292. | H | 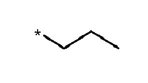 | 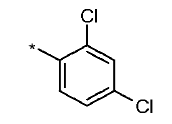 | Schema 1 |
| 293. | H | 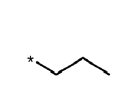 | 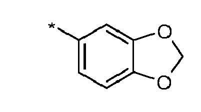 | Schema 1 |
| 294. | H | 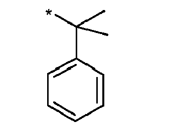 | 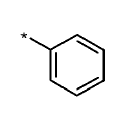 | Schema 1 |
| 295. | H | 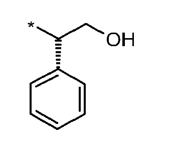 | 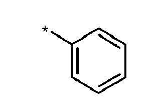 | Schema 1 |
| 296. | H | 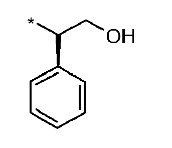 | 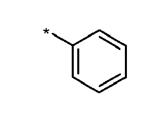 | Schema 1 |

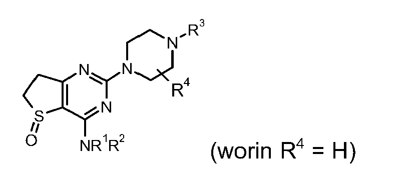

| **#** | **R¹** | **R²** | **R³** | **Herstellung** |
|---|---|---|---|---|
| 297 | H | 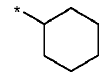 | 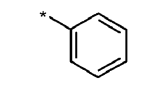 | Schema 8 |
| 298 | H | 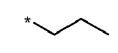 | 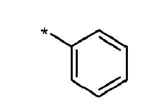 | Schema 8 |
| 299 | H | 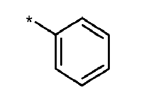 | 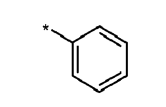 | Schema 8 |
| 300 | H | 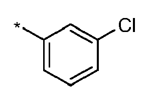 | 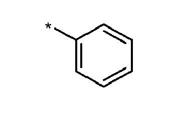 | Schema 8 |
| 301 | H | 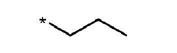 | 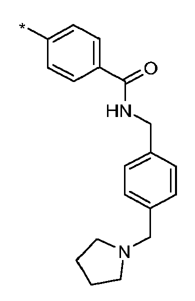 | Schema 8 |
| 302 | H | 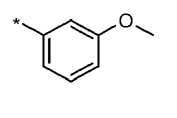 | 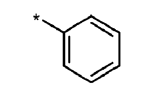 | Schema 8 |

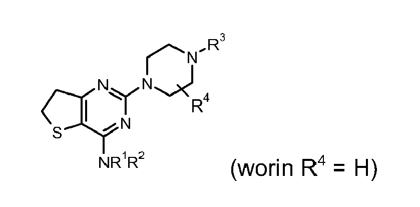

| **#** | **NR¹R²** | **R³** | **Herstellung** |
|---|---|---|---|
| 303. | 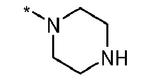 | 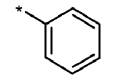 | **Schema 1** |

Weitere Beispiele wären:

| **#** | **Struktur** | **Herstellung** |
|---|---|---|
| 304. | 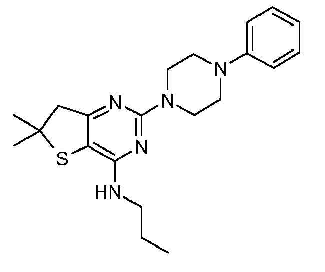 | Schema 1 |
| 305. | 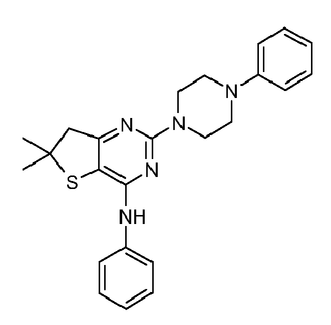 | |
| 306. | 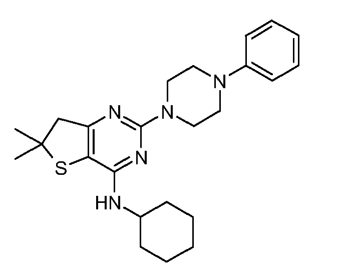 | Schema 1 |
| 307. | 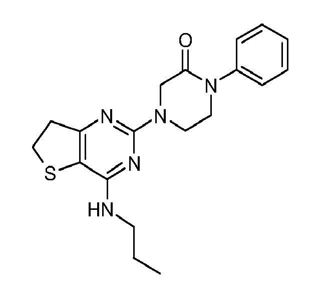 | Schema 1 |
| 308. | 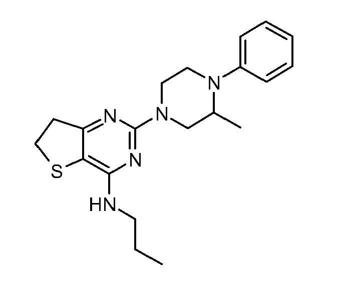 | Schema 1 |
| 309. | 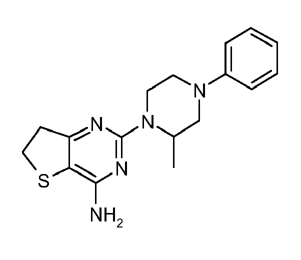 | Schema 1 |

### Beispiele A1

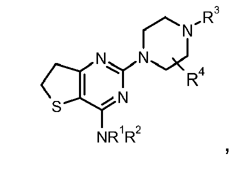

wobei R⁴ = H ist

| **#** | **R¹** | **R²** | **R³** | **Herstellung** |
|---|---|---|---|---|
| 310. | H | 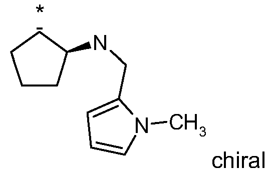 | 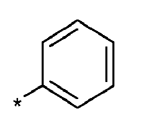 | Schema 7 |
| 311. | H | 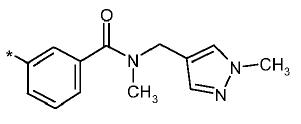 | 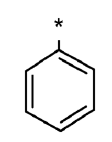 | Schema 12 |
| 312. | H | 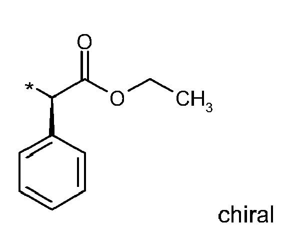 | 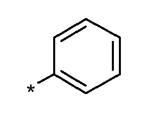 | Schema 1 |
| 313. | H | 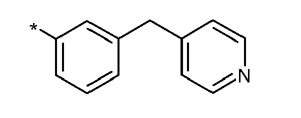 | 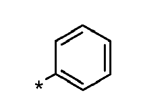 | Schema 1 |
| 314. | H | 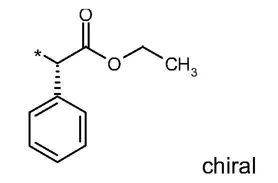 | 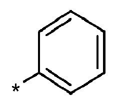 | Schema 1 |
| 315. | H | 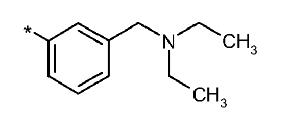 | 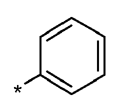 | Schema 1 |
| 316. | H | 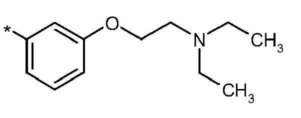 | 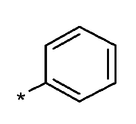 | Schema 1 |
| 317. | H | 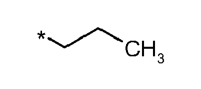 | 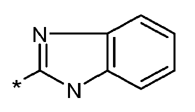 | Schema 1 |
| 318. | H | 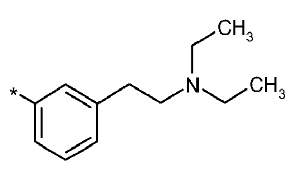 | 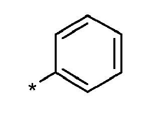 | Schema 1 |
| 319. | H | 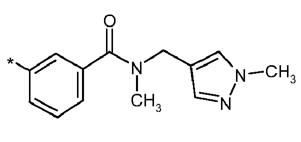 | 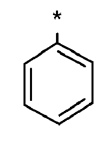 | Schema 12 |
| 320. | H | 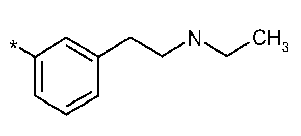 | 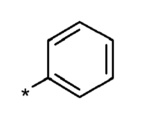 | Schema 1 |
| 321. | H | 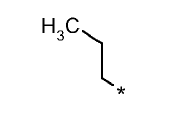 | 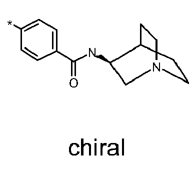 | Schema 2 |
| 322. | H | 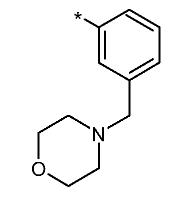 | 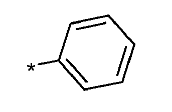 | Schema 1 |
| 323. | H | 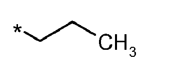 | 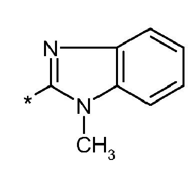 | Schema 1 |
| 324. | H | 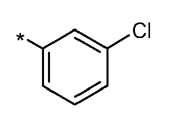 | 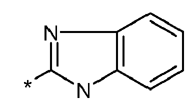 | Schema 1 |
| 325. | H | 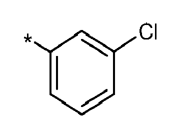 | 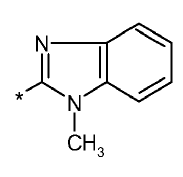 | Schema 1 |
| 326. | H | 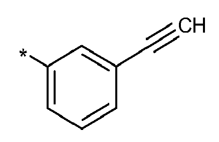 | 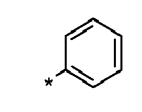 | Schema 1 |
| 327. | H | 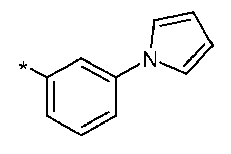 | 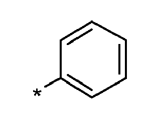 | Schema 1 |
| 328. | H | 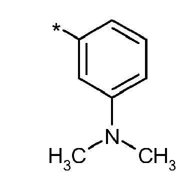 | 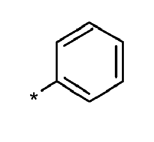 | Schema 1 |
| 329. | H | 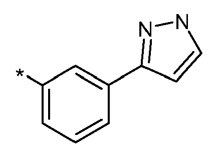 | 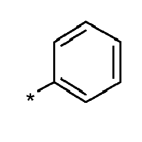 | Schema 1 |
| 330. | H | 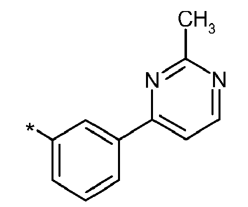 | 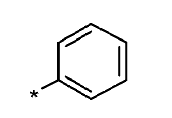 | Schema 1 |
| 331. | H | 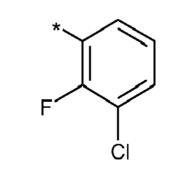 | 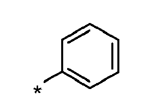 | Schema 1 |
| 332. | H | 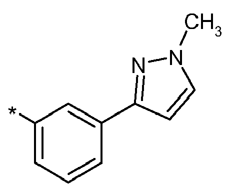 | 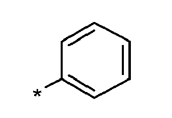 | Schema 1 |
| 333. | H | 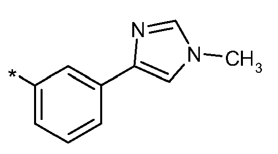 | 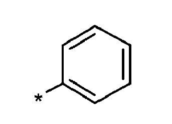 | Schema 1 |
| 334. | H | 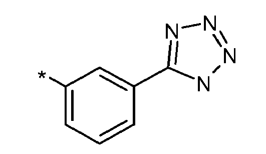 | 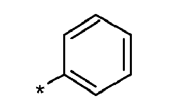 | Schema 1 |
| 335. | H | 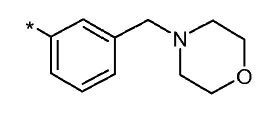 | 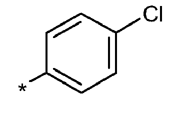 | Schema 1 |
| 336. | H | 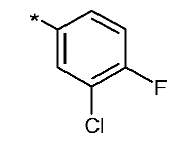 | 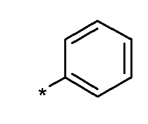 | Schema 1 |
| 337. | H | 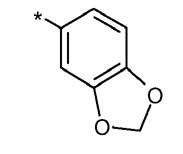 | 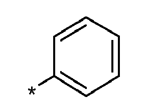 | Schema 1 |
| 338. | H | 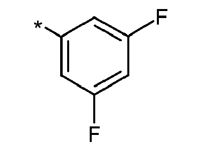 | 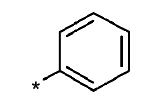 | Schema 1 |
| 339. | H | 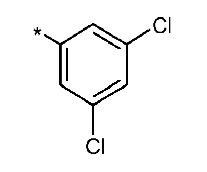 | 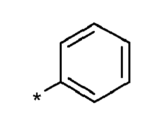 | Schema 1 |
| 340. | H | 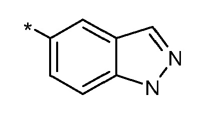 | 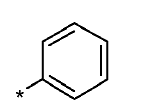 | Schema 1 |
| 341. | H | 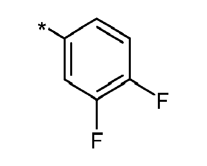 | 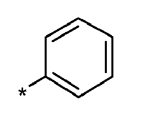 | Schema 1 |
| 342. | H | 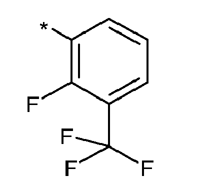 | 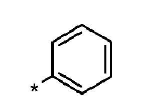 | Schema 1 |
| 343. | H | 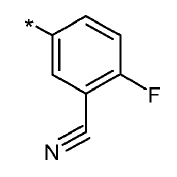 | 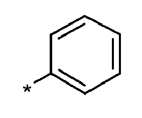 | Schema 1 |
| 344. | H | 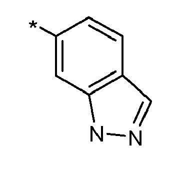 | 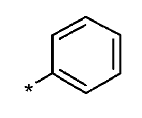 | Schema 1 |
| 345. | H | 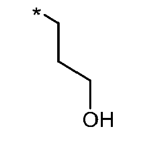 | 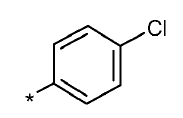 | Schema 1 |
| 346. | H | 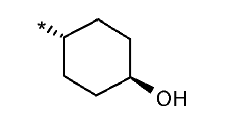 | 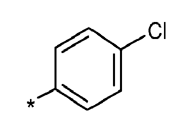 | Schema 1 |
| 347. | H | 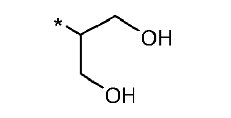 | 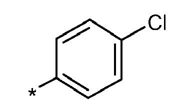 | Schema 1 |
| 348. | H | 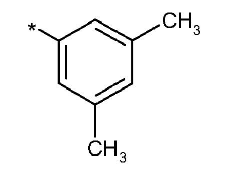 | 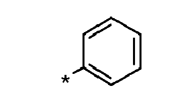 | Schema 1 |
| 349. | H | 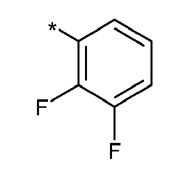 | 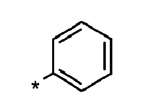 | Schema 1 |
| 350. | H | 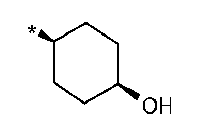 | 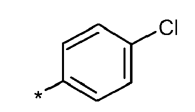 | Schema 1 |
| 351. | H | 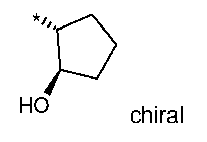 | 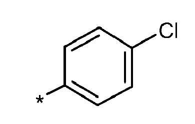 | Schema 1 |
| 352. | H | 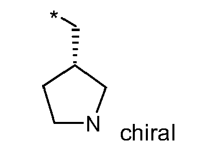 | 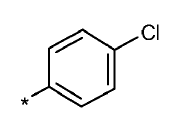 | Schema 1 |
| 353. | H | 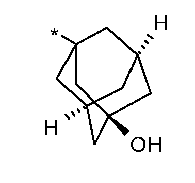 | 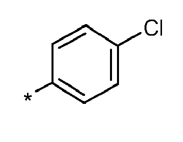 | Schema 1 |
| 354. | H | 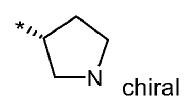 | 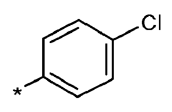 | Schema 1 |
| 355. | H | 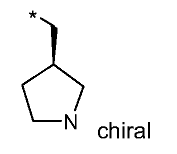 | 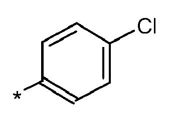 | Schema 1 |
| 356. | H | 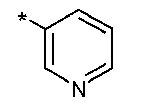 | 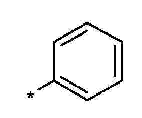 | Schema 1 |
| 357. | H | 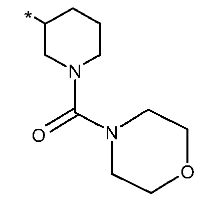 | 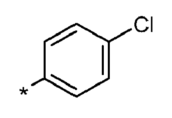 | Schema 11 |
| 358. | H | 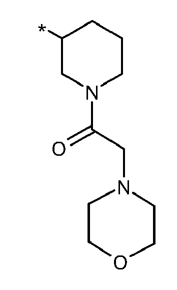 | 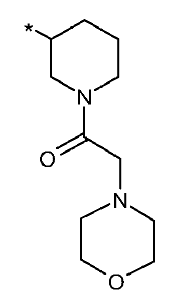 | Schema 11 |
| 359. | H | 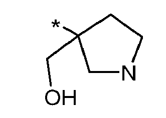 | 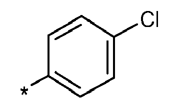 | Schema 1 |
| 360. | H | 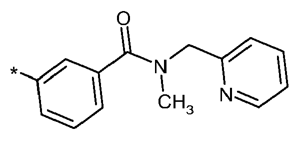 | 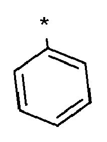 | Schema 12 |
| 361. | H | 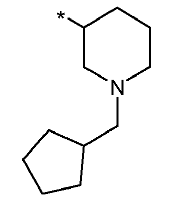 | 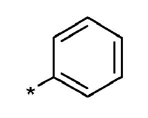 | Schema 11 |
| 362. | H | 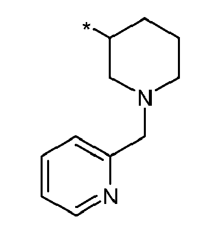 | 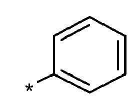 | Schema 11 |
| 363. | H | 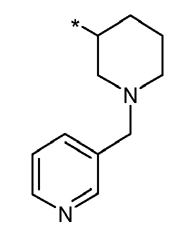 | 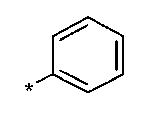 | Schema 11 |
| 364. | H | 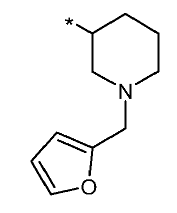 | 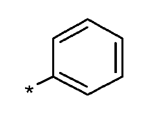 | Schema 11 |
| 365. | H | 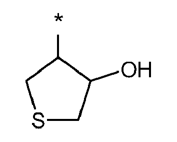 | 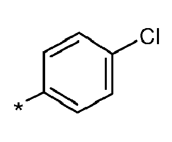 | Schema 1 |
| 366. | H | 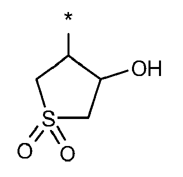 | 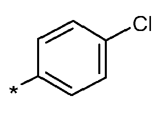 | Schema 1 |

### Beispiel B1

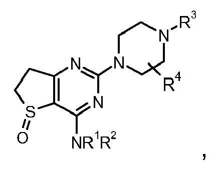

wobei R⁴ = H

| **#** | **R¹** | **R**² | **R**³ | **Herstellung** |
|---|---|---|---|---|
| 367. | H | 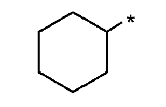 | 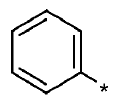 | Schema 1 |
| 368. | H | 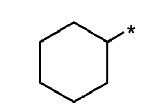 | 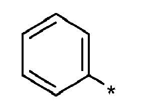 | Schema 1 Enantiomer 2 vom Racemat 297 |
| 369. | H | 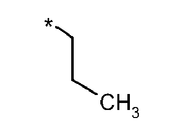 | 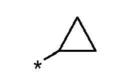 | Schema 1 |
| 370. | H | 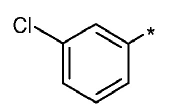 | 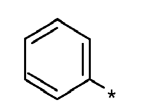 | Schema 1 Enantiomer 1 vom Racemat 300 |
| 371. | H | 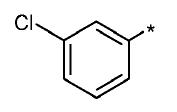 | 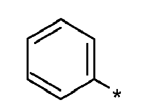 | Schema 1 Enantiomer 2 vom Racemat 300 |
| 372. | H | 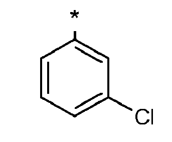 | 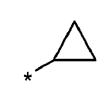 | Schema 1 |

### Beispiel C1

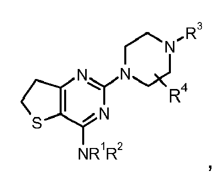

wobei R⁴ = H ist,

| **#** | **NR¹R²** | **R**³ | **Herstellung** |
|---|---|---|---|
| 373. | 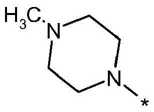 | 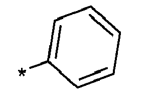 | Schema 1 |
| 374. | 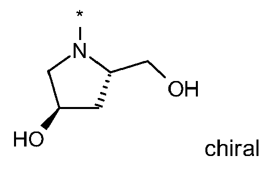 | 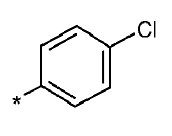 | Schema 1 |

Weitere Beispiele wären:

### Beispiele D1:

| **#** | **Struktur** | **Herstellung** |
|---|---|---|
| 375. | 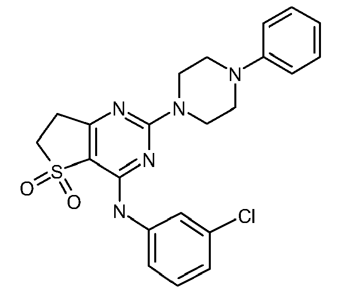 | Schema 9 |
| 376. | 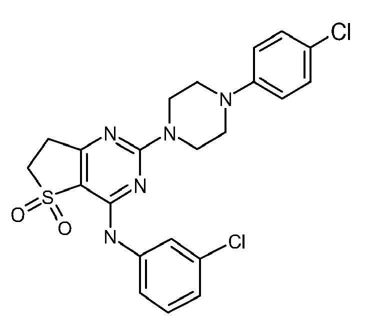 | Schema 9 |
| 377. | 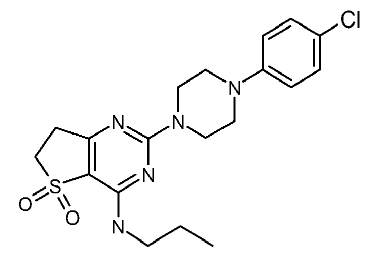 | Schema 9 |
| 378. | 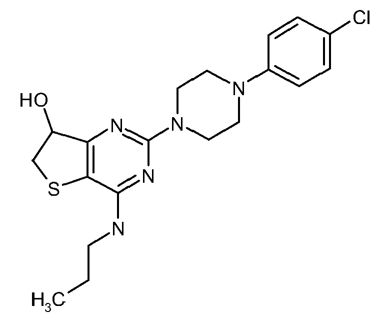 | Schema 14 |

### INDIKATIONSGEBIETE

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel **1** zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel **1** zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel **1** zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weitere PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon, wie beispielsweise Kombinationen von
- Betamimetika mit Corticosteroiden, PDE4-Inhibitoren, EGFR-Hemmern oder LTD4-Antagonisten,
- Anticholinergika mit Betamimetika, Corticosteroiden, PDE4-Inhibitoren, EGFR-Hemmern oder LTD4-Antagonisten,
- Corticosteroiden mit PDE4-Inhibitoren, EGFR-Hemmern oder LTD4-Antagonisten
- PDE4-inhibitoren mit EGFR-Hemmern oder LTD4-Antagonisten
- EGFR-Hemmern mit LTD4-Antagonisten
- MRP4-Inhibitoren.
Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus der Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1H-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropylphenyl)-1,1dimethyl-ethylamino]-ethyl}-4H-benzo[1,4]oxazin-3-on, 8-{2-[2-(4-Ethylphenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika sind ausgewählt aus der Gruppe bestehend aus Fenoterol, Formoterol, Salmeterol, 3-(4-{6-[2-Hydroxy-2-(4-hydroxy-3-hydroxymethylphenyl)-ethylamino]-hexyloxy}-butyl)-benzenesulfoneamid, 5-[2-(5,6-Diethyl-indan-2-ylamino)-1-hydroxy-ethyl]-8-hydroxy-1H-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxyessigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, lpratropiumsalzen, Glycopyrroniumsalzen, Trospiumsalzen 2,2-Diphenylpropionsäuretropenolester-methobromid, 2,2-Diphenylpropionsäurescopinestermethobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäurescopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäurescopinester Methobromid, Benzilsäurecyclopropyltropinester-Methobromid, 2,2-Diphenylpropionsäurecyclopropyltropinester -Methobromid, 9-Hydroxy-xanthen-9-carbonsäurecyclopropyltropinesterMethobromid, 9-Methyl-fluoren-9-carbonsäurecyclopropyltropinester-Methobromid, 9-Methyl-xanthen-9-carbonsäurecyclopropyltropinester -Methobromid, 9-Hydroxy-fluoren-9-carbonsäurecyclopropyltropinester -Methobromid, 4,4'-Difluorbenzilsäuremethylestercyclopropyltropinester - Methobromid, 9-Hydroxy-xanthen-9-carbonsäuretropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid. 9-Hydroxymethyl-xanthen-9-carbonsäurescopinester -Methobromid, gegebenenfalls in Form ihrer Solvate oder Hydrate.

In den vorstehend genannten Salzen stellen die Kationen Tiotropium, Oxitropium, Flutropium, lpratropium, Glycopyrronium und Trospium die pharmakologisch aktiven Bestandteile dar. Als Anionen können die vorstehend genannten Salze bevorzugt enthalten Chlorid, Bromid, lodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, lodid, Sulfat, Methansulfonat oder p-Toluolsulfonat als Gegenionen bevorzugt sind. Von allen Salzen sind die Chloride, Bromide, lodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort,RPR-106541, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, Dexamethason,NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy]-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als weitere PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370,N-(3,5-Dichloro-1-oxo-pyridin-4-yl)-4-difluoromethoxy-3-cyclopropylmethoxybenzamid, (-)p-[(4aR*,10bS*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-l-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Enprofyllin, Roflumilast, Ariflo (Cilomilast), Arofyllin, Atizoram,AWD-12-281 (GW-842470), T-440, T-2585, PD-168787, V-11294A, CI-1018, CDC-801, D-22888, YM-58997, Z-15370, N-(3,5-Dichloro-1-oxo-pyridin-4-yl)-4-difluoromethoxy-3-cyclopropylmethoxybenzamid, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin,AWD-12-281 (GW-842470), 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-l-ol], Atizoram, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2- hydroxy-2-propyl)phenyl)thio)methylcyclopropan-essigsäure, 1-(((1(R)-3(3-(2-(2,3-Dichloro-thieno[3,2-b]pyridin-5-yl)-(E)-ethenyl)phenyl)-3-(2-(1-hydroxy-1-methylethyl)phenyl)-propyl)thio)methyl)cyclopropanessigsäure und [2-[[2-(4-tert-Butyl-2-thiazolyl)-5-benzofuranyl]oxymethyl]phenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenylethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluorphenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluorphenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenylethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluorphenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methylpiperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluorphenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl]-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amlno]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluorphenyl)amino]-6-(1-ethyl-piperidin-4-yloxy)-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluorphenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluorphenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin und 4-[(3-Chlor-4-fluor-phenyl)amino]-6-(1-cyano-piperidin-4-yloxy)-7-methoxy-chinazolin gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-*S*-glutathione, Estradiol 17-β-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), α-Naphthyl-β-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen SäureAdditionssalze und Hydrate.

Vorzugsweise bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus *N*-Acetyl-dinitrophenyl-Cysteine, Dehydroepiandrosterone 3-sulphate, Dilazep, Dinitrophenyl-*S*-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil, Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen SäureAdditionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen SäureAdditionssalze und Hydrate. Die Trennung von Enantiomeren aus den Racematen kann durch bekannte Verfahren nach dem Stand der Technik durchgeführt werden (z.B. durch Chromatographie an chiralen Phasen etc.).

Mit Säure-Additionssalzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromaleaten, Hydroacetaten, Hydrobenzoaten, Hydrocitraten, Hydrofumaraten, Hydrotartraten, Hydrooxalaten, Hydrosuccinaten, Hydrobenzoaten and Hydro-p-toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen PDE4B-Inhibitoren, von MRP4-Inhibitoren und einer weiteren aktiven Substanz wie z.B. einem Anticholinergikum, einem Steroid, einem LTD4-Antagonist oder einem Betamimetikum enthalten, sowie deren Herstellung und deren Verwendung zur Behandlung von Atemwegserkrankungen.

### DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **1** gemäß der obigen bevorzugten Ausführungsformen.

Besonders bevorzugt ist es, wenn die Verbindungen der Formel **1** oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **1** inhalativ verabreicht werden, besonders bevorzugt ist es, wenn die Verabreichung ein oder zweimal täglich erfolgt. Hierzu müssen die Verbindungen der Formel **1** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **1** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, ein Pteridin und ein oder mehrere Kombinationspartner ausgewählt aus der oben beschriebenen Gruppe, bereit gestellt.

## Patentansprüche

1. Verbindungen der Formel **1**, 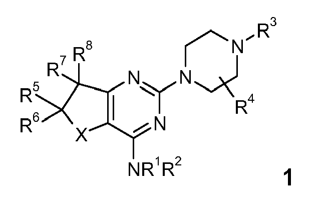 worin
**X** O, S, SO oder SO₂;
**R¹** H, C₁₋₆-Alkyl, C₂₋₆-Alkenyl, C₂₋₆-Alkinyl, C₆₋₁₀-Aryl oder C₆₋₁₀-Aryl-C₁₋₆-alkylen, C₅₋₁₀-Heteroaryl-C₁₋₆-alkylen;
**R²** H oder ein gegebenenfalls einfach oder mehrfach substituierter Rest ausgewählt aus der Gruppe bestehend aus C₁₋₆-Alkyl, C₂₋₆-Alkenyl, C₂₋₆-Alkinyl, ein gegebenenfalls einfach oder mehrfach überbrücktes mono- oder bicyclisches C₃₋₁₀-Cycloalkyl, C₆₋₁₀-Aryl, ein aromatischer oder nicht aromatischer, heterocyclischer C₃₋₁₀-Ring, ein bicyclischer Ring und ein mit einem C₃₋₁₀-Heterocyclus kondensiertes C₆₋₁₀-Aryl;
oder **NR¹R²** bedeutet gemeinsam einen heterocyclischen Ring, der gegebenenfalls mit einem oder mehreren Resten ausgewählt aus C₁₋₄-Alkyl, C₂₋₆-Alkenyl, C₂₋₆-Alkinyl, linearem oder verzweigtem C₁₋₆-Alkanol und Oxo substituiert ist;
**R³** ein einfach oder mehrfach substituierter Rest ausgewählt aus der Gruppe bestehend aus einem heterocyclischen C₆₋₁₀-Ring, C₃₋₇-Cycloalkyl, C₆₋₁₀-Aryl-C₁₋₆-alkylen, C₅₋₁₀-Heteroaryl-C₁₋₆-alkylen, einem kondensierten, bicyclischen Ring, der gegebenenfalls 1-4 Heteroatome unabhängig voneinander ausgewählt aus N, O, S enthalten kann,
oder **R³** bedeutet gegebenenfalls substituiertes Phenyl;
oder **R³** bedeutet eine Gruppe COR^{3.7}, COCH₂R^{3.8}, CONHR^{3.8} oder SO₂R^{3.8};
wobei
R^{3.7} H, C₁₋₆-Alkyl, C₂₋₆-Alkenyl, C₂₋₆-Alkinyl oder C₆₋₁₀-Aryl;
R^{3.8} C₁₋₆-Alkyl, C₂₋₆-Alkenyl, C₂₋₆-Alkinyl, C₃₋₇-Cycloalkyl oder ein Rest ausgewählt aus der Gruppe bestehend aus C₆₋₁₀-Aryl, einem heterocyclischen C₃₋₁₀-Ring und einem bicyclischen Ring, der gegebenenfalls mit einem oder mehreren Resten ausgewählt aus der Gruppe bestehend aus C₁₋₆-Alkyl, C₂₋₆-Alkenyl, C₂₋₆-Alkinyl, Halogen, NR^{3.8.1}R^{3.8.2}, C₆₋₁₀-Aryl und einem heterocyclischen C₃₋₁₀-Ring substituiert ist;
wobei
R^{3.8.1} H, C₁₋₆-Alkyl, C₂₋₆-Alkenyl oder C₂₋₆-Alkinyl;
R^{3.8.2} H, C₁₋₆-Alkyl, C₂₋₆-Alkenyl oder C₂₋₆-Alkinyl;
**R⁴** H, C₁₋₄-Alkyl, C₂₋₄-Alkenyl, C₂₋₄-Alkinyl, oder Oxo;
**R⁵** H, C₁₋₄-Alkyl, C₂₋₄-Alkenyl oder C₂₋₄-Alkinyl;
**R⁶** H, C₁₋₄-Alkyl, C₂₋₄-Alkenyl oder C₂₋₄-Alkinyl;
**R⁷** H, C₁₋₄-Alkyl, C₂₋₄-Alkenyl, C₂₋₄-Alkinyl, C₆₋₁₀-Aryl, oder OH;
**R⁸** H, C₁₋₄-Alkyl, C₂₋₄-Alkenyl, C₂₋₄-Alkinyl, C₆₋₁₀-Aryl oder OH;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

2. Verbindungen der Formel 1, nach Anspruch 1, worin
**X** O, S, SO oder SO₂;
**R¹** H, C₁₋₆-Alkyl, C₆₋₁₀-Aryl, C₆₋₁₀-Aryl-C₁₋₆-alkylen, C₅₋₁₀-Heteroaryl-C₁₋₆-alkylen,
**R²** H oder C₁₋₆-Alkyl, das gegebenenfalls mit einem oder mehreren Resten ausgewählt aus der Gruppe bestehend aus C₁₋₆-Haloalkyl, CN, OR^{2.1}, NR^{2.1}R^{2.2}, COOR^{2.1}, CONR^{2.1}R^{2.2}, gegebenenfalls mit C₁₋₄-Alkyl oder mit Oxo substituiertes C₃₋₇-Cycloalkyl, gegebenenfalls mit C₁₋₄-Alkyl, mit Oxo, mit OH oder mit Halogen substituierter aromatischer oder nicht aromatischer Heterocyclus, gegebenenfalls mit C₁₋₄-Alkyl oder mit Oxo substituiertes C₆₋₁₀-Aryl und mit einem C₅₋₆-Heterocyclus kondensiertes C₆₋₁₀-Aryl, wobei dieses kondensierte Ringsystem gegebenenfalls mit C₁₋₄-Alkyl oder mit Oxo substituiert sein kann, substituiert sein kann
wobei
R^{2.1} H oder C₁₋₆-Alkyl;das gegebenenfalls substituiert ist mit einem jeweils gegebenenfalls substituierten C₃₋₇-Cycloalkyl, C₃₋₁₀-Heterocyclus oder C₆₋₁₀-Aryl
R^{2.2} H oder C₁₋₆-Alkyl;
oder **R²** ein Rest ausgewählt aus gegebenenfalls einfach oder mehrfach überbrücktem C₃₋₁₀-Cycloalkyl oder ein C₃₋₁₀-Cycloalkyl, das gegebenenfalls kondensiert sein kann mit einem C₆₋₁₀-Arylring, der gegebenenfalls mit einem oder mehreren Resten ausgewählt aus der Gruppe bestehend aus C₁₋₆-Alkyl, OH, CH₂OR^{2.3}, COOR^{2.3}, COR^{2.3}, CONR^{2.3}R^{2.4}, O-C₁₋₆-Alkyl, O-C₇₋₁₁-Aralkyl, NR^{2.3}R^{2.4} und NHCOR^{2.5}substituiert sein kann ,
wobei
R^{2.3} H oder ein Heterocyclus ist oder ein C₁₋₆-Alkyl ist, welches gegebenenfalls mit einem Rest ausgewählt aus C₃₋₇-Cycloalkyl, C₃₋₁₀-Heterocyclus und C₆₋₁₀-Aryl substituiert sein kann, wobei dieser Rest jeweils gegebenenfalls mit einem oder mehreren Resten aus der Gruppe C₁₋₆-Alkyl, Halogen, OH und O-C₁₋₆-Alkyl substituiert sein kann ;
wobei
R^{2.4} H oder C₁₋₆-Alkyl;
R^{2.5} ein Rest ausgewählt aus der Gruppe bestehend aus C₃₋₇-Cycloalkyl, ein heterocyclischer C₃₋₁₀-Ring und C₁₋₆-Alkyl, welcher gegebenenfalls mit OH substituiert sein kann;
oder R² bedeutet eine Gruppe der Formel **1a,** 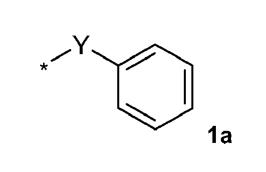
Y C₁₋₆-Alkylen, gegebenenfalls substituiert mit einem oder zwei R^{2.7}
wobei
R^{2.7} jeweils unabhängig voneinander ausgewählt sind aus C₁₋₆-Alkyl,COOH, CONH₂, OR^{2.1}, und COOR^{2.1}; oder R^{2.7} bildet gemeinsam mit einem oder zwei Kohlenstoffatomen von Y einen carbocyclischen Ring mit 3 Kohlenstoffatomen
oder **R²** C₆₋₁₀-Aryl, das gegebenenfalls mit einem oder mehreren Resten unabhängig voneinander ausgewählt aus der Gruppe bestehend aus C₁₋₆-Alkyl, C₁₋₆-Haloalkyl, C₂₋₆-Alkenyl, C₂₋₆-Alkinyl, CN, Halogen, OR^{2.8}, COOR^{2.8}, COR^{2.10}, NHCOMe, CONR^{2.3}R^{2.4}, einem mit NR^{2.1}R^{2.2} substituierten C₁₋₄Alkylenrest, NR^{2.1}R^{2.2} substituiert sein kann
oder **R²** C₆₋₁₀-Aryl, das gegebenenfalls mit einem oder mehreren Resten unabhängig voneinander ausgewählt aus der Gruppe bestehend aus C₆₋₁₀-Aryl-C₁₋₆-alkylen, C₅₋₁₀-Heteroaryl-C₁₋₆-alkylen, C₃₋₇-Cycloalkyl, ein C₃₋₇Cycloalkyl-C₁₋₄-alkylen, C₆₋₁₀-Aryl, und einem heterocyclischen C₃₋₁₀-Ring substituiert sein kann, wobei diese Reste jeweils gegebenenfalls mit einem oder mehreren Resten ausgewählt aus C₁₋₆-Alkyl, C₁₋₆-Haloalkyl, COOR^{2.8}, CN, Halogen, OR^{2.8}, NHCOR^{2.8}, Oxo, einem C₃₋₁₀-Heterocyclus, einem C₃₋₇-Cycloalkyl-C₁₋₄alkylen, ein C₅₋₁₀-Heterocyclus -C₁₋₄-alkylen und einem NR^{2.1}R^{2.2} -C₁₋₄ alkylen substituiert sein können,
wobei
R^{2.8} H, C₁₋₆-Alkyl, C₆₋₁₀-Aryl, ein NR^{2.1}R^{2.2}-C₁₋₄alkylenrest, ;
R^{2.10} NHR^{2.10.1} oder ein heterocyclischer C₃₋₁₀-Ring, der gegebenenfalls mit C₁₋₄-Alkyl substituiert sein kann;
R^{2.10.1} H, C₃₋₇-CYCloalkyl, C₂₋₆-Alkenyl, C₂₋₆-Alkinyl,C₁₋₆-Alkyl oder C₁₋₆-Alkyl-O-C₁₋₄-Alkyl
oder **R²** bedeutet C₆₋₁₀-Aryl, an das ein aromatischer oder nicht aromatischer C₃₋₁₀-Heterocyclus ankondensiert ist;
oder **R²** aromatischer oder nicht aromatischer heterocyclischer C₃₋₁₀-Ring, der gegebenenfalls mit einem oder mehreren Resten ausgewählt aus der Gruppe bestehend aus Halogen, OH, Oxo-, CN, C₁₋₆-Alkyl, C₁₋₆-Alkanol, C₆₋₁₀-Aryl-C₁₋₆-alkylen, C₅₋₁₀-Heteroaryl-C₁₋₆-alkylen, COR^{2.11}, C₃₋₇-Cycloalkyl-C₁₋₄-alkylen und C₃₋₁₀-Heterocyclus-C₁₋₄-alkylen substituiert sein kann;
wobei
R^{2.11} ein Rest ausgewählt aus der Gruppe bestehend aus C₃₋₁₀-Heterocyclus-C₁₋₄-alkylen, C₃₋₇-Cycloalkyl und einem heterocyclischen aromatischen oder nichtaromatischen C₃₋₁₀-Ring,
welcher gegebenenfalls mit C₁₋₆-Alkyl substituiert sein kann, das wiederum gegebenenfalls mit OH, CH₂OH, OMe, NH₂, einem C₃₋₁₀-Heterocyclus oder NHCOO-^{t}Bu substituiert sein kann;
oder **R²** ein Rest ausgewählt aus der Gruppe bestehend aus C₂₋₆-Alkenyl oder einem bicyclischen Ring, welcher gegebenenfalls mit Methyl substituiert sein kann;
oder **NR¹R²** ein heterocyclischer Ring, der gegebenenfalls mit einem oder mehreren Resten ausgewählt aus der Gruppe C₁₋₄-Alkyl, OH und C₁₋₄-Alkanol substituiert sein kann;
**R³** ein Rest ausgewählt aus der Gruppe bestehend aus einem heterocyclischen C₃₋₁₀-Ring, einem C₃₋₇-Cycloalkyl, einem bicyclischen, kondensierten aromatischen oder nicht aromatischem Ringsystem, das gegebenenfalls 1 bis 4 Heteroatome ausgewählt aus S, N, O enthält, C₆₋₁₀-Aryl-C₁₋₆-alkylen, C₅₋₁₀-Heteroaryl-C₁₋₆-alkylen und CH₂-Benzo[1, 3]dioxolyl, der gegebenenfalls mit einem oder mehreren Resten ausgewählt aus OH, Halogen, C₁₋₆-Alkyl, O-C₁₋₆-Alkyl, C₁₋₆-Haloalkyl und CO-R^{3.1} substituiert sein kann,
oder **R³** Phenyl, welches gegebenenfalls mit einem oder mehreren Resten ausgewählt aus der Gruppe bestehend aus C₁₋₆-Alkyl, C₂₋₆-Alkenyl, C₂₋₆-Alkinyl, C₁₋₄-Haloalkyl, C₁₋₆-Alkylen-NR^{3.1}R^{3.2}, CN, COOR^{3.1}, CONR^{3.1}R^{3.2}, NR^{3.1}R^{3.2}, NHCOR^{3.1}, CF₃, OR^{3.1}, Halogen, NHCOR^{3.1}, NO₂, SO₂NR^{3.1}R^{3.2} und C₁₋₆-Alkylen-NHCOR^{3.1}substituiert sein kann;
wobei
R^{3.1} H ,C₁₋₆-Alkyl; C₂₋₆-Alkenyl oder C₂₋₆-Alkinyl, gegebenenfalls überbrückter, mono- oder bicyclischer C₃₋₁₀-Heterocyclus oder C₃₋₁₀-Heterocyclus-C₁₋₄-Alkylenrest;
R^{3.2} H , C₁₋₆-Alkyl, , C₂₋₆-Alkenyl oder C₂₋₆-Alkinyl;
oder **R³** eine Gruppe der Formel 1 b 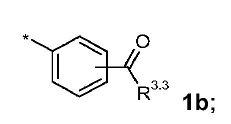 worin
R^{3.3} ein Rest ausgewählt aus der Gruppe bestehend aus einem heterocyclischen C₃₋₁₀-Ring, der gegebenenfalls mit einem oder mehreren Resten ausgewählt aus der Gruppe bestehend aus C₁₋₆-Alkyl, Oxo, COR^{3.3.1}, COR^{3.3.2}, C₁₋₆-Alkylen-R^{3.3.2}, CH₂CO-Pyrrolidin und einem heterocyclischen C₃₋₁₀-Ring, worin ein gegebenenfalls im heterocyclischen Ring enthaltenes Schwefelatom, gegebenenfalls als Oxid oder Dioxid vorliegen kann, substituiert sein kann;
wobei
R^{3.3.1} C₁₋₆-Alkyl;
R^{3.3.2} NH₂, NH(C₁₋₆-Alkyl), N(C₁₋₆-Alkyl)₂;
oder R^{3.3} bicyclischer Ring oder heterocyclischer Spiroring;
oder **R³** eine Gruppe der Formel **1c;** 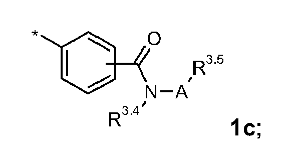 worin
A Bindung oder C₁₋₆-Alkyl, das gegebenenfalls mit Oxo oder NMe₂ substituiert sein kann;
R^{3.4} H oder C₁₋₆-Alkyl;
R^{3.5} ein Rest ausgewählt aus der Gruppe bestehend aus
C₁₋₆-Alkyl, das gegebenenfalls mit einem oder mehreren Resten ausgewählt aus C₃₋₇-Cycloalkyl, C₆₋₁₀-Aryl und einem C₃₋₁₀-Heterocyclus substituiert sein kann, wobei dieser Rest jeweils auch gegebenenfalls mit einem Rest ausgewählt aus der Gruppe OH, Oxo, C₁₋₆-Alkyl, O-C₁₋₆-Alkyl, C₁₋₆-Haloalkyl substituiert sein kann,
oder ein Rest ausgewählt aus einem heterocyclischer C₃₋₁₀-Ring und einem bicyclischen Ring, der gegebenenfalls mit einem oder mehreren Resten unabhängig voneinander ausgewählt aus der Gruppe bestehend aus Oxo, C₁₋₆-Alkyl, OH, C₆₋₁₀-Aryl, einem heterocyclischen C₃₋₁₀-Ring,
C₁₋₆-Alkylen-R^{3.5.1}, O-C₁₋₆-Alkylen-R^{3.5.1}und NH-C₁₋₆-Alkylen-R^{3.5.1}substituiert sein kann,
R^{3.5.1} ein Rest ausgewählt aus der Gruppe bestehend aus C₆₋₁₀-Aryl, heterocyclischer C₃₋₁₀-Ring, der gegebenenfalls mit C₁₋₆-Alkyl substituiert sein kann;
oder **R³** bedeutet eine Gruppe der Formel **1d;** 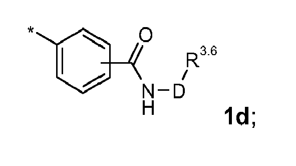 worin
D C₂₋₄-Alkinyl; ein gegebenenfalls überbrückter, bicyclischer C₃₋₁₀-Cycloalkylrest, der gegebenenfalls einem oder mehreren Resten ausgewählt aus mit C₁₋₆-Alkyl, Halogen, OH, C₁₋₆-Haloalkyl und O-C₁₋₆-Alkyl substituiert sein kann,
R^{3.6} Pyridinyl;
oder **R³** bedeutet eine einen Rest ausgewählt aus der Gruppe aus COR^{3.7}, COCH₂R^{3.8}, CONHR^{3.8}, SO₂R^{3.8} und einem mit einem C₆₋₁₀-Arylrest kondensierten Heterocyclus, der gegebenenfalls mit Methyl substituiert sein kann,
oder **R³** eine Gruppe der Formel **1e;** 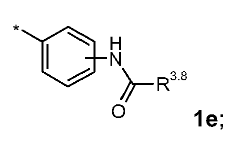
R^{3.7} H, C₁₋₆-Alkyl, C₆₋₁₀-Aryl;
R^{3.8} Rest ausgewählt aus der Gruppe aus C₁₋₆-Alkyl, C₃₋₇-Cycloalkyl oder ein Rest ausgewählt aus der Gruppe bestehend aus C₆₋₁₀-Aryl, heterocyclischer C₃₋₁₀-Ring und ein bicyclischer Ring, der gegebenenfalls mit einem oder mehreren Resten ausgewählt aus der Gruppe bestehend aus C₁₋₆-Alkyl, Halogen, NR^{3.8.1}R^{3.8.2}, C₆₋₁₀-Aryl und einem heterocyclischen C₃₋₁₀-Ring substituiert sein kann;
R^{3.8.1} H oder C₁₋₆-Alkyl;
R^{3.8.2} H oder C₁₋₆-Alkyl;
**R⁴** H, C₁₋₄-Alkyl oder Oxo;
**R⁵** H oder C₁₋₄-Alkyl;
**R⁶** H oder C₁₋₄-Alkyl;
**R⁷** H, C₁₋₄-Alkyl, C₆₋₁₀-Aryl oder OH;
**R⁸** H, C₁₋₄-Alkyl, C₆₋₁₀-Aryl oder OH;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

3. Verbindungen der Formel **1,** nach einem der Ansprüche 1 oder 2, worin
**X** O, S, SO oder SO₂;
**R¹** H, Methyl, Ethyl oder Propyl;
**R²** H oder C₁₋₆-Alkyl, das gegebenenfalls mit einem oder mehreren Resten ausgewählt aus der Gruppe bestehend aus CF₃, CN, OH, NMe₂, OMe, COOH und CONMe₂ substituiert sein kann,
oder **R²** C₁₋₆-Alkyl, das gegebenenfalls mit einem oder mehreren Resten ausgewählt aus Cyclopropyl, Cyclopentyl, Cyclohexyl, Phenyl, Pyrrolidinyl, Imidazolidinyl, Pyrazolyl, Imidazolyl und Pyridinyl substitutiert sein kann, der gegebenenfalls mit Methyl oder Oxo substituiert sein kann;
oder **R²** C₃₋₇-Cycloalkyl, das gegebenenfalls mit einem Rest ausgewählt aus der Gruppe bestehend aus Methyl, OR^{2.3} ,CH₂OR^{2.3}, COOH, CONR^{2.3}R^{2.4}, CONH-^{t}Bu, O-Benzyl, NR^{2.3}R^{2.4} und NHCOR^{2.5} substituiert sein kann
wobei
R^{2.3} H oder Methyl, 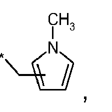
R^{2.4} H oder Methyl;
R^{2.5} CH₂C(CH₃)₃, CH₂C(CH₃)₂(CH₂OH), Cyclopentyl, Tetrahydrofuranyl, Pyrrolyl, Pyrazolyl, Imidazolyl oder Isoxazolyl;
oder **R²** bedeutet eine Gruppe der Formel **1a,** 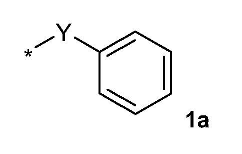
Y C₁₋₄-Alkylen, gegebenenfalls substituiert mit einem oder zwei R^{2.7}
R^{2.7} jeweils unabhängig voneinander C₁₋₄-Alkyl, COOH, CONH₂; oder R^{2.7} bildet gemeinsam mit einem oder zwei Kohlenstoffatomen von Y einen carbocyclischen Ring mit 3 Kohlenstoffatomen
oder **R²** C₆₋₁₀-Aryl, das gegebenenfalls mit einem oder mehreren Resten unabhängig voneinander ausgewählt aus der Gruppe bestehend aus Methyl, *tert*-Butyl, CN, F, Cl, Br, OH, OMe, OEt, O-Phenyl, COOH, COOMe, COR^{2.10}, NHCOMe und mit Morpholin substituiertes C₁₋₄-Alkylen substituiert sein kann,
wobei
R^{2.10} NH₂, NHMe, NH-ⁱPr, NH-Cyclopropyl, NHCH₂CH₂OMe oder ein heterocyclischer, nichtaromatischer C₃₋₁₀-Ring, der ein, zwei oder drei Heteroatome ausgewählt aus der Gruppe Sauerstoff und Stickstoff enthalten kann;
oder **R²** C₆₋₁₀-Aryl, das gegebenenfalls mit einem Rest ausgewählt aus der Gruppe bestehend aus Phenyl und einem heterocyclischen C₃₋₁₀-Ring substituiert sein kann, welcher gegebenenfalls mit einem oder mehreren Resten aus der Gruppe bestehend aus Methyl, *tert*-Butyl, COOH, COOMe, CN, F, Cl, Br, OH, OMe, OEt und NHCOMe, Oxo substituiert sein kann;
oder **R²** ein heterocyclischer nichtaromatischer C₃₋₁₀-Ring, der gegebenenfalls mit einem Rest ausgewählt aus der Gruppe bestehend aus Benzyl und COR^{2.11} substituiert sein kann;
wobei
R^{2.11} ein Rest ausgewählt aus der Gruppe bestehend aus Cyclopentyl, Tetrahydrofuranyl, Furan, Pyridyl, Pyrrolyl, Pyrrazolyl, Imidazolyl, der gegebenenfalls mit einer oder zwei Methylgruppen, oder mit einem oder mehreren Resten aus der Gruppe CH₂C(CH₃)₃, C(CH₃)₂(CH₂OH), CH₂OMe, C(CH₃)₂NH₂ und C(CH₃)₂NHCOO-^{t}Bu substituiert sein kann;
oder **R²** ein Rest ausgewählt aus der Gruppe bestehend aus C₂₋₆-Alkenyl, Indanyl, 1, 2, 3, 4-Tetrahydro-naphthalyl und 8-Methyl-8-aza-bicyclo[3.2.1]octan;
oder **NR¹R²** ein Rest ausgewählt aus der Gruppe bestehend aus Pyrrolidinyl, Piperidinyl, Piperazinyl und Morpholinyl, der gegebenenfalls mit Methyl substituiert sein kann;
**R³** ein Rest ausgewählt aus der Gruppe bestehend aus Pyridinyl, Pyrimidin, Benzyl, und CH₂-Benzo[1, 3]dioxolyl;
oder **R³** Phenyl, das gegebenenfalls mit einem, zwei oder drei Resten ausgewählt aus der Gruppe bestehend aus Methyl, CH₂NH₂, CN, COOH, CONH₂, CF₃, OH, F, Cl, Br, OMe, NHCOMe, NR^{3.1}COR^{3.2}, CONR^{3.1}R^{3.2}, NO₂, SONMe₂ und CH₂NHCOMe substituiert sein kann;
wobei
R^{3.1} H , C₁₋₆-Alkyl oder ein gegebenenfalls überbrückter, mono- oder bicyclischer C₃₋₁₀-Heterocyclus
R^{3.2} H oder C₁₋₆-Alkyl;
oder **R³** eine Gruppe der Formel **1b** 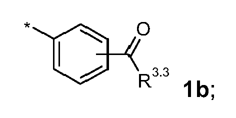 worin
R^{3.3} ein Rest ausgewählt aus der Gruppe bestehend aus Piperidinyl, Piperazinyl, Azepanyl, der gegebenenfalls mit einem oder mehreren Resten unabhängig voneinander ausgewählt aus der Gruppe bestehend aus Methyl, Oxo, COCH₃, CONH₂, CH₂NEt₂, CH₂CH₂NMe₂, CH₂CO-Pyrrolidin, Pyridinyl, Isothiazolidinyl-1, 1-dioxid und Thiazolidinyl-1, 1-dioxid substituiert sein kann;
oder R^{3.3} bedeutet einen Rest der Formel 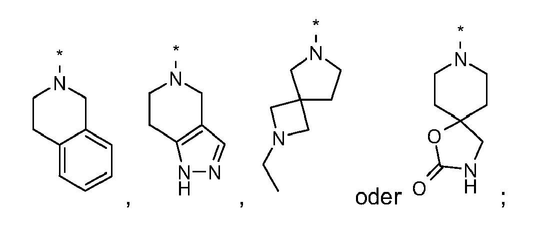
oder **R³** bedeutet eine Gruppe der Formel 1 c; 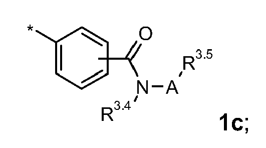 worin
A Bindung oder C₁₋₄-Alkyl, das gegebenenfalls mit Oxo oder mit NMe₂ substituiert sein kann,
R^{3.4} H oder Methyl;
R^{3.5} ein Rest ausgewählt aus der Gruppe bestehend aus Pyrrolidinyl, Piperidinyl, Piperazinyl, Morpholinyl, Cyclohexyl, Imidazolyl, Pyrazolyl, Phenyl, Pyridinyl, Benzimidazolyl, Imidazolidin-2-on, Pyrrolidin-2-on, Pyrrolidin-3-on, Tetrahydro-thiophene 1, 1-dioxide und 1-Aza-bicyclo[2.2.2]octan, der gegebenenfalls mit einem oder mehreren Resten unabhängig voneinander ausgewählt aus der Gruppe bestehend aus Methyl, Ethyl, OH, Phenyl, Pyridinyl, Pyrazolyl, Pyrrolidinyl, (CH₂)ₒ-R^{3.5.1}, O-(CH₂)ₒ-R^{3.5.1} und NH-(CH₂)ₒ-R^{3.5.1}substituiert sein kann;
wobei
o 0, 1 oder 2
R^{3.5.1} ein Rest ausgewählt aus der Gruppe bestehend aus Phenyl, Pyrrolidinyl, Piperidinyl und Imidazolidin-2-on, der gegebenenfalls mit Methyl substituiert sein kann;
oder **R³** bedeutet eine **Gruppe der Formel 1d;** 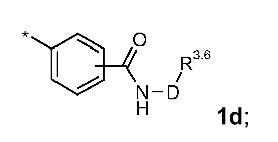 worin
D C₂₋₄-Alkinyl;
R^{3.6} Pyridinyl;
oder **R³** bedeutet eine Gruppe COR^{3.7}, COCH₂R^{3.8}, CONHR^{3.8}, SO₂R^{3.8} oder eine Gruppe der Formel **1e;** 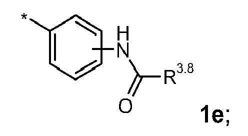 worin
R^{3.7} H, Methyl, Phenyl;
R^{3.8} ein Rest ausgewählt ist aus *iso*-Propyl, Cyclopropyl, Cyclopentyl, Cyclohexyl, Pyrrolidinyl, Pyrrolidin-2-on, Furanyl und Azabicyclo[2.2.2]octanyl oder ein Rest ausgewählt ist aus der Gruppe bestehend aus Piperidinyl, Pyrazolyl, Imidiazolyl, Isoxazolyl, Pyridinyl, Phenyl, Benzyl, der gegebenenfalls mit einem oder mehreren Resten ausgewählt aus der Gruppe bestehend aus Methyl, Chlor, NH₂, NMe₂, Phenyl und Morpholinyl substituiert sein kann;
**R⁴** H, Methyl oder Oxo;
**R⁵** H oder Methyl;
**R⁶** H oder Methyl;
**R⁷** H, Methyl oder OH; bevorzugt H oder Methyl;
**R⁸** H, Methyl oder OH; bevorzugt H oder Methyl;
oder **R⁷** und **R⁸** bilden gemeinsam Oxo;
bedeuten, sowie pharmakologisch verträgliche Salze, Diastereomere, Enantiomere, Racemate, Hydrate oder Solvate davon.

4. Verbindungen der Formel **1,** nach einem der Ansprüche 1 bis 3, worin
**R¹** H;
bedeutet, sowie pharmakologisch verträgliche Salze, Diastereomere, Enantiomere, Racemate, Hydrate oder Solvate davon.

5. Verbindungen der Formel **1,** nach einem der Ansprüche 1 bis 4, worin
**X** SO;
bedeutet, sowie pharmakologisch verträgliche Salze, Diastereomere, Enantiomere, Racemate, Hydrate oder Solvate davon.

6. Verbindungen der Formel **1,** nach einem der Ansprüche 1 bis 5, worin
R⁵, R⁶, R⁷, R⁸ H;
bedeuten, sowie pharmakologisch verträgliche Salze, Diastereomere, Enantiomere, Racemate, Hydrate oder Solvate davon.

7. Verbindungen der Formel **1,** nach einem der Ansprüche 1 bis 6, worin
**R⁴** H;
bedeutet, sowie pharmakologisch verträgliche Salze, Diastereomere, Enantiomere, Racemate, Hydrate oder Solvate davon.

8. Verbindungen der Formel **1,** nach einem der Ansprüche 1 bis 7, worin
**R²** C₆₋₁₀-Aryl, das gegebenenfalls mit einem oder mehreren Resten unabhängig voneinander ausgewählt aus der Gruppe bestehend aus C₁₋₆-Alkyl, C₁₋₄-Haloalkyl, C₂₋₆-Alkenyl, C₂₋₆-Alkinyl, CN, Halogen, OR^{2.8}, COOR^{2.8}, COR^{2.10}, NR^{2.8}R^{2.9}, NHCOR^{2.8}, SR^{2.8}, SOR^{2.8}, SO₂R^{2.8} und SO₂NR^{2.8}R^{2.9} substituiert sein kann
oder **R²** C₆₋₁₀-Aryl, das gegebenenfalls mit einem oder mehreren Resten ausgewählt aus der Gruppe bestehend aus C₆₋₁₀-Aryl-C₁₋₆-alkylen, C₅₋₁₀-Heteroaryl-C₁₋₆-alkylen, C₆₋₁₀-Aryl und einem heterocyclischen C₃₋₁₀-Ring substituiert sein kann, der gegebenenfalls mit einem Rest ausgewählt aus der Gruppe bestehend aus C₁₋₆-Alkyl, C₁₋₆-Haloalkyl, COOR^{2.8}, CN, Halogen, OR^{2.8} , NHCOR^{2.8}, Oxo, ein C₃₋₇-Cycloalkyl-C₁₋₄ alkylen, ein Heterocyclus-C₁₋₄alkylen und ein NR^{2.1}R^{2.2}-C₁₋₄-alkylen substituiert sein kann,
wobei
R^{2.8} H, C₁₋₆-Alkyl, C₂₋₆-Alkenyl, C₂₋₆-Alkinyl, ein NR^{2.1}R^{2.2}-C₁₋₄-alkylen oder C₆₋₁₀-Aryl;
R^{2.9} H, C₁₋₆-Alkyl, C₂₋₆-Alkenyl oder C₂₋₆-Alkinyl;
R^{2.10} NHR^{2.10.1} ,C₁₋₆-Alkylen-O-C₁₋₄-Alkyl, oder ein heterocyclischer C₃₋₁₀-Ring, der gegebenenfalls mit C₁₋₄-Alkyl substituiert sein kann;
R^{2.10.1} H, C₁₋₆-Alkyl, C₂₋₆-Alkenyl, C₂₋₆-Alkinyl oder C₃₋₇-Cycloalkyl;
oder **R²** bedeutet C₆₋₁₀-Aryl, an das ein aromatischer oder nicht aromatischer C₃₋₁₀-Heterocyclus ankondensiert ist;
oder **R²** C₆₋₁₀-Aryl, das gegebenenfalls mit einem Rest ausgewählt aus der Gruppe bestehend aus C₆₋₁₀-Aryl und einem heterocyclischen C₃₋₁₀-Ring substituiert sein kann, der gegebenenfalls mit einem oder mehreren Resten aus der Gruppe C₁₋₆-Alkyl, C₂₋₆-Alkenyl, C₂₋₆-Alkinyl, C₁₋₄-Haloalkyl, CN, Halogen, OR^{2.8}, COOR^{2.8}, COR^{2.10} NR^{2.8}R^{2.9}, NHCOR^{2.8}, SR^{2.8}, SOR^{2.8}, SO₂R^{2.8}, SO₂NR^{2.8}R^{2.9} und Oxo substituiert sein kann;
bedeutet, sowie pharmakologisch verträgliche Salze, Diastereomere, Enantiomere, Racemate, Hydrate oder Solvate davon.

9. Verbindungen der Formel **1,** nach einem der Ansprüche 1 bis 8, worin
**R²** C₆₋₁₀-Aryl, das gegebenenfalls mit einem oder mehreren Resten ausgewählt aus der Gruppe bestehend aus C₁₋₄-Alkyl, CN, Halogen, OR^{2.8}, COOR^{2.8}, COR^{2.10} und NHCOMe substituiert sein kann
R^{2.8} C₁₋₄-Alkyl oder C₆₋₁₀-Aryl;
R^{2.10} NHR^{2.10.1}, Morpholinyl, Methyl-Piperazinyl;
R^{2.10.1} H, Cyclopropyl oder C₁₋₄-Alkyl, das gegebenenfalls mit einem oder mehreren Resten ausgewählt aus O-C₁₋₄-Alkyl, OH oder C₆₋₁₀-Aryl substituiert sein kann;
oder **R²** C₆₋₁₀-Aryl, das gegebenenfalls mit einem Rest ausgewählt aus der Gruppe bestehend aus Phenyl und einem heterocyclischen C₃₋₁₀-Ring substituiert sein kann, der gegebenenfalls mit C₁₋₄-Alkyl, COOR^{2.8}, CN, Halogen, OR^{2.8}, NHCOMe und Oxo substituiert sein kann;
bedeutet, sowie pharmakologisch verträgliche Salze, Diastereomere, Enantiomere, Racemate, Hydrate oder Solvate davon.

10. Verbindungen der Formel **1,** nach einem der Ansprüche 1 bis 7, worin
**R²** ein Rest ausgewählt aus der Gruppe bestehend aus C₁₋₆-Alkyl, C₂₋₆-Alkenyl und C₂₋₆-Alkinyl, welcher gegebenenfalls mit einem Rest ausgewählt aus der Gruppe bestehend aus C₁₋₆-Haloalkyl, CN, OR^{2.1}, NR^{2.1}R^{2.2}, NHCOR^{2.1}, SR^{2.1}, SOR^{2.1}, SO₂R^{2.1}, SO₂NR^{2.1}R^{2.2}, COOR^{2.1} und CONR^{2.1}R^{2.2} substituiert sein kann , der gegebenenfalls mit einem Rest ausgewählt aus der Gruppe bestehend aus C₃₋₇-Cycloalkyl, C₆₋₁₀-Aryl und einem heterocyclischen C₃₋₁₀-Ring substituiert sein kann, der gegebenenfalls wiederum mit einem oder mehreren Resten ausgewählt aus der Gruppe aus C₁₋₆-Alkyl, C₂₋₆-Alkenyl, C₂₋₆-Alkinyl oder Oxo substituiert sein kann;
wobei
R^{2.1} H, C₁₋₆-Alkyl, C₂₋₆-Alkenyl oder C₂₋₆-Alkinyl;
R^{2.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

11. Verbindungen der Formel **1,** nach einem der Ansprüche 1 bis 7 und 10, worin
**R²** C₁₋₆-Alkyl, das gegebenenfalls mit einem Rest ausgewählt aus der Gruppe bestehend aus C₁₋₄-Haloalkyl, CN, OR^{2.1}, NR^{2.1}R^{2.2}, COOR^{2.1} und CONR^{2.1}R^{2.2} substituiert sein kann oder das gegebenenfalls mit einem Rest ausgewählt aus der Gruppe bestehend aus C₃₋₇-Cycloalkyl, C₆₋₁₀-Aryl und einem heterocyclischen, aromatischen C₃₋₁₀-Ring substituiert sein kann, welcher gegebenenfalls wiederum mit Methyl oder Oxo substituiert sein kann;
worin
R^{2.1} H oder C₁₋₄-Alkyl;
R^{2.2} H oder C₁₋₄-Alkyl;
bedeutet, sowie pharmakologisch verträgliche Salze, Diastereomere, Enantiomere, Racemate, Hydrate oder Solvate davon.

12. Verbindungen der Formel **1,** nach einem der Ansprüche 1 bis 9, worin
**R²** C₆₋₁₀-Aryl, das gegebenenfalls in meta-Position mit einem oder mehreren Resten unabhängig voneinander ausgewählt aus der Gruppe bestehend aus C₁₋₄-Alkyl, CN, Halogen, OR^{2.8}, COOR^{2.8}, COR^{2.10} und NHCOMe substituiert sein kann
wobei
R^{2.8} C₁₋₄-Alkyl oder C₆₋₁₀-Aryl;
R^{2.10} NHR^{2.10.1}, Morpholinyl, Methyl-Piperazinyl;
R^{2.10.1} H, Cyclopropyl oder C₁₋₄-Alkyl, wobei das C₁₋₄-Alkyl gegebenenfalls mit einem oder mehreren Resten ausgewählt aus der Gruppe aus O-C₁₋₄-Alkyl, OH und C₆₋₁₀-Aryl substituiert sein kann;
**R²** NH(R^{2.10.1}), Cyclohexyl
oder **NR¹R²** ein heterocyclischer C₅₋₆-Ring ausgewählt aus der Gruppe bestehend aus Pyrrolidin und Piperazin, der gegebenenfalls mit einem oder mehreren Resten ausgewählt aus der Gruppe bestehend aus C₁₋₄-Alkyl, OH und C₁₋₄-Alkanol substituiert sein kann
bedeutet, sowie pharmakologisch verträgliche Salze, Diastereomere, Enantiomere, Racemate, Hydrate oder Solvate davon.

13. Verbindungen der Formel **1,** nach einem der Ansprüche 1 bis 9 und 12, worin
**R³** Phenyl, das gegebenenfalls mit einem oder mehreren Resten ausgewählt aus der Gruppe bestehend aus C₁₋₆-Alkyl, C₂₋₆-Alkenyl, C₂₋₆-Alkinyl, C₁₋₄-Haloalkyl, C₁₋₆-Alkylen-NR^{3.1}R^{3.2}, CN, Halogen, OR^{3.1}, COOR^{3.1}, CONR^{3.1}R^{3.2}, NR^{3.1}R^{3.2}, NHCOR^{3.1}, NO₂, SR^{3.1}, SOR^{3.1}, SO₂R^{3.1}, SO₂NR^{3.1}R^{3.2} und C₁₋₆-Alkylen-NHCOR^{3.1} substituiert sein kann;
worin
R^{3.1} H, C₁₋₆-Alkyl, C₂₋₆-Alkenyl oder C₂₋₆-Alkinyl;
R^{3.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

14. Verbindungen der Formel **1,** nach einem der Ansprüche 1 bis 9 und 12 bis 13, worin
**R³** Phenyl, welches gegebenenfalls in para-Position mit einem Rest ausgewählt aus der Gruppe bestehend aus C₁₋₆-Alkyl, C₂₋₆-Alkenyl, C₂₋₆-Alkinyl, C₁₋₄-Haloalkyl, C₁₋₆-alkylen-NR^{3.1}R^{3.2}, CN, Halogen, OR^{3.1}, COOR^{3.1}, CONR^{3.1}R^{3.2}, NR^{3.1}R^{3.2}, NHCOR^{3.1}, NO₂, SR^{3.1}, SOR^{3.1}, SO₂R^{3.1}, SO₂NR^{3.1}R^{3.2} und C₁₋₆-alkylen-NHCOR^{3.1} substituiert sein kann,
worin
R^{3.1} H, C₁₋₆-Alkyl, C₂₋₆-Alkenyl oder C₂₋₆-Alkinyl;
R^{3.2} H, C₁₋₆-Alkyl, C₂₋₆-Alkenyl oder C₂₋₆-Alkinyl;
bedeutet, sowie pharmakologisch verträgliche Salze, Diastereomere, Enantiomere, Racemate, Hydrate oder Solvate davon.

15. Verbindungen der Formel **1,** nach einem der Ansprüche 1 bis 9 und 12 bis 14, worin **R³** eine Gruppe der Formel **1b** 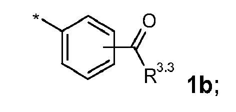 worin
R^{3.3} ein heterocyclischer C₃₋₁₀-Ring, der gegebenenfalls mit einem oder mehreren Resten ausgewählt aus der Gruppe bestehend aus C₁₋₆-Alkyl, Oxo, COR^{3.3.1}, COR^{3.3.2}, C₁₋₆-Alkylen-R^{3.3.2}, CH₂CO-Pyrrolidin und einem heterocyclischen C₃₋₁₀-Ring substituiert sein kann, worin ein gegebenenfalls im heterocyclischen C₃₋₁₀-Ring enthaltenes Schwefelatom gegebenenfalls auch als Oxid oder Dioxid vorliegen kann;
wobei
R^{3.3.1} C₁₋₆-Alkyl;
R^{3.3.2} NH₂, NH(C₁₋₆-Alkyl) oder N(C₁₋₆-Alkyl)₂;
oder R^{3.3} ein bicyclischer Ring oder ein heterocyclischer Spiroring;
bedeutet, sowie pharmakologisch verträgliche Salze, Diastereomere, Enantiomere, Racemate, Hydrate oder Solvate davon.

16. Verbindungen der Formel **1,** nach einem der Ansprüche 1 bis 9 und 12 bis 15, worin **R³** eine Gruppe der Formel **1c;** 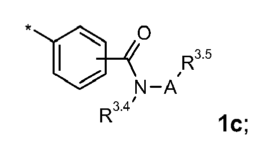 worin
A Bindung oder C₁₋₆-Alkyl, das gegebenenfalls mit Oxo oder NMe₂ substituiert sein kann;
R^{3.4} H oder C₁₋₆-Alkyl;
R^{3.5} ein Rest ausgewählt aus der Gruppe bestehend aus C₁₋₆-Alkyl, das gegebenenfalls durch einen Rest ausgewählt aus der Gruppe bestehend aus C₃₋₇-Cycloalkyl, C₆₋₁₀-Aryl und einem C₅₋₁₀-Heterocyclus substituiert sein kann, welcher jeweils auch gegebenenfalls mit einem oder mehreren Resten ausgewählt aus der Gruppe aus Halogen, OH, Oxo, C₁₋₆-Alkyl, O-C₁₋₆-Alkyl, C₁₋₆-Haloalkyl, einem heterocyclischen C₃₋₁₀-Ring und einem bicyclischen Ring substituiert sein kann, wobei diese Reste jeweils gegebenenfalls mit einem oder mehreren Resten ausgewählt aus der Gruppe bestehend aus Oxo, C₁₋₆-Alkyl, OH, C₆₋₁₀-Aryl, einem heterocyclischen Ring, C₁₋₆-Alkylen-R^{3.5.1}, O-C₁₋₆-Alkylen-R^{3.5.1} und NH-C₁₋₆-Alkylen-R^{3.5.1}substituiert sein können,
wobei
R^{3.5.1} ein Rest ausgewählt aus der Gruppe bestehend aus C₆₋₁₀-Aryl und heterocyclischer C₃₋₁₀-Ring, der gegebenenfalls mit C₁₋₆-Alkyl substituiert sein kann ;
bedeutet, sowie pharmakologisch verträgliche Salze, Diastereomere, Enantiomere, Racemate, Hydrate oder Solvate davon.

17. Verbindungen der Formel **1** nach einem der Ansprüche 1 bis 9 und 12 bis 16, worin **R³** eine Gruppe der Formel **1d;** 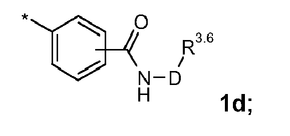 worin
D C₂₋₄-Alkinyl;
R^{3.6} Pyridinyl;
bedeutet, sowie pharmakologisch verträgliche Salze, Diastereomere, Enantiomere, Racemate, Hydrate oder Solvate davon.

18. Verbindungen der Formel **1,** nach einem der Ansprüche 1 bis 9 und 12 bis17, worin **R³** eine Gruppe der Formel **1e;** 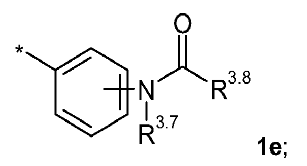 worin
R^{3.7} H, Halogen, C₁₋₆-Alkyl, C₂₋₆-Alkenyl;C₂₋₆-Alkinyl oder C₁₋₆-Haloalkyl;
R^{3.8} H, OH, Halogen oder ein Rest ausgewählt aus der Gruppe bestehend aus C₁₋₆-Alkyl, C₂₋₆-Alkenyl;C₂₋₆-Alkinyl, C₁₋₆-Haloalkyl, O-C₁₋₆-Alkyl, C₆₋₁₀-Aryl, ein heterocyclischer C₃₋₁₀-Ring und ein bicyclischer Ring, der gegebenenfalls mit einem oder mehreren Resten ausgewählt aus der Gruppe aus Halogen, C₁₋₆-Alkyl, OH, C₁₋₆-Haloalkyl und O-C₁₋₆-Alkyl substituiert sein kann,
bedeutet, sowie pharmakologisch verträgliche Salze, Diastereomere, Enantiomere, Racemate, Hydrate oder Solvate davon.

19. Zwischenprodukte ausgewählt aus der Gruppe bestehend aus:
- Verbindungen nach Formel VIII nach Herstellungsweg B nach Schema 1
- Verbindungen nach Formel IV und V nach Herstellungsweg A nach Schema 1
- Verbindungen nach Formel I nach Schema 1
- Verbindungen nach Formel IV, VI, VII und VIII nach Schema 2
- Verbindungen nach Formel V, VII und VIII nach Schema 3
- Verbindungen nach Formel V, VII und IX nach Schema 4
- Verbindungen nach Formel V, VI, VII, VIII und IX nach Schema 5
- Verbindungen nach Formel V, VII, VIII und IX nach Schema 6
- Verbindungen nach Formel V, VII, VIII, XI und XII nach Schema 7
- Verbindungen nach Formel I und II nach Schema 8
- Verbindungen nach Formel II und III nach Schema 9
- Verbindungen nach Formel V, VI, VIII und X nach Schema 10
- Verbindungen nach Formel III und V nach Schema 11
- Verbindungen nach Formel III und V nach Schema 12
- Verbindungen nach Formel II, III, IV und V nach Schema 13
- Verbindungen nach Formel IV, V, VII, VIII, IX und X nach Schema 14,
worin R¹, R², R³, R⁴, R⁵, R⁶, R⁷, R⁸ und X die in den Ansprüchen 1 bis 18 definierten Bedeutungen haben,
und worin R¹, R², R³, R⁴, R⁵, R⁶, R⁷, R⁸ und X die vorstehend definierten Bedeutungen haben und wobei R⁹, R¹⁰, R¹¹, R¹² jeweils unabhängig voneinander einen Rest ausgewählt aus der Gruppe bestehend aus H, Halogen, C₁₋₆-Alkyl, C₁₋₆-Haloalkyl, C₆₋₁₀-Aryl, C₃₋₇-Cycloalkyl, aromatischer oder nicht aromatischer C₃₋₁₀-Heterocyclus, C₆₋₁₀-Aryl-C₁₋₆-Alkylen, C₃₋₇-Cycloalkyl- C₁₋₆-Alkylen und C₃₋₁₀-Heterocyclus-C₁₋₆-Alkylen bedeuten, der gegebenenfalls mit einem oder mehreren Resten ausgewählt aus der Gruppe bestehend aus OH, Oxo, C₁₋₆-Alkyl, Phenyl C₃₋₇-Cycloalkyl, C₃₋₇-Heterocyclus und Halogen substituiert sein kann,
und worin R¹³ ausgewählt ist aus der Gruppe bestehend aus OH, Halogen, O-C₁₋₆-Alkyl, C₁₋₆-Alkyl, Phenyl und C₃₋₇-Cycloalkyl und C₃₋₇-Heterocyclus.

20. Zwischenprodukte nach Anspruch 19 ausgewählt aus der Gruppe bestehend aus:
- Verbindungen nach Formel I nach Schema 1
- Verbindungen nach Formel VI, VII und VIII nach Schema 2
- Verbindungen nach Formel VII und VIII nach Schema 3
- Verbindungen nach Formel VII und IX nach Schema 4
- Verbindungen nach Formel VI, VII, VIII und IX nach Schema 5
- Verbindungen nach Formel VII, VIII und IX nach Schema 6
- Verbindungen nach Formel VII, VIII, XI und XII nach Schema 7
- Verbindungen nach Formel I und II nach Schema 8
- Verbindungen nach Formel II und III nach Schema 9
- Verbindungen nach Formel X nach Schema 10
- Verbindungen nach Formel III und V nach Schema 11
- Verbindungen nach Formel III und V nach Schema 12
- Verbindungen nach Formel II, III, IV und V nach Schema 13
- Verbindungen nach Formel VII, VIII, IX und X nach Schema 14.

21. Verbindungen nach einem der Ansprüche 1 bis 18 als Arzneimittel.

22. Verwendung von Verbindungen nach einem der Ansprüche 1 bis 18 zur Herstellung eines Medikaments zur Behandlung von Krankheiten, die sich durch Inhibition des PDE4-Enzyms behandeln lassen.

23. Verwendung von Verbindungen nach einem der Ansprüche 1 bis 18 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

24. Verwendung von Verbindungen nach einem der Ansprüche 1 bis 18 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

25. Verwendung von Verbindungen nach einem der Ansprüche 1 bis 18 zur Herstellung eines Medikaments zur Behandlung von entzündlichen und obstruktiven Erkrankungen wie COPD, chronischer Sinusitis, Asthma, Morbus Crohn, Colitis ulcerosa.

26. Verwendung von Verbindungen nach einem der Ansprüche 1 bis 18 zur Herstellung eines Medikaments zur Behandlung von entzündlichen Erkrankungen des Gastrointestinaltraktes.

27. Verwendung von Verbindungen nach einem der Ansprüche 1 bis 18 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

28. Pharmazeutische Formulierungen **gekennzeichnet durch** den Gehalt an einer oder mehreren Verbindungen der Formel **1** nach einem der Ansprüche 1 bis 18.

29. Inhalativ applizierbare pharmazeutische Formulierung, **gekennzeichnet durch** einen Gehalt einer Verbindung der Formel **1** gemäß einem Ansprüche 1 bis 18.
